(19) Europäisches Patentamt
European Patent Office
Office européen des brevets

(11) EP 4 631 943 A1

(12) EUROPEAN PATENT APPLICATION
published in accordance with Art. 153(4) EPC

(43) Date of publication:
**15.10.2025 Bulletin 2025/42**

(21) Application number: **23900000.3**

(22) Date of filing: **06.12.2023**

(51) International Patent Classification (IPC):
***C07D 471/04*** (2006.01) ***A61K 31/53*** (2006.01)
***A61P 3/04*** (2006.01) ***A61P 3/06*** (2006.01)
***A61P 3/10*** (2006.01) ***A61P 1/16*** (2006.01)
***A61P 9/10*** (2006.01) ***A61P 9/00*** (2006.01)
***A61P 17/14*** (2006.01) ***A61P 35/00*** (2006.01)
***A61P 5/14*** (2006.01)

(52) Cooperative Patent Classification (CPC):
**A61K 31/53; A61P 1/16; A61P 3/04; A61P 3/06; A61P 3/10; A61P 5/14; A61P 9/00; A61P 9/10; A61P 17/14; A61P 35/00; C07D 471/04**

(86) International application number:
**PCT/CN2023/136702**

(87) International publication number:
**WO 2024/120426 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.12.2022 CN 202211563493**

(71) Applicant: **KPC PHARMACEUTICALS, INC**
**Kunming, Yunnan 650106 (CN)**

(72) Inventors:
- **YANG, Haili**
  **Kunming, Yunnan 650106 (CN)**
- **XI, Liang**
  **Kunming, Yunnan 650106 (CN)**
- **LIU, Junfeng**
  **Kunming, Yunnan 650106 (CN)**
- **JIANG, Guanyu**
  **Kunming, Yunnan 650106 (CN)**
- **XU, Anchao**
  **Kunming, Yunnan 650106 (CN)**
- **LI, Kui**
  **Kunming, Yunnan 650106 (CN)**

(74) Representative: **V.O.**
**P.O. Box 87930**
**2508 DH Den Haag (NL)**

# (54) THYROID HORMONE ß RECEPTOR AGONIST, CRYSTAL FORM, PREPARATION METHOD AND USE

(57) The present disclosure belongs to the field of medicines. Disclosed in the present invention are a thyroid hormone β receptor agonist, a crystalline form, a preparation method and the use. The compound is a compound having a structural formula as represented by formula (I) or a pharmaceutically acceptable salt thereof. The X-ray powder diffraction pattern of a crystal form A of the compound of formula (I) comprises characteristic peaks at 6.10 ± 0.20°, 12.08 ±0.20° and 16.49 ±0.20° 2θ, as determined by means of using Cu-Ka radiation, The present invention develops a new 2-pyridone derivative. The compound has high agonistic activity and selectivity for the thyroid hormone β receptor, and can be used for treating metabolic diseases, particularly for treating diseases related to thyroid hormone receptor. The present invention also studies a series of crystal forms of the compound. The crystal form A compound has the advantages of good stability, low hygroscopicity, and small heat influence.

EP 4 631 943 A1

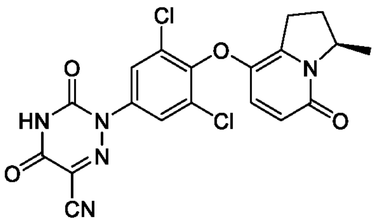

(I)

## Description

This application claims the priority to Chinese Patent Application No. 202211563493.0, titled "THYROID HORMONE β RECEPTOR AGONIST, CRYSTAL FORM, PREPARATION METHOD AND USE", filed on December 7, 2022 with the China National Intellectual Property Administration, which is incorporated herein by reference in its entirety.

## FIELD

The present disclosure relates to the field of medicines, and in particular to a thyroid hormone β receptor agonist, a crystalline form, a preparation method and use.

## BACKGROUND

Thyroid hormones play a key role in normal growth and development of the body and in maintaining metabolic balance (Physiological Reviews 2001, 81(3), 1097-1126.). Thyroid hormones are produced by the thyroid and are secreted into the circulatory system (hypothalamic/pituitary/thyroid system) in two different forms, T4 and T3, with T4 being the predominant form secreted by the thyroid and T3 being the more physiologically active form. T4 is converted to T3 by tissue-specific deiodinase, which is present in all tissues, but mainly in liver and kidney tissues.

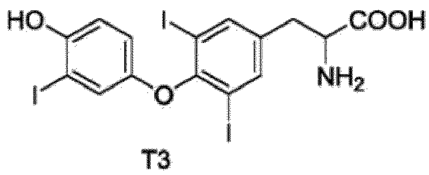

T3

The circulating level of thyroid hormones is strictly regulated by the feedback mechanism in hypothalamic/pituitary/thyroid axis. Thyroid dysfunction leading to hypothyroidism or hyperthyroidism has a profound impact on heart/-body weight/metabolism/metabolic rate/body temperature/cholesterol/bone/muscle and behavior.

The physiological activity of thyroid hormones is mediated by thyroid hormone receptors (THRs) (Endocrine Reviews 1993, 14, 348-399.). THR, a member of nuclear receptor family, is encoded by different genes α and β located on human chromosomes 17 and 3. Different protein isoforms are generated by selective splicing of the primary transcript. Each gene produces two isoforms, namely THRα1, THRα2, THRβ1 and THRβ2. THRβ1 and THRβ2 are obtained by differential expression from promoters, and the two isoforms differ only at the amino terminus. THRα1 and THRα2 result from differential splicing of pre-mRNAs, and mainly differ at the carboxy terminus. THRα1, THRβ1 and THRβ2 can bind to thyroid hormones. THRβ is mainly distributed in liver/kidney/pituitary and brain tissue, and plays an important role in regulating TRH and thyroid hormone behavior in liver, and THRα is widely distributed all over the body, mainly related to cardiovascular and skeletal/muscular adverse reactions outside the liver (Drugs (2017) 77 1613-1621). Therefore, a thyroid hormone analog, if the adverse effects of hyperthyroidism and hypothyroidism can be avoided while maintaining the beneficial effects of thyroid hormones, may be used in the treatment responsive to diseases such as metabolic diseases including obesity, hyperlipidemia, hypercholesterolemia, diabetes and other conditions such as hepatic steatosis and nonalcoholic steatohepatitis (NASH), atherosclerosis, cardiovascular disease, hypothyroidism, thyroid cancer, thyroid disease and the like.

In the prior art, a series of thyroid hormone agonists have been developed, and almost all of these structural agonists are designed and developed based on the structure of natural ligand T3 of THR receptor. For example, thyroid hormone analogs with different structures from the compounds of the present disclosure have been disclosed (Agricultural and Biol. Chem. 1974, 38 (6), 1169; J. Med. Chem. 1989, 32, 320; J. Med. Chem. 2014, 57(10), 3912; WO2007009913; WO2010122980). Among them, Example 8 (Compound 31) disclosed in WO2007009913 is MGL-3196, a small molecular selective agonist of liver thyroid hormone receptor β isoforms (THR-β) for oral administration that is most advanced in clinical development currently. The preclinical toxicology and clinical data show that MGL-3196 is a potential treatment method for nonalcoholic steatohepatitis (NASH) and dyslipidemia. MGL-3196 can significantly reduce LDL cholesterol, triglycerides and lipoproteins, making it an ideal candidate medicine for reducing the cardiovascular risk in NASH patients and for dyslipidemia patients with moderate statin dosage or intolerant to statins.

After that, many documents, such as WO2020073974, CN111320609A, WO2019240938, have disclosed a series of structural modifications in the pyridazinone ring in different directions based on the MGL-3196 structure, but the parent nucleus always had a pyridazinone structure similar to that of MGL-3196. The recently published WO2020169069 and WO2021067791 both disclose another pyridone analog structure, but there are problems that both THRβ biological activity and THRβ/THRα selectivity are reduced, especially that most compounds almost lose their pharmacological

activity after the nitrogen atom on pyridone is replaced, or the stability is extremely poor in the metabolic process in vivo, so it is difficult to develop drugs.

These compounds and experimental medicines disclosed in the prior art still have some problems in their druggabilities. Therefore, it is necessary to continue to discover and develop new compounds with high activity and high selectivity that have beneficial effects of thyroid hormone and can avoid adverse effects, and new compounds with good in vivo pharmacokinetic effects are used to treat diseases related to thyroid hormone receptors.

In view of that, the present disclosure is provided.

## SUMMARY

The technical problem to be solved by the present disclosure is to overcome the shortcomings of the prior art and provide a thyroid hormone β receptor agonist, crystalline form, preparation method and use. The present disclosure develops a new 2-pyridone derivative, which has high agonist activity and selectivity on thyroid hormone β receptor and can be used for treating metabolic diseases, particularly for treating diseases related to thyroid hormone receptor. In addition, the present disclosure also studies a series of crystalline forms of the compound, which has the advantages of good stability, low hygroscopicity and small heat influence.

In order to solve above-mentioned technical problems, the technical solutions with the following basic concepts are adopted by the present disclosure.

The first object of the present disclosure is to provide a compound having a structural formula represented by formula (I) or a pharmaceutically acceptable salt thereof:

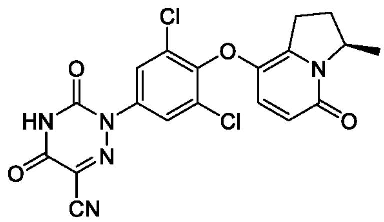

(I)

.

The second object of the present disclosure is to provide a medicine, which comprises a compound represented by formula (I) or a pharmaceutically acceptable salt thereof as an active ingredient.

Preferably, the medicine is a thyroid hormone β receptor agonist. Further, the medicine also comprises a pharmaceutically acceptable excipient.

The third object of the present disclosure is to provide use of a compound represented by formula (I) or a pharmaceutically acceptable salt thereof in manufacture of a medicament for treating a metabolic disease; preferably, the medicament is a thyroid hormone β receptor agonist; preferably, the metabolic disease includes obesity, hyperlipidemia, hypercholesterolemia, diabetes, hepatic steatosis, nonalcoholic steatohepatitis, atherosclerosis, cardiovascular diseases, thyroid diseases, and tumor in intrahepatic bile duct. Preferably, the thyroid diseases include thyroid cancer and hypothyroidism.

The fourth object of the present disclosure is to provide crystalline form A of the compound represented by formula (I), which X-ray powder diffraction pattern shows characteristic diffraction peaks at 2θ angles of 6.10±0.20° 12.08 ±0.20° and 16.49±0.20°

In some embodiments of the present disclosure, the X-ray powder diffraction pattern of the above-mentioned crystalline form A shows characteristic diffraction peaks at 2θ angles of 6.10±0.20° 12.08±0.20° 13.61±0.20° 15.71 ±0.20° 16.49±0.20° 20.05±0.20 °, 21.47±0.20° and 22.49±0.20°.

In some embodiments of the present disclosure, the X-ray powder diffraction pattern of the above-mentioned crystalline form A shows characteristic diffraction peaks at 2θ angles of 6.10±0.20° 12.08±0.20°, 13.61±0.20°, 15.71 ±0.20°, 16.49±0.20° 20.05±0.20 °, 20.73±0.20°, 21.47±0.20°, 21.80±0.20° and 22.49±0.20°.

In some embodiments of the present disclosure, the X-ray powder diffraction pattern of the above-mentioned crystalline form A shows characteristic diffraction peaks at 2θ angles of 6.10±0.20°, 6.86±0.20°, 8.64±0.20°, 10.14 ±0.20°, 12.08±0.20°, 12.75±0.20°, 13.64±0.20°, 14.44±0.20°, 15.22±0.20°, 15.45±0.20°, 15.75±0.20°, 16.49 ±0.20° 17.14 ±0.20°, 17.89±0.20°, 18.08±0.20°, 18.62±0.20°, 18.82±0.20°, 19.09±0.20°, 19.59±0.20°, 20.07 ±0.20°, 21.53±0.20°, 21.83±0.20°, 22.51±0.20°, 23.26±0.20°, 23.90±0.20°, 24.16±0.20°, 24.47±0.20°, 24.89 ±0.20°, 25.56±0.20°, 25.77±0.20°, 26.17±0.20°, 26.46±0.20°, 2 6.65±0.20°, 26.97±0.20°, 27.51±0.20°, 27.79 ±0.20°, 28.41±0.20°, 28.83±0.20°, 29.05±0.20°, 29.59±0.20°, 30.47±0.20°, 31.03±0.20°, 31.37±0.20°, 31.97±0.20°, 32.30±0.20°, 32.71±0.20°, 33.26±0.20°, 33.37±0.20°, 34.35±0.20°, 35.62±0.20°,36.09±0.20°,38.20±0.20°, 38.74±0.20° and 39.40±0.20° 。

In some embodiments of the present disclosure, the XRPD pattern of the above-mentioned crystalline form A is shown in FIG. 1.

In some embodiments of the present disclosure, analysis data of the XRPD pattern of the above-mentioned crystalline form A are shown in Table 1:

Table 1. Analytical data of XRPD pattern of the crystalline form A

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.101 | 14.4738 | 100 | 29 | 24.891 | 3.5742 | 8.5 |
| 2 | 6.864 | 12.8669 | 2 | 30 | 25.556 | 3.4827 | 2.5 |
| 3 | 8.640 | 10.2257 | 3.9 | 31 | 25.773 | 3.4539 | 1.1 |
| 4 | 10.141 | 8.7157 | 1.4 | 32 | 26.172 | 3.4021 | 6.6 |
| 5 | 12.081 | 7.3200 | 35.3 | 33 | 26.462 | 3.3654 | 3.9 |
| 6 | 12.753 | 6.9357 | 2.4 | 34 | 26.649 | 3.3422 | 3.1 |
| 7 | 13.646 | 6.4838 | 20.1 | 35 | 26.968 | 3.3034 | 12.1 |
| 8 | 14.443 | 6.1278 | 12.5 | 36 | 27.508 | 3.2399 | 4.2 |
| 9 | 15.223 | 5.8154 | 5.9 | 37 | 27.793 | 3.2072 | 10.4 |
| 10 | 15.447 | 5.7315 | 10.7 | 38 | 28.410 | 3.1390 | 1.6 |
| 11 | 15.746 | 5.6235 | 14 | 39 | 28.830 | 3.0942 | 4.3 |
| 12 | 16.487 | 5.3723 | 29.2 | 40 | 29.046 | 3.0716 | 6.4 |
| 13 | 17.136 | 5.1704 | 1 | 41 | 29.593 | 3.0161 | 3.9 |
| 14 | 17.890 | 4.9540 | 6.7 | 42 | 30.472 | 2.9311 | 5.7 |
| 15 | 18.083 | 4.9016 | 3.2 | 43 | 31.030 | 2.8797 | 3.5 |
| 16 | 18.620 | 4.7614 | 1.8 | 44 | 31.366 | 2.8496 | 2.9 |
| 17 | 18.820 | 4.7112 | 4 | 45 | 31.974 | 2.7967 | 4.1 |
| 18 | 19.091 | 4.6451 | 2.8 | 46 | 32.297 | 2.7695 | 4.2 |
| 19 | 19.590 | 4.5278 | 10.6 | 47 | 32.711 | 2.7354 | 1 |
| 20 | 20.069 | 4.4208 | 11.1 | 48 | 33.256 | 2.6918 | 1 |
| 21 | 20.749 | 4.2775 | 13 | 49 | 33.370 | 2.6829 | 1.3 |
| 22 | 21.527 | 4.1246 | 15.2 | 50 | 34.353 | 2.6083 | 3.6 |
| 23 | 21.829 | 4.0682 | 13.7 | 51 | 35.620 | 2.5184 | 3.2 |
| 24 | 22.509 | 3.9468 | 18.8 | 52 | 36.095 | 2.4864 | 1.4 |
| 25 | 23.264 | 3.8203 | 1.8 | 53 | 36.954 | 2.4305 | 4.5 |
| 26 | 23.908 | 3.7188 | 4.4 | 54 | 38.194 | 2.3544 | 5.1 |
| 27 | 24.160 | 3.6807 | 2.1 | 55 | 38.741 | 2.3224 | 1.5 |
| 28 | 24.469 | 3.6349 | 1.5 | 56 | 39.400 | 2.2850 | 1.9 |

In some embodiments of the present disclosure, the differential scanning calorimetry curve of the above-mentioned crystalline form A shows an endothermic peak at 300.1±3.0°C .

In some embodiments of the present disclosure, the DSC curve of the above-mentioned crystalline form A is shown in FIG. 2.

In some embodiments of the present disclosure, the thermogravimetric analysis curve of the above-mentioned crystalline form A shows a weight loss of 0.6% at 150.0C ±3.0°C .

In some embodiments of the present disclosure, the TGA curve of the above-mnetioned crystalline form A is

shown in FIG. 3.

Further, the present disclosure provides use of the crystalline form A of the compound represented by formula (I) in the manufacture of a medicament for treating a metabolic disease.

Further, the present disclosure provides use of the crystalline form A of the compound represented by formula (I) in the manufacture of a medicament for treating a disease related to thyroid hormone receptors.

Preferably, the medicament is used as a thyroid hormone β receptor agonist; preferably, the metabolic disease includes obesity, hyperlipidemia, hypercholesterolemia, diabetes, hepatic steatosis, nonalcoholic steatohepatitis, atherosclerosis, cardiovascular diseases, thyroid diseases, and tumor in intrahepatic bile duct.

After analysis, it is found that the crystalline form A of the compound represented by formula (I) has good stability under high temperature, high humidity, long-term and accelerated conditions, and has low hygroscopicity.

The fifth object of the present disclosure is to provide a method for preparing crystalline form A of the compound represented by formula (I), which comprises:

(1) dissolving a compound represented by formula (I) in a mixed solvent of tetrahydrofuran and methanol, and heating to complete dissolution;

(2) adding water into the solution under stirring, cooling the solution slowly, continuously stirring and filtering the solution; and;

(3) washing the obtained filter cake with methanol, collecting the filter cake, and drying in vacuum to constant weight to obtain the crystalline form A of the compound represented by formula (I).

Preferably, in step (1), the volume ratio of tetrahydrofuran to methanol in the mixed solvent is 2:1 to 1:2.

Preferably, in step (1), the concentration of the compound represented by formula (I) in the mixed solvent is 0.02 g/ml to 0.2 g/ml.

Preferably, in step (1), the heating is performed until 45°C to 65C .

Preferably, in step (2), the volume ratio of the added water to the mixed solvent in step (1) is 1:2 to 3:1.

Preferably, in step (2), water is added to the solution at 15°C to 35°C ; the solution is slowly cooled to 15°C to 30°C and continuously stirr ed for 0.5 h to 1 h.

Or, the preparation method comprises:

(1) dissolving the compound represented by formula (I) in N,N-dimethylformamide to obtain a clear solution, and then filtering;

(2) adding water to the solution under stirring, precipitating solid, then stirring overnight and centrifuging; and;

(3) washing the obtained filter cake with water, collecting the filter cake, and drying in vacuum to constant weight to obtain the crystalline form A of the compound represented by formula (I).

Preferably, in step (1), the concentration of the compound represented by formula (I) in N,N-dimethylformamide is 0.1 g/ml to 0.3 g/ml.

Preferably, in step (2), the volume ratio of the added water to N,N-dimethylformamide in step (1) is 1:2 to 3:1.

Preferably, in step (2), water is added to the solution at 15°C to 35C .

Or, the preparation method comprises:

(1) dissolving the compound represented by formula (I) in dimethyl sulfoxide to obtain a clear solution, and then filtering;

(2) adding water to the solution under stirring, precipitating solid, then stirring overnight and centrifuging; and;

(3) washing the obtained filter cake with water, collecting the filter cake, and drying in vacuum to constant weight to obtain the crystalline form A of the compound represented by formula (I).

Preferably, in step (1), the concentration of the compound represented by formula (I) in dimethyl sulfoxide is 0.3 g/ml to 0.8 g/ml.

Preferably, in step (2), the volume ratio of the added water to dimethyl sulfoxide in step (1) is 3: 1 to 7:1.

Preferably, in step (2), water is added to the solution at 15°C to 35°C .

Or, the preparation method comprises:

(1) dissolving the compound represented by formula (I) in methanol, heating to complete dissolution, and filtering;

(2) removing part of methanol by concentration until solid is precipitated, cooling and centrifuging; and;

(3) washing the obtained filter cake with methanol, collecting the filter cake, and drying in vacuum to constant weight to obtain crystalline form A of the compound represented by formula (I).

Preferably, in step (1), the concentration of the compound represented by formula (I) in methanol is 0.01 g/ml to 0.1 g/ml.

Preferably, in step (1), the heating is performed at 45C to 65C .

Preferably, in step (2), part of methanol is removed by concentration at a temperature of 30C to 50°C .

Preferably, in step (2), the cooling is performed at room temperature of 20°C to 30°C .

The sixth object of the present disclosure is to provide crystalline form B of the compound represented by formula (I), and its X-ray powder diffraction pattern shows characteristic diffraction peaks at 2θ angles of 9.3530.20° , 10.36±0.20° and 18.32±0.20° .

In some embodiments of the present disclosure, the X-ray powder diffraction pattern of the above-mentioned crystalline form B shows characteristic diffraction peaks at 2θ angles of 9.35±0.20°, 10.36±0.20°, 11.77±0.20°, 12.65 ±0.20°, 15.21±0.20°, 18.32±0.20°, 19.60±0.20° and 22.74±0.20°.

In some embodiments of the present disclosure, the X-ray powder diffraction pattern of the above-mentioned crystalline form B shows characteristic diffraction peaks at 2θ angles of 9.35±0.20°, 10.36±0.20°, 11.77±0.20°, 12.65 ±0.20°, 15.21±0.20°, 18.32±0.20°, 19.60±0.20°, 20.03±0.20°, 21.30±0.20° and 22.74±0.20°.

In some embodiments of the present disclosure, the X-ray powder diffraction pattern of the above-mentioned crystalline form B shows characteristic diffraction peaks at 2θ angles of 4.99°,7.54°,9.34°, 10.35°, 11.02°, 11.36°, 11.76°, 12.64°, 13.97°, 15.21°, 16.24°, 17.46°, 18.32°, 18.69°, 19.33°, 19.59°, 20.03°, 20.42°, 20.72°, 21.29°, 22.13°, 22.74°, 23.59°, 24.13°, 24.37°, 24.95°, 25.39°, 25.83°, 26.24°, 26.49°, 26.97°, 28.04°, 28.18°, 28.81°, 29.83°, 30.87°, 33.56°, 35.64°,37.66° and 39.26° 。

In some embodiments of the present disclosure, the XRPD pattern of the above-mentioned crystalline form B is shown in FIG. 4.

In some embodiments of the present disclosure, analysis data of the XRPD pattern of the above-mentioned crystalline form B are shown in Table 2:

Table 2. Analytical data of the XRPD pattern of crystalline form B

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 4.998 | 17.6655 | 3.2 | 21 | 22.133 | 4.0130 | 4.1 |
| 2 | 7.546 | 11.7061 | 6.4 | 22 | 22.743 | 3.9067 | 23.8 |
| 3 | 9.348 | 9.4533 | 55.1 | 23 | 23.590 | 3.7683 | 6.1 |
| 4 | 10.357 | 8.5339 | 54.6 | 24 | 24.131 | 3.6850 | 10.6 |
| 5 | 11.026 | 8.0179 | 9.3 | 25 | 24.378 | 3.6482 | 3.9 |
| 6 | 11.366 | 7.7788 | 10.8 | 26 | 24.958 | 3.5647 | 9.1 |
| 7 | 11.766 | 7.5151 | 25.6 | 27 | 25.392 | 3.5048 | 5.1 |
| 8 | 12.646 | 6.9939 | 25.9 | 28 | 25.832 | 3.4461 | 5.7 |
| 9 | 13.975 | 6.3316 | 5 | 29 | 26.247 | 3.3926 | 6.8 |
| 10 | 15.212 | 5.8196 | 39 | 30 | 26.492 | 3.3617 | 3.7 |
| 11 | 16.242 | 5.4527 | 12.9 | 31 | 26.971 | 3.3031 | 5.2 |
| 12 | 17.462 | 5.0745 | 3.5 | 32 | 28.040 | 3.1796 | 4.4 |
| 13 | 18.320 | 4.8388 | 100 | 33 | 28.187 | 3.1633 | 3.2 |
| 14 | 18.696 | 4.7423 | 4.2 | 34 | 28.816 | 3.0957 | 7 |
| 15 | 19.331 | 4.5877 | 11 | 35 | 29.833 | 2.9924 | 4.4 |
| 16 | 19.598 | 4.5259 | 39.9 | 36 | 30.874 | 2.8939 | 3.9 |
| 17 | 20.030 | 4.4292 | 16.7 | 37 | 33.566 | 2.6677 | 4 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 18 | 20.429 | 4.3437 | 4.2 | 38 | 35.645 | 2.5167 | 3 |
| 19 | 20.726 | 4.2820 | 6.3 | 39 | 37.665 | 2.3862 | 2.4 |
| 20 | 21.290 | 4.1700 | 13.3 | 40 | 39.263 | 2.2927 | 2.8 |

In some embodiments of the present disclosure, the differential scanning calorimetry curve of the above-mentioned crystalline form B shows endothermic peaks at 139.5°C±3.0°C and 315.6±3.0°C respectively.

In some embodiments of the present disclosure, the DSC curve of the above-mentioned crystalline form B is shown in FIG. 5.

In some embodiments of the present disclosure, the thermogravimetric analysis curve of the above-mentioned crystalline form B shows a weight loss of 16.0% at 150.0°C ±3.0°C .

In some embodiments of the present disclosure, the TGA curve of the above-mentioned crystalline form B is shown in FIG. 6.

The seventh object of the present disclosure is to provide crystalline form C of the compound represented by formula (I), which X-ray powder diffraction pattern shows characteristic diffraction peaks at 2θ angles of 5.96±0.20°, 10.96 ±0.20° and 22.84±0.20°.

In some embodiments of the present disclosure, the X-ray powder diffraction pattern of the above-mentioned crystalline form C shows characteristic diffraction peaks at 2θ angles of 5.96±0.20°, 9.25±0.20°, 10.96±0.20°, 11.94 ±0.20°, 21.92±0.20°, 22.84±0.20°, 23.69±0.20° and 28.12±0.20°.

In some embodiments of the present disclosure, the X-ray powder diffraction pattern of the above-mentioned crystalline form C shows characteristic diffraction peaks at 2θ angles of 5.96±0.20°, 9.25±0.20°, 10.96±0.20°, 11.94 ±0.20°, 15.52±0.20°, 19.50±0.20°, 21.92±0.20°, 22.84±0.20°, 23.69±0.20° and 28.12±0.20°.

In some embodiments of the present disclosure, the X-ray powder diffraction pattern of the above-mentioned crystalline form C shows characteristic diffraction peaks at 2θ angles of 5.96°, 9.25°, 9.91°, 10.96°, 11.51°, 11.93°, 12.29°, 12.55°, 13.56°, 14.41°, 15.04°, 15.52°, 16.41°, 16.68°, 17.30°, 17.51°, 17.92°, 18.45°, 18.79°, 19.50°, 20.01°, 20.33°, 20.70°, 21.42°, 21.92°, 22.24°, 22.83°, 23.68°, 24.11°, 24.48°, 24.78°, 25.23°, 25 .69°, 25.96°, 26.20°, 26.41°, 26.70°, 26.95°, 27.86°, 28.12°, 28.54°, 28.99°, 29.60°, 30.49°, 30.96°, 31.50°, 32.02°, 32.59°, 33.16°, 33.3°, 33.64°, 34.18°, 34.94°, 35.28°, 35.85°, 36.83°, 37.46°, 38.02°, 39.11° and 39.347°

In some embodiments of the present disclosure, the XRPD pattern of the above-mentioned crystalline form C is shown in FIG. 7.

In some embodiments of the present disclosure, analysis data of the XRPD pattern of the above-mentioned crystalline form C are shown in Table 3:

Table 3. Analytical data of the XRPD pattern of the crystalline form C

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 5.962 | 14.8115 | 98.1 | 31 | 24.782 | 3.5897 | 10.8 |
| 2 | 9.252 | 9.5503 | 53.9 | 32 | 25.237 | 3.5260 | 5.6 |
| 3 | 9.916 | 8.9130 | 5.3 | 33 | 25.694 | 3.4643 | 1.9 |
| 4 | 10.962 | 8.0645 | 74.5 | 34 | 25.966 | 3.4286 | 3.6 |
| 5 | 11.514 | 7.6788 | 7 | 35 | 26.209 | 3.3974 | 8.2 |
| 6 | 11.937 | 7.4080 | 27.3 | 36 | 26.416 | 3.3712 | 3 |
| 7 | 12.290 | 7.1956 | 6.6 | 37 | 26.707 | 3.3351 | 11.9 |
| 8 | 12.557 | 7.0435 | 2.4 | 38 | 26.950 | 3.3056 | 5.8 |
| 9 | 13.561 | 6.5240 | 2.5 | 39 | 27.866 | 3.1990 | 5.8 |
| 10 | 14.416 | 6.1390 | 8.2 | 40 | 28.120 | 3.1707 | 16 |
| 11 | 15.049 | 5.8824 | 4.8 | 41 | 28.543 | 3.1247 | 1.8 |
| 12 | 15.523 | 5.7037 | 12.8 | 42 | 28.992 | 3.0773 | 2.9 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 13 | 16.415 | 5.3956 | 6.9 | 43 | 29.607 | 3.0147 | 6.3 |
| 14 | 16.682 | 5.3101 | 2.1 | 44 | 30.498 | 2.9287 | 2.4 |
| 15 | 17.308 | 5.1192 | 11.2 | 45 | 30.961 | 2.8859 | 1.4 |
| 16 | 17.517 | 5.0585 | 7.8 | 46 | 31.507 | 2.8372 | 11.5 |
| 17 | 17.920 | 4.9457 | 5.3 | 47 | 32.025 | 2.7924 | 2.8 |
| 18 | 18.450 | 4.8050 | 8.1 | 48 | 32.591 | 2.7452 | 3.7 |
| 19 | 18.799 | 4.7166 | 6.7 | 49 | 33.167 | 2.6988 | 1.7 |
| 20 | 19.505 | 4.5472 | 15.4 | 50 | 33.3 | 2.6884 | 1.7 |
| 21 | 20.016 | 4.4323 | 2.3 | 51 | 33.641 | 2.6619 | 2.5 |
| 22 | 20.334 | 4.3637 | 4.6 | 52 | 34.188 | 2.6205 | 1.4 |
| 23 | 20.700 | 4.2874 | 1.6 | 53 | 34.946 | 2.5654 | 4 |
| 24 | 21.427 | 4.1436 | 2.8 | 54 | 35.282 | 2.5418 | 2.1 |
| 25 | 21.921 | 4.0513 | 52.8 | 55 | 35.854 | 2.5025 | 10.8 |
| 26 | 22.245 | 3.9930 | 2.5 | 56 | 36.833 | 2.4382 | 1.1 |
| 27 | 22.836 | 3.8911 | 100 | 57 | 37.469 | 2.3982 | 2 |
| 28 | 23.688 | 3.7529 | 18 | 58 | 38.023 | 2.3646 | 2.9 |
| 29 | 24.112 | 3.6878 | 4.7 | 59 | 39.117 | 2.3009 | 1.4 |
| 30 | 24.480 | 3.6333 | 4.4 | 60 | 39.347 | 2.288 | 1.3 |

In some embodiments of the present disclosure, the differential scanning calorimetry curve of the above-mentioned crystalline form C shows an endothermic peak at 123.7°C±3.0°C and 315.7±3.0°C respectively.

In some embodiments of the present disclosure, the DSC curve of the above-mentioned crystalline form C is shown in FIG. 8.

In some embodiments of the present disclosure, the thermogravimetric analysis curve of the above-mentioned crystalline form C shows a weight loss of 10.9% at 150.0°C±3.0°C .

In some embodiments of the present disclosure, the TGA curve of the above-mentioned crystalline form C is shown in FIG. 9.

The eighth object of the present disclosure is to provide the D crystalline form of the compound represented by formula (I), which X-ray powder diffraction pattern shows characteristic diffraction peaks at 2θ angles of 7.97±0.20°, 11.66 ±0.20° and 15.83±0.20°.

In some embodiments of the present disclosure, the X-ray powder diffraction pattern of the above-mentioned crystalline form D shows characteristic diffraction peaks at 2θ angles of 7.97±0.20°, 9.08±0.20°, 11.66±0.20°, 12.47 ±0.20°, 15.83±0.20°, 17.98±0.20°, 20.47±0.20° and 20.74±0.20°.

In some embodiments of the present disclosure, the X-ray powder diffraction pattern of the above-mentioned crystalline form D shows characteristic diffraction peaks at 2θ angles of 7.97°, 9.08°, 11.66°, 12.47°, 15.83°, 17.98°, 20.47°, 20.74°, 23.37° and 25.04°

In some embodiments of the present disclosure, the X-ray powder diffraction pattern of the above-mentioned crystalline form D shows characteristic diffraction peaks at 2θ angles of 4.91°, 6.96°, 7.97°, 9.07°, 9.30°, 10.16°, 11.65°, 12.46°, 13.19°, 13.82°, 14.35°, 14.88°, 15.82°, 17.15°, 17.43°, 17.97°, 18.22°, 18.75°, 19.50°, 20.47°, 20.74°, 21.69°, 23.12°, 23.36°, 23.86°, 24.45°, 25.04°, 25.33°, 26.32°, 26.57°, 26.95°, 27.37°, 28.0 7°, 28.91°, 30.61°, 31.96°, 32.45°, 32.75°, 34.36°, 35.25°and 37.64°.

In some embodiments of the present disclosure, the XRPD pattern of the above-mentioned crystalline form D is shown in FIG. 10.

In some embodiments of the present disclosure, analysis data of the XRPD pattern of the above-mentioned crystalline form D are shown in Table 4:

Table 4. Analytical data of the XRPD pattern of the crystalline form D

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 4.913 | 17.9723 | 6.2 | 21 | 20.743 | 4.2787 | 21.3 |
| 2 | 6.963 | 12.6851 | 7.4 | 22 | 21.692 | 4.0935 | 12.5 |
| 3 | 7.974 | 11.0789 | 56 | 23 | 23.121 | 3.8437 | 14.2 |
| 4 | 9.077 | 9.7346 | 22.6 | 24 | 23.367 | 3.8038 | 20.4 |
| 5 | 9.303 | 9.4980 | 16.2 | 25 | 23.864 | 3.7256 | 19.9 |
| 6 | 10.165 | 8.6945 | 7.4 | 26 | 24.453 | 3.6373 | 18.8 |
| 7 | 11.655 | 7.5864 | 74.9 | 27 | 25.044 | 3.5527 | 20.1 |
| 8 | 12.469 | 7.0930 | 38.5 | 28 | 25.337 | 3.5123 | 5.3 |
| 9 | 13.196 | 6.7039 | 14.7 | 29 | 26.327 | 3.3824 | 9.4 |
| 10 | 13.823 | 6.4012 | 16.9 | 30 | 26.570 | 3.3520 | 11.4 |
| 11 | 14.358 | 6.1635 | 5.9 | 31 | 26.958 | 3.3047 | 4.4 |
| 12 | 14.887 | 5.9460 | 5.2 | 32 | 27.372 | 3.2557 | 11.7 |
| 13 | 15.825 | 5.5954 | 100 | 33 | 28.076 | 3.1756 | 8.6 |
| 14 | 17.158 | 5.1637 | 5.5 | 34 | 28.917 | 3.0851 | 10.7 |
| 15 | 17.432 | 5.0832 | 10.5 | 35 | 30.610 | 2.9182 | 8 |
| 16 | 17.979 | 4.9298 | 48.2 | 36 | 31.968 | 2.7972 | 7.4 |
| 17 | 18.221 | 4.8646 | 14.3 | 37 | 32.458 | 2.7562 | 5.6 |
| 18 | 18.756 | 4.7271 | 9.8 | 38 | 32.753 | 2.7320 | 3.9 |
| 19 | 19.504 | 4.5476 | 12.5 | 39 | 34.368 | 2.6072 | 5.8 |
| 20 | 20.472 | 4.3346 | 21.9 | 40 | 35.258 | 2.5434 | 4.1 |
| | | | | 41 | 37.642 | 2.3877 | 3.8 |

The ninth object of the present disclosure is to provide the crystalline form E of the compound represented by formula (I), which X-ray powder diffraction pattern shows characteristic diffraction peaks at 2θ angles of 12.43±0.20°, 12.70±0.20° and 17.94±0.20°.

In some embodiments of the present disclosure, the X-ray powder diffraction pattern of the above-mentioned crystalline form E shows characteristic diffraction peaks at 2θ angles of 9.02±0.20°, 10.15±0.20°, 12.43±0 .20°, 12.70 ±0.20°, 17.94±0.20°, 19.08±0.20°, 20.66±0.20° and 24.96±0.20°.

In some embodiments of the present disclosure, the X-ray powder diffraction pattern of the above-mentioned crystalline form E shows characteristic diffraction peaks at 2θ angles of 9.02±0.20°, 10.14±0.20°, 12.43±0.20°, 12.70 ±0.20°, 17.94±0.20°, 19.08±0.20°, 20.66±0.20°, 22.28±0.20°, 24.95±0.20° and 26.55±0.20°.

In some embodiments of the present disclosure, the X-ray powder diffraction pattern of the above-mentioned crystalline form E shows characteristic diffraction peaks at 2θ angles of 3.17°, 6.10°, 6.37°, 6.88°, 7.11°, 7.89°, 9.02°, 10.14°, 11.60°, 12.01°, 12.18°, 12.43°, 12.69°, 13.19°, 13.73°, 14.77°, 15.59°, 15.91°, 16.75°, 17.10°, 17.94°, 18.70°, 19.07°, 19.54°, 19.86°, 20.66°, 21.99°, 22.28°, 22.68°, 23.34°, 23.60°, 23.80°, 24.07°, 24.39°, 24.95°, 26.55°, 27.20°, 27.46°, 27.96°, 28.25°, 28.87°, 29.33°, 29.77°, 30.02°, 30.35°, 30.51°, 31.66°, 32.07°, 32.74°, 33.94°, 34.49°, 34.70°, 35.71°, 36.27°, 38.61° and 39.66°.

In some embodiments of the present disclosure, the XRPD pattern of the above-mentioned crystalline form E is shown in FIG. 11.

In some embodiments of the present disclosure, analysis data of the XRPD pattern of the above-mentioned crystalline form E are shown in Table 5:

Table 5. Analytical data of the XRPD pattern of crystalline form E

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 3.170 | 27.8488 | 3.9 | 29 | 22.687 | 3.9163 | 14.4 |
| 2 | 6.103 | 14.4689 | 4.1 | 30 | 23.349 | 3.8066 | 7.2 |
| 3 | 6.376 | 13.8499 | 22.9 | 31 | 23.603 | 3.7663 | 7.5 |
| 4 | 6.885 | 12.8276 | 9.5 | 32 | 23.806 | 3.7346 | 4.9 |
| 5 | 7.110 | 12.4219 | 15.3 | 33 | 24.077 | 3.6932 | 3.3 |
| 6 | 7.897 | 11.1859 | 3.3 | 34 | 24.396 | 3.6455 | 8.3 |
| 7 | 9.024 | 9.7911 | 51.6 | 35 | 24.956 | 3.5651 | 33.4 |
| 8 | 10.145 | 8.7119 | 25.4 | 36 | 26.553 | 3.3542 | 24.9 |
| 9 | 11.600 | 7.6221 | 4.9 | 37 | 27.207 | 3.2749 | 3.1 |
| 10 | 12.009 | 7.3637 | 6 | 38 | 27.466 | 3.2447 | 5.8 |
| 11 | 12.181 | 7.2600 | 10.4 | 39 | 27.964 | 3.1880 | 6 |
| 12 | 12.431 | 7.1143 | 100 | 40 | 28.251 | 3.1563 | 5.5 |
| 13 | 12.698 | 6.9654 | 71.7 | 41 | 28.877 | 3.0893 | 5 |
| 14 | 13.197 | 6.7032 | 6.3 | 42 | 29.332 | 3.0424 | 2.8 |
| 15 | 13.733 | 6.4427 | 7 | 43 | 29.771 | 2.9985 | 9.7 |
| 16 | 14.777 | 5.9897 | 4.3 | 44 | 30.020 | 2.9742 | 5.9 |
| 17 | 15.593 | 5.6781 | 10.5 | 45 | 30.359 | 2.9417 | 11.5 |
| 18 | 15.917 | 5.5632 | 23.1 | 46 | 30.515 | 2.9271 | 5.7 |
| 19 | 16.754 | 5.2874 | 2.8 | 47 | 31.660 | 2.8238 | 3.5 |
| 20 | 17.106 | 5.1794 | 4.2 | 48 | 32.079 | 2.7879 | 4.9 |
| 21 | 17.941 | 4.9401 | 94.9 | 49 | 32.742 | 2.7328 | 3.3 |
| 22 | 18.700 | 4.7412 | 7.5 | 50 | 33.946 | 2.6387 | 3.2 |
| 23 | 19.079 | 4.6479 | 64.1 | 51 | 34.493 | 2.5981 | 3.9 |
| 24 | 19.540 | 4.5393 | 3.8 | 52 | 34.702 | 2.5829 | 3.5 |
| 25 | 19.864 | 4.4660 | 2.9 | 53 | 35.715 | 2.5119 | 2.5 |
| 26 | 20.663 | 4.2951 | 35.8 | 54 | 36.272 | 2.4746 | 5.3 |
| 27 | 21.998 | 4.0373 | 18.2 | 55 | 38.612 | 2.3299 | 23.3 |
| 28 | 22.286 | 3.9858 | 23.8 | 56 | 39.665 | 2.2704 | 2.5 |

In some embodiments of the present disclosure, the differential scanning calorimetry curve of the above-mentioned crystalline form E has endothermic peaks at 151.1°C±3.0°C and 315.2±3.0°C respectively.

In some embodiments of the present disclosure, the DSC curve of the above-mentioned crystalline form E is shown in FIG. 12.

In some embodiments of the present disclosure, the thermogravimetric analysis curve of the above-mentioned crystalline form E shows a weight loss of 6.6% at 150.0°C±3.0°C .

In some embodiments of the present disclosure, the TGA spectrum of the above-mentioned crystalline form E is shown in FIG. 13.

The tenth object of the present disclosure is to provide the crystalline form F of the compound represented by formula (I), which X-ray powder diffraction pattern shows characteristic diffraction peaks at 2θ angles of 7.13±0.20°, 12.18 ±0.20° and 18.25±0.20°.

In some embodiments of the present disclosure, the X-ray powder diffraction pattern of the above-mentioned crystalline form F shows characteristic diffraction peaks at 2θ angles of 7.13±0.20°, 12.18±0.20°, 12.68±0.20°, 15.59 ±0.20°, 18.25±0.20°, 19.05±0.20°, 22.34±0.20° and 22.70±0.20°.

In some embodiments of the present disclosure, the X-ray powder diffraction pattern of the above-mentioned crystalline form F shows characteristic diffraction peaks at 2θ angles of 7.13±0.20°, 12.18±0.20°, 12.68±0.20°, 15.59 ±0.20°, 17.73±0.20°, 18.25±0.20°, 19.05±0.20°, 22.34±0.20°, 22.70±0.20° and 24.63±0.20°.

In some embodiments of the present disclosure, the X-ray powder diffraction pattern of the above-mentioned crystalline form F shows characteristic diffraction peaks at 2θ angles of 6.087°, 6.34°, 7.13°, 9.72°, 10.10°, 12.18°, 12.68°, 12.86°, 14.18°, 14.77° , 15.15°, 15.59°, 15.89°, 16.70°, 17.10°, 17.48°, 17.72°, 18.24°, 18.68°, 19.04°, 19.48°, 19.84°, 20.22°, 20.62°, 21.10°, 21.31°, 21.96°, 22.34°, 22.70°, 23.36°, 23.61°, 23.84°, 24.10°, 24.62°, 25.15°, 25.31°, 25.71°, 26.32°, 26.49°, 26.68°, 27.03°, 27.20° , 27.84°, 28.07°, 28.28°, 29.37°, 29.77°, 29.99°, 30.32°, 30.50°, 31.92°, 32.58°, 32.99°, 32.99°, 34.52°, 35.08°, 35.77°, 36.14°, 36.48°, 37.75°, 38.22°, 38.61°, 39.44° and 39.54°.

In some embodiments of the present disclosure, the XRPD pattern of the above-mentioned crystalline form F is shown in FIG. 14.

In some embodiments of the present disclosure, analysis data of the XRPD pattern of the above-mentioned crystalline form F are shown in Table 6:

Table 6. Analytical data of the XRPD pattern of crystalline form F

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.087 | 14.5081 | 8.6 | 31 | 23.619 | 3.7638 | 12.1 |
| 2 | 6.340 | 13.9286 | 18.1 | 32 | 23.840 | 3.7293 | 41 |
| 3 | 7.133 | 12.3834 | 90.7 | 33 | 24.109 | 3.6883 | 33.7 |
| 4 | 9.727 | 9.0857 | 27.2 | 34 | 24.628 | 3.6118 | 49.8 |
| 5 | 10.106 | 8.7456 | 13.9 | 35 | 25.156 | 3.5371 | 12.4 |
| 6 | 12.183 | 7.2588 | 71.4 | 36 | 25.312 | 3.5157 | 6.3 |
| 7 | 12.680 | 6.9752 | 67 | 37 | 25.712 | 3.4619 | 7.7 |
| 8 | 12.869 | 6.8732 | 40.3 | 38 | 26.323 | 3.3830 | 7.6 |
| 9 | 14.187 | 6.2375 | 23.1 | 39 | 26.498 | 3.3609 | 15.8 |
| 10 | 14.778 | 5.9895 | 15.8 | 40 | 26.686 | 3.3378 | 26 |
| 11 | 15.158 | 5.8400 | 10.8 | 41 | 27.037 | 3.2952 | 8.8 |
| 12 | 15.590 | 5.6793 | 47.7 | 42 | 27.205 | 3.2752 | 9.5 |
| 13 | 15.896 | 5.5705 | 33 | 43 | 27.846 | 3.2012 | 5.8 |
| 14 | 16.700 | 5.3043 | 17.4 | 44 | 28.077 | 3.1754 | 21.6 |
| 15 | 17.102 | 5.1804 | 12.6 | 45 | 28.284 | 3.1526 | 16.4 |
| 16 | 17.480 | 5.0694 | 24.8 | 46 | 29.370 | 3.0385 | 15.5 |
| 17 | 17.726 | 4.9994 | 45.1 | 47 | 29.770 | 2.9986 | 9.2 |
| 18 | 18.247 | 4.8579 | 100 | 48 | 29.996 | 2.9765 | 14.9 |
| 19 | 18.684 | 4.7452 | 30.1 | 49 | 30.329 | 2.9446 | 17.8 |
| 20 | 19.048 | 4.6554 | 57.4 | 50 | 30.508 | 2.9277 | 11.5 |
| 21 | 19.482 | 4.5526 | 29.8 | 51 | 31.928 | 2.8007 | 5.7 |
| 22 | 19.845 | 4.4702 | 34.9 | 52 | 32.582 | 2.7459 | 5.5 |
| 23 | 20.228 | 4.3864 | 7.4 | 53 | 32.990 | 2.7129 | 6.6 |
| 24 | 20.624 | 4.3031 | 32.1 | 54 | 32.990 | 2.7129 | 6.6 |
| 25 | 21.108 | 4.2055 | 13.3 | 55 | 34.524 | 2.5958 | 8.5 |
| 26 | 21.311 | 4.1659 | 25 | 56 | 35.085 | 2.5556 | 7.2 |
| 27 | 21.961 | 4.0439 | 31.4 | 57 | 35.777 | 2.5077 | 6.6 |
| 28 | 22.340 | 3.9763 | 58.7 | 58 | 36.144 | 2.4831 | 12 |
| 29 | 22.702 | 3.9137 | 58.2 | 59 | 36.485 | 2.4606 | 6.3 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 30 | 23.367 | 3.8037 | 22.2 | 60 | 37.757 | 2.3807 | 8.2 |
| | | | | | 38.223 | 2.3527 | 12.7 |
| | | | | | 38.611 | 2.3299 | 11.2 |
| | | | | | 39.449 | 2.2823 | 4.7 |
| | | | | | 39.548 | 2.2768 | 5.3 |

In some embodiments of the present disclosure, the differential scanning calorimetry curve of the above-mentioned crystalline form F shows endothermic peaks at 158.9°C±3.0°C and 315.6±3.0°C respectively.

In some embodiments of the present disclosure, the DSC curve of the above-mentioned crystalline form F is shown in FIG. 15.

In some embodiments of the present disclosure, the thermogravimetric analysis curve of the above-mentioned crystalline form F shows a weight loss of 13.5% at 150.0°C±3.0°C .

In some embodiments of the present disclosure, the TGA spectrum of the above-mentioned crystalline form F is shown in FIG. 16.

The eleventh object of the present disclosure is to provide the crystalline form G of the compound represented by formula (I), which X-ray powder diffraction pattern shows characteristic diffraction peaks at 2θ angles of 12.15±0.20°, 12.74±0.20° and 15.37±0.20°,

In some embodiments of the present disclosure, the X-ray powder diffraction pattern of the above-mentioned crystalline form G shows characteristic diffraction peaks at 2θ angles of 7.09±0.20°, 12.15±0.20°, 12.74±0.20°, 15.37 ±0.20°, 17.63±0.20°, 18.15±0.20°, 22.57±0.20° and 22.76±0.20°.

In some embodiments of the present disclosure, the X-ray powder diffraction pattern of the above-mentioned crystalline form G shows characteristic diffraction peaks at 2θ angles of 7.09±0.20°, 12.15±0.20°, 12.74±0.20°, 15.37 ±0.20°, 17.63±0.20°, 18.15±0.20°, 22.21±0.20°, 22.57±0.20°, 22.76±0.20° and 23.64±0.20°.

In some embodiments of the present disclosure, the X-ray powder diffraction pattern of the above-mentioned crystalline form G shows characteristic diffraction at 2θ angles of 6.012°, 6.33°, 7.09°, 9.66°, 10.03°, 10.96°, 11.49°, 12.14°, 12.73°, 14.11°, 14.31°, 14.68°, 15.04°, 15.37°, 15.78°, 16. 56°, 16.92°, 17.33°, 17.63°, 18.14°, 18.54°, 18.98°, 19.31°, 19.74°, 20.32°, 20.60°, 21.06°, 21.93°, 22.20°, 22.56°, 22.76°, 23.25°, 23.63°, 23.82°, 24.44°, 24.64°, 26.66°, 26.95°, 27.90°, 28.28°, 29.69°, 29.98°, 30.35°, 31.31°, 31.62°, 32.93°, 34.22°, 34. 84°, 36.85°, 38.09° and 38.69°.

In some embodiments of the present disclosure, the XRPD pattern of the above-mentioned crystalline form G is shown in FIG. 17.

In some embodiments of the present disclosure, analysis data of the XRPD pattern of the above-mentioned crystalline form G are shown in Table 7:

Table 7. Analytical data of the XRPD pattern of crystalline form G

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.012 | 14.6877 | 25.5 | 31 | 22.761 | 3.9037 | 31.3 |
| 2 | 6.339 | 13.9309 | 8.1 | 32 | 23.257 | 3.8215 | 21.7 |
| 3 | 7.094 | 12.4503 | 32 | 33 | 23.636 | 3.7611 | 28 |
| 4 | 9.666 | 9.1431 | 16.3 | 34 | 23.828 | 3.7312 | 14.7 |
| 5 | 10.032 | 8.8095 | 22.2 | 35 | 24.440 | 3.6391 | 18.3 |
| 6 | 10.964 | 8.0629 | 6.8 | 36 | 24.647 | 3.6090 | 14.7 |
| 7 | 11.499 | 7.6891 | 10 | 37 | 26.668 | 3.3399 | 17.6 |
| 8 | 12.148 | 7.2798 | 100 | 38 | 26.953 | 3.3052 | 9.1 |
| 9 | 12.738 | 6.9439 | 64.4 | 39 | 27.909 | 3.1941 | 7.4 |
| 10 | 14.111 | 6.2712 | 6.8 | 40 | 28.281 | 3.1530 | 5.6 |
| 11 | 14.318 | 6.1810 | 10.4 | 41 | 29.697 | 3.0058 | 6.2 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 12 | 14.687 | 6.0263 | 15.9 | 42 | 29.983 | 2.9778 | 12.7 |
| 13 | 15.041 | 5.8854 | 11.6 | 43 | 30.356 | 2.9420 | 7.4 |
| 14 | 15.371 | 5.7598 | 47.1 | 44 | 31.314 | 2.8541 | 5.6 |
| 15 | 15.789 | 5.6083 | 20.2 | 45 | 31.623 | 2.8270 | 5.1 |
| 16 | 16.567 | 5.3467 | 9.5 | 46 | 32.938 | 2.7170 | 5.9 |
| 17 | 16.929 | 5.2329 | 6.2 | 47 | 34.228 | 2.6176 | 5.1 |
| 18 | 17.331 | 5.1124 | 23.9 | 48 | 34.846 | 2.5725 | 6.2 |
| 19 | 17.633 | 5.0255 | 35.2 | 49 | 36.858 | 2.4366 | 6.4 |
| 20 | 18.148 | 4.8842 | 37.1 | 50 | 38.095 | 2.3603 | 5.6 |
| 21 | 18.546 | 4.7801 | 11.1 | 51 | 38.692 | 2.3252 | 6.8 |
| 22 | 18.987 | 4.6703 | 19 | 52 | 31.623 | 2.8270 | 5.1 |
| 23 | 19.311 | 4.5926 | 15 | 53 | 32.938 | 2.7170 | 5.9 |
| 24 | 19.745 | 4.4926 | 13.5 | 54 | 34.228 | 2.6176 | 5.1 |
| 25 | 20.326 | 4.3654 | 18.9 | 55 | 34.846 | 2.5725 | 6.2 |
| 26 | 20.607 | 4.3067 | 26.5 | 56 | 36.858 | 2.4366 | 6.4 |
| 27 | 21.062 | 4.2146 | 10.1 | 57 | 38.095 | 2.3603 | 5.6 |
| 28 | 21.936 | 4.0486 | 15.3 | 58 | 38.692 | 2.3252 | 6.8 |
| 29 | 22.209 | 3.9994 | 30.2 | | | | |
| 30 | 22.568 | 3.9365 | 30.4 | | | | |

In some embodiments of the present disclosure, the differential scanning calorimetry curve of the above-mentioned crystalline form G shows endothermic peaks at 157.5°C±3.0°C and 316.6±3.0°C respectively.

In some embodiments of the present disclosure, the DSC curve of the above-mentioned crystalline form G is shown in FIG. 18.

In some embodiments of the present disclosure, the thermogravimetric analysis curve of the above-mentioned crystalline form G shows a weight loss of 11.9% at 175.00°C±3.0°C .

In some embodiments of the present disclosure, the TGA spectrum of the above-mentioned crystalline form G is shown in FIG. 19.

The twelfth object of the present disclosure is to provide the crystalline form H of the compound represented by formula (I), which X-ray powder diffraction pattern shows characteristic diffraction peaks at 2θ angles of 9.59±0.20°, 10.18 ±0.20° and 18.66±0.20°.

In some embodiments of the present disclosure, the X-ray powder diffraction pattern of the above-mentioned crystalline form H shows characteristic diffraction peaks at 2θ angles of 9.59±0.20°, 10.18±0.20°, 11.2 9±0.20°, 11.83 ±0.20°, 15.57±0.20°, 18.66±0.20°, 20.13±0.20° and 22.80±0.20°.

In some embodiments of the present disclosure, the X-ray powder diffraction pattern of the above-mentioned crystalline form H shows characteristic diffraction peaks at 2θ angles of 9.59±0.20°, 10.18±0.20°, 11.29±0.20°, 11.83 ±0.20°, 12.77±0.20°, 15.57±0.20°, 18.66±0.20°, 20.13±0.20°, 22.80±0.20° and 25.12±0.20°.

In some embodiments of the present disclosure, the X-ray powder diffraction pattern of the above-mentioned crystalline form H shows characteristic diffraction peaks at 2θ angles of 9.594°, 10.18°, 11.29°, 11.82°, 12.37°, 12.77°, 13.93°, 14.16°, 15.23°, 15.57°, 16.22°, 16.44°, 17.65°, 17.96°, 18.66°, 19.22°, 19.67°, 20.12°, 20.93°, 21.21°, 22.79°, 23.74°, 24.28°, 25.12°, 26.26°, 26.87°, 27.37°, 27.64°, 28.18°, 28.38°, 29.01°, 29.59°, 30.15°, 30.30°, 31.29°, 34.41°, 35.59° and 35.94°.

In some embodiments of the present disclosure, the XRPD pattern of the above-mentioned crystalline form H is shown in FIG. 20.

In some embodiments of the present disclosure, analysis data of the XRPD pattern of the above-mentioned crystalline form H are shown in Table 8:

Table 8. Analytical data of the XRPD pattern of crystalline form H

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 9.594 | 9.2112 | 67.6 | 21 | 22.797 | 3.8975 | 35.9 |
| 2 | 10.184 | 8.6783 | 79.7 | 22 | 23.748 | 3.7436 | 5.5 |
| 3 | 11.291 | 7.8301 | 31.8 | 23 | 24.281 | 3.6626 | 4.8 |
| 4 | 11.826 | 7.4773 | 34.9 | 24 | 25.124 | 3.5416 | 17.5 |
| 5 | 12.374 | 7.1472 | 5.2 | 25 | 26.264 | 3.3903 | 10.1 |
| 6 | 12.775 | 6.9236 | 19.8 | 26 | 26.879 | 3.3142 | 12.6 |
| 7 | 13.936 | 6.3492 | 9.7 | 27 | 27.370 | 3.2559 | 4.7 |
| 8 | 14.166 | 6.2466 | 7.4 | 28 | 27.645 | 3.2240 | 5.1 |
| 9 | 15.237 | 5.8100 | 15.8 | 29 | 28.189 | 3.1631 | 4.4 |
| 10 | 15.574 | 5.6850 | 40.4 | 30 | 28.385 | 3.1417 | 4.4 |
| 11 | 16.226 | 5.4580 | 5.7 | 31 | 29.013 | 3.0751 | 4.2 |
| 12 | 16.448 | 5.3848 | 7.7 | 32 | 29.597 | 3.0157 | 8.4 |
| 13 | 17.659 | 5.0182 | 8.4 | 33 | 30.153 | 2.9614 | 7.9 |
| 14 | 17.968 | 4.9326 | 4.5 | 34 | 30.304 | 2.9469 | 7 |
| 15 | 18.663 | 4.7506 | 100 | 35 | 31.296 | 2.8558 | 4.3 |
| 16 | 19.227 | 4.6124 | 5.1 | 36 | 34.417 | 2.6036 | 6 |
| 17 | 19.674 | 4.5086 | 4.9 | 37 | 35.591 | 2.5204 | 3.3 |
| 18 | 20.129 | 4.4077 | 56.6 | 38 | 35.945 | 2.4964 | 3 |
| 19 | 20.934 | 4.2399 | 7 | 39 | | | |
| 20 | 21.216 | 4.1842 | 13.6 | 40 | | | |

The thirteenth object of the present disclosure is to provide the crystalline form I of the compound represented by formula (I), which X-ray powder diffraction pattern shows characteristic diffraction peaks at 2θ angles of 9.06±0.20° 12.45 ±0.20°and 17.99±0.20°.

In some embodiments of the present disclosure, the X-ray powder diffraction pattern of the above-mentioned crystalline form I shows characteristic diffraction peaks at 2θ angles of 9.06±0.20°, 10.18±0.20°, 12.45±0. 20°, 13.18 ±0.20°, 17.99±0.20° ,20.79±0.20°, 24.42±0.20°, 25.05±0.20° and 26.57±0.20°.

In some embodiments of the present disclosure, the X-ray powder diffraction pattern of the above-mentioned crystalline form I shows characteristic diffraction peaks at 2θ angles of 9.06±0.20°, 10.18±0.20°, 12.45±0.20°, 17.99 ±0.20°, 18.74±0.20° , 20.53±0.20° , 20.79±0.20°, 24.42±0.20°, 25.05±0.20° and 26.57±0.20°.

In some embodiments of the present disclosure, the X-ray powder diffraction pattern of the above-mentioned crystalline form I shows characteristic diffraction peaks at 2θ angles of 6.041°, 6.90°, 9.06°, 9.32°, 10.18°, 12.45°, 13.17°, 13.72°, 15.55°, 15.99°, 16.70°, 17.17°, 17.99°, 18.74°, 19.55°, 20.52°, 20.79°, 22.01°, 22.41°, 22.68°, 24.41°, 25.04°, 26.34°, 26.57°, 27.48°, 28.01°, 28.89°, 29.79°, 30.64°, 31.63°, 32.86°, 34.55°, 34.67°and 36.24° 。

In some embodiments of the present disclosure, the XRPD pattern of the above-mentioned crystalline form I is shown in FIG. 21.

In some embodiments of the present disclosure, analysis data of the XRPD pattern of the above-mentioned crystalline form I are shown in Table 9:

Table 9. Analytical data of the XRPD pattern of crystalline form I

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 6.041 | 14.619 | 3.6 | 19 | 22.418 | 3.9626 | 6.6 |
| 2 | 6.903 | 12.7942 | 7.4 | 20 | 22.689 | 3.9159 | 4.9 |

(continued)

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 3 | 9.061 | 9.7512 | 55.7 | 21 | 24.418 | 3.6424 | 23.1 |
| 4 | 9.326 | 9.4753 | 4.2 | 22 | 25.045 | 3.5526 | 46.7 |
| 5 | 10.182 | 8.6801 | 36 | 23 | 26.343 | 3.3804 | 16.6 |
| 6 | 12.452 | 7.1026 | 70.2 | 24 | 26.572 | 3.3518 | 28.9 |
| 7 | 13.177 | 6.7133 | 18.4 | 25 | 27.488 | 3.2421 | 14.6 |
| 8 | 13.727 | 6.4455 | 11.3 | 26 | 28.016 | 3.1823 | 4.4 |
| 9 | 15.555 | 5.6920 | 4.2 | 27 | 28.897 | 3.0872 | 15.9 |
| 10 | 15.995 | 5.5366 | 16.4 | 28 | 29.792 | 2.9964 | 9.1 |
| 11 | 16.706 | 5.3025 | 3.6 | 29 | 30.648 | 2.9147 | 6.1 |
| 12 | 17.178 | 5.1579 | 12.9 | 30 | 31.637 | 2.8258 | 4.9 |
| 13 | 17.997 | 4.9249 | 100 | 31 | 32.860 | 2.7233 | 7 |
| 14 | 18.742 | 4.7308 | 18.2 | 32 | 34.555 | 2.5936 | 4.4 |
| 15 | 19.555 | 4.5357 | 3.5 | 33 | 34.670 | 2.5852 | 3.6 |
| 16 | 20.528 | 4.3229 | 17.8 | 34 | 36.244 | 2.4764 | 3 |
| 17 | 20.795 | 4.2680 | 32.3 | | | | |
| 18 | 22.011 | 4.0350 | 5.5 | | | | |

In some embodiments of the present disclosure, the differential scanning calorimetry curve of the above-mentioned crystalline form I shows an endothermic peak at 316.0°C±3.0°C .

In some embodiments of the present disclosure, the DSC curve of the above-mentioned crystalline form I is shown in FIG. 22.

In some embodiments of the present disclosure, the thermogravimetric analysis curve of the above-mentioned crystalline form I shows a weight loss of 1.1% at 150°C±3.0°C .

In some embodiments of the present disclosure, the TGA curve of the above-mentioned crystalline form I is shown in FIG. 23.

The fourteenth object of the present disclosure is to provide the crystalline form J of the compound represented by formula (I), which X-ray powder diffraction pattern shows characteristic diffraction peaks at 2θ angles of 9.15±0.20°, 18.08 ±0.20° and 19.23±0.20°.

In some embodiments of the present disclosure, the X-ray powder diffraction pattern of the above-mentioned crystalline form J shows characteristic diffraction peaks at 2θ angles of 9.15±0.20°, 10.66±0.20°, 14.91±0.20°, 15.75 ±0.20°, 18.08±0.20°, 18.28±0.20°, 19.23±0.20° and 28.19±0.20°.

In some embodiments of the present disclosure, the X-ray powder diffraction pattern of the above-mentioned crystalline form J shows characteristic diffraction peaks at 2θ angles of 9.15±0.20°, 10.66±0.20°, 14.91±0.20°, 15.75 ±0.20°, 18.08±0.20°, 18 .28±0.20°, 19.23±0.20°, 23.27±0.20°, 23.66±0.20° and 28.19±0.20°.

In some embodiments of the present disclosure, the X-ray powder diffraction pattern of the above-mentioned crystalline form J shows characteristic diffraction peaks at 2θ angles of 7.47°, 9. 15°, 10.66°, 10.86°, 11.22°, 11.77°, 12.62°, 12.85°, 13.72°, 14.91°, 15.75°, 18.07°, 18.28°, 19.23°, 19.90°, 20.60°, 21.00°, 21.38°, 21.86°, 22.11°, 22.46°, 23.01°, 23.27°, 23.65°, 24.05°, 24.35°, 25.13°, 25.29°, 25.79°, 26.43°, 27.29°, 27.54°, 28.19°, 29. 21°, 30.57°, 32.36°, 32.68°, 32.82°, 34.82°, 35.83°, 36.64°, 36.99°, 37.41°and 38.28°.

In some embodiments of the present disclosure, the XRPD pattern of the above-mentioned crystalline form J is shown in FIG. 24.

In some embodiments of the present disclosure, analysis data of the XRPD pattern of the above-mentioned crystalline form J are shown in Table 10:

Table 10. Analytical data of the XRPD pattern of crystalline form J

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 7.472 | 11.8222 | 6.6 | 25 | 24.054 | 3.6966 | 2.7 |
| 2 | 9.151 | 9.6559 | 80.3 | 26 | 24.359 | 3.6511 | 9.2 |
| 3 | 10.662 | 8.2908 | 68 | 27 | 25.138 | 3.5397 | 2.8 |
| 4 | 10.867 | 8.1346 | 13.5 | 28 | 25.298 | 3.5177 | 6 |
| 5 | 11.226 | 7.8756 | 9.2 | 29 | 25.795 | 3.4510 | 15.3 |
| 6 | 11.778 | 7.5077 | 11.1 | 30 | 26.435 | 3.3689 | 6.7 |
| 7 | 12.624 | 7.0064 | 12.9 | 31 | 27.298 | 3.2643 | 5.7 |
| 8 | 12.852 | 6.8825 | 4.4 | 32 | 27.547 | 3.2353 | 14.9 |
| 9 | 13.727 | 6.4458 | 12 | 33 | 28.193 | 3.1626 | 19.7 |
| 10 | 14.912 | 5.9358 | 69.2 | 34 | 29.218 | 3.0540 | 10.8 |
| 11 | 15.752 | 5.6212 | 25.2 | 35 | 30.573 | 2.9216 | 5.4 |
| 12 | 18.077 | 4.9032 | 100 | 36 | 32.360 | 2.7643 | 2.4 |
| 13 | 18.281 | 4.8489 | 23.8 | 37 | 32.685 | 2.7375 | 4.7 |
| 14 | 19.233 | 4.6111 | 72.2 | 38 | 32.828 | 2.7259 | 4.1 |
| 15 | 19.905 | 4.4568 | 9.6 | 39 | 34.824 | 2.5741 | 2.5 |
| 16 | 20.609 | 4.3062 | 10.7 | 40 | 35.832 | 2.5040 | 4.6 |
| 17 | 21.008 | 4.2252 | 6.9 | 41 | 36.647 | 2.4501 | 2.2 |
| 18 | 21.386 | 4.1515 | 8.6 | 42 | 36.997 | 2.4277 | 2.9 |
| 19 | 21.865 | 4.0615 | 13.8 | 43 | 37.417 | 2.4014 | 2.5 |
| 20 | 22.110 | 4.0171 | 7.5 | 44 | 38.289 | 2.3488 | 1.9 |
| 21 | 22.460 | 3.9554 | 10.4 | | | | |
| 22 | 23.012 | 3.8616 | 3.8 | | | | |
| 23 | 23.276 | 3.8183 | 18.2 | | | | |
| 24 | 23.657 | 3.7577 | 17 | | | | |

In some embodiments of the present disclosure, the differential scanning calorimetry curve of the above-mentioned crystalline form J shows endothermic peaks at 135.3°C±3.0°C and 315.2±3.0°C respectively.

In some embodiments of the present disclosure, the DSC curve of the above-mentioned crystalline form J is shown in FIG. 25.

In some embodiments of the present disclosure, the thermogravimetric analysis curve of the above-mentioned crystalline form J shows a weight loss of 15.3% at 170.0°C±3.0°C .

In some embodiments of the present disclosure, the TGA curve of the above-mentioned crystalline form J is shown in FIG. 26.

The fifteenth object of the present disclosure is to provide the K crystalline form of the compound represented by formula (I), which X-ray powder diffraction pattern shows characteristic diffraction peaks at 2θ angles of 9.11±0.20° 16.30 ±0.20°and 18.19± 0.20°.

In some embodiments of the present disclosure, the X-ray powder diffraction pattern of the above-mentioned crystalline form K shows characteristic diffraction peaks at 2θ angles of 9.11±0.20°, 16.30±0.20°, 17.15±0.20°, 18.19 ±0.20°, 19.60±0.20°, 24.45±0.20°, 25.01±0.20° and 27.39±0.20°.

In some embodiments of the present disclosure, the X-ray powder diffraction pattern of the above-mentioned crystalline form K shows characteristic diffraction peaks at 2θ angles of 9.11±0.20°, 10.41±0.20°, 16.30±0.20°, 17.15 ±0.20°, 18.19±0.20°, 19.60±0.20°, 24.45±0.20°, 25.01±0.20°, 27.39±0.20° and 36.75±0.20°.

In some embodiments of the present disclosure, the X-ray powder diffraction pattern of the above-mentioned crystalline form K shows characteristic diffraction peaks at 2θ angles of 8.611°, 9.11°, 10.15°, 10.41°, 10.88°, 12.58°,

13.64°, 14.96°, 15.13°, 16.29°, 17.15°, 17.32°, 18.18°, 18.72°, 19.59°, 19.92°, 20.17°, 20.45°, 20.83°, 22.28°, 22.47°, 22.89°, 23.78°, 24.07°, 24.45°, 25.01°, 26.59°, 27.38°, 28.22°, 28.82°, 29.81°, 30.40°, 31.12°, 34.11°, 34.66°, 35.96°, 36.74° and 38.09°

In some embodiments of the present disclosure, the XRPD pattern of the above-mentioned crystalline form K is shown in FIG. 27.

In some embodiments of the present disclosure, analysis data of the XRPD pattern of the above-mentioned crystalline form K are shown in Table 11:

Table 11. Analytical data of the XRPD pattern of crystalline form K

| No. | 2θ angle (°) | Interplanar spacing (Å) | Relative intensity (%) | No. | 2θ angle (°) | Interplanar spacing (Å) | Relative intensity (%) |
|---|---|---|---|---|---|---|---|
| 1 | 8.611 | 10.2602 | 2.8 | 20 | 22.287 | 3.9857 | 3.8 |
| 2 | 9.117 | 9.6915 | 100 | 21 | 22.476 | 3.9526 | 2.4 |
| 3 | 10.150 | 8.7079 | 3.1 | 22 | 22.897 | 3.8808 | 2.7 |
| 4 | 10.415 | 8.4869 | 10.1 | 23 | 23.787 | 3.7376 | 1.9 |
| 5 | 10.885 | 8.1212 | 1.5 | 24 | 24.073 | 3.6938 | 4.2 |
| 6 | 12.588 | 7.0259 | 9.2 | 25 | 24.454 | 3.6371 | 19.3 |
| 7 | 13.647 | 6.4833 | 2.2 | 26 | 25.011 | 3.5573 | 53.6 |
| 8 | 14.964 | 5.9155 | 1.5 | 27 | 26.591 | 3.3494 | 1.8 |
| 9 | 15.136 | 5.8485 | 1.5 | 28 | 27.388 | 3.2537 | 30.3 |
| 10 | 16.297 | 5.4344 | 99.4 | 29 | 28.225 | 3.1592 | 2.9 |
| 11 | 17.155 | 5.1646 | 11.9 | 30 | 28.825 | 3.0947 | 2.6 |
| 12 | 17.329 | 5.1130 | 3.3 | 31 | 29.814 | 2.9943 | 3.5 |
| 13 | 18.189 | 4.8732 | 55.7 | 32 | 30.406 | 2.9373 | 2.4 |
| 14 | 18.729 | 4.7340 | 2.6 | 33 | 31.126 | 2.8710 | 1.5 |
| 15 | 19.595 | 4.5267 | 11.5 | 34 | 34.119 | 2.6257 | 6 |
| 16 | 19.923 | 4.4528 | 2.3 | 35 | 34.666 | 2.5855 | 1.3 |
| 17 | 20.171 | 4.3987 | 2.3 | 36 | 35.963 | 2.4951 | 2.9 |
| 18 | 20.457 | 4.3378 | 1.9 | 37 | 36.747 | 2.4437 | 10.8 |
| 19 | 20.835 | 4.2599 | 5.9 | 38 | 38.098 | 2.3601 | 1.5 |

The present disclosure also provides use of crystalline form A, crystalline form B, crystalline form C, crystalline form D, crystalline form E, crystalline form F, crystalline form G, crystalline form H, crystalline form I, crystalline form J or crystalline form K of the compound in the manufacture of a medicament for treating a metabolic disease.

Preferably, the medicament is used as a thyroid hormone β receptor agonist.

Preferably, the metabolic disease includes obesity, hyperlipidemia, hypercholesterolemia, diabetes, hepatic steatosis, nonalcoholic steatohepatitis, atherosclerosis, cardiovascular diseases, thyroid diseases, and tumor in intrahepatic bile duct. Preferably, the thyroid diseases include thyroid cancer and hypothyroidism.

The present disclosure also provides a medicament, which comprises crystalline form A, crystalline form B, crystalline form C, crystalline form D, crystalline form E, crystalline form F, crystalline form G, crystalline form H, crystalline form I, crystalline form J or crystalline form K of the compound as an active ingredient.

Preferably, the medicament is a thyroid hormone β receptor agonist. Further, the medicament also comprises a pharmaceutically acceptable excipient.

By adopting the above-mentioned technical solutions, the present disclosure produces the following beneficial effects as compared to the prior art:

1. The present disclosure develops a new 2-pyridone derivative, which has high activity and selectivity for thyroid hormone β receptor and can be used to treat a metabolic disease, particularly for treating a disease related to thyroid hormone receptor.

In vitro enzyme activity test of thyroid receptor β (THRβ) showed that the $EC_{50}$ value of the compound represented by formula (I) was 0.04596 μM, indicating that the compound can promote the binding of THRβ and its co-activating peptide, and has potential agonist activity on THRβ at the same time. Cytological activity test of thyroid receptor β (THRβ) showed that the $EC_{50}$ of the compound represented by formula (I) was 0.214 μM at the cellular level, indicating that the compound has good agonist activity for thyroid receptor β (THRβ). Pharmacokinetic evaluation in rats showed that the compound represented by formula (I) had low blood drug concentration and relatively high liver drug concentration in rats, so the compound represented by formula (I) has good liver targeting properties.

2. In the present disclosure a series of crystalline forms of the compound, including crystalline forms A to K were also studied, which has the advantages of good stability, low hygroscopicity and being less affected by heat. Among them, the crystalline form A of the compound represented by formula (I) has good stability and low hygroscopicity under high temperature, high humidity, long-term and accelerated conditions.

The specific embodiments of the present disclosure will be described in further detail below in conjunction with the accompanying drawings.

**BRIEF DESCRIPTION OF THE DRAWINGS**

FIG. 1 is the Cu-kα radiated XRPD pattern of the crystalline form A of the compound represented by formula (I);

FIG. 2 is the DSC curve of the crystalline form A of the compound represented by formula (I);

FIG. 3 is the TGA curve of the crystalline form A of the compound represented by formula (I);

FIG. 4 is the Cu-kα radiated XRPD pattern of the crystalline form B of the compound represented by formula (I);

FIG. 5 is the DSC curve of the crystalline form B of the compound represented by formula (I);

FIG. 6 is the TGA curve of the crystalline form B of the compound represented by formula (I);

FIG. 7 is the Cu-kα radiated XRPD pattern of the crystalline form C of the compound represented by formula (I);

FIG. 8 is the DSC curve of the crystalline form C of the compound represented by formula (I);

FIG. 9 is the TGA curve of the crystalline form C of the compound represented by formula (I);

FIG. 10 is the Cu-kα radiated XRPD pattern of the crystalline form D of the compound represented by formula (I);

FIG. 11 is the Cu-kα radiated XRPD pattern of the crystalline form E of the compound represented by formula (I);

FIG. 12 is the DSC curve of the crystalline form E of the compound represented by formula (I);

FIG. 13 is the TGA curve of the crystalline form E of the compound represented by formula (I);

FIG. 14 is the Cu-kα radiated XRPD pattern of the crystalline form F of the compound represented by formula (I);

FIG. 15 is the DSC curve of the crystalline form F of the compound represented by formula (I);

FIG. 16 is the TGA curve of the crystalline form F of the compound represented by formula (I);

FIG. 17 is the Cu-kα radiated XRPD pattern of the crystalline form G of the compound represented by formula (I);

FIG. 18 is the DSC curve of the crystalline form G of the compound represented by formula (I);

FIG. 19 is the TGA curve of the crystalline form G of the compound represented by formula (I);

FIG. 20 is the Cu-kα radiated XRPD pattern of the crystalline form H of the compound represented by formula (I);

FIG. 21 is the Cu-kα radiated XRPD pattern of the crystalline form I of the compound represented by formula (I);

FIG. 22 is the DSC curve of the crystalline form I of the compound represented by formula (I);

FIG. 23 is the TGA curve of the crystalline form I of the compound represented by formula (I);

FIG. 24 is the Cu-kα radiated XRPD pattern of the crystalline form J of the compound represented by formula (I) in;

FIG. 25 is the DSC curve of the crystalline form J of the compound represented by formula (I);

FIG. 26 is the TGA curve of the crystalline form J of the compound represented by formula (I);

FIG. 27 is the Cu-kα radiated XRPD pattern of the crystalline form K of the compound represented by formula (I);

FIG. 28 is the DVS graph of the crystalline form A of the compound represented by formula (I).

It should to be noted that these drawings and the word description are provided to explain the concept of the present disclosure by referring to particular examples for those skilled in the art, but are not intended to limit the scope of the concept of the present disclosure in any way.

**DETAILED DESCRIPTION**

In order to make the purposes, technical solutions and advantages of examples of the present disclosure clearer, hereafter the technical solutions in the examples will be described clearly and completely in conjunction with the drawings. The following examples are given to describe the present disclosure, and are not intended to limit the scope of the present disclosure.

Definition and description

Unless otherwise specified, the following terms and phrases used herein are intended to have the following meanings. A particular phrase or term should not be considered indefinite or unclear without a specific definition, but should be understood in its ordinary meaning. When a trade name appears herein, it is intended to refer to its corresponding commercial product or its active ingredient.

The intermediate compounds of the present disclosure can be prepared by a variety of synthetic methods well-known to those skilled in the art, including the specific embodiments given below, the embodiments formed by these specific embodiments in combination with other chemical synthesis methods, as well as their equivalent alternative methods well-known to those skilled in the art. The preferred embodiments include, but are not limited to, the examples of the present disclosure.

The chemical reactions of specific embodiments of the present disclosure are carried out in appropriate solvents suitable for the chemical changes of the present disclosure and their required reagents and materials. In order to obtain the compounds of the present disclosure, it is sometimes necessary for those skilled in the art to modify or select the synthetic steps or reaction schemes on the basis of the existing embodiments.

The present disclosure will be specifically described below by means of examples, which do not imply any limitation of the present disclosure.

All solvents used in the present disclosure are commercially available and used without further purification.

The solvents used in the present disclosure are commercially available. The following abbreviations are used in the present disclosure: EtOH represents ethanol; MeOH represents methanol; EtOAc represents ethyl acetate; DCM represents dichloromethane; mp represents melting point; THF represents tetrahydrofuran; $K_2CO_3$ represents potassium carbonate; DMF represents N,N-dimethylformamide.

Compounds are named according to conventional nomenclature in the art or are named by using ChemDraw® software, and commercially available compounds use their supplier catalog names.

**1. X-ray powder diffractometer (XRPD) method of the present disclosure**

Instrument model: Bruker D8 Advance Diffractometer, test method: >2 mg sample for XRPD detection.

The detailed XRPD parameters were as follows: X-ray source: Cu, kα radiation, light tube voltage: 40 kV, light

tube current: 40 mA,

Divergent slit: 0.6 mm, Soller slit: 2.5 mm, receiving slit: 8 mm, filter: nickel sheet, measuring time: 6 min,

Scanning angle range: 3° to 40° 2θ/3° to 30° 2θ, step width angle: 0.02° 2θ, step length: 18.3 seconds /36.6 seconds, rotational speed of sample tray: none.

**2. Differential scanning calorimetry (DSC) method of the present disclosure**

Instrument model: TA Instruments Q200 DSC differential scanning calorimeter.

Test method: the sample (0.5 mg to 5 mg) was placed in a covered aluminum crucible (covered without punching/covered with punching), and heated from 0°C to 3 20°C /350C at a heating rate of 10°C/min under the protection of 50 mL/min dry N2, while the heat change of the sample during the heating process was recorded by the TA software.

**3. Thermal Gravimetric Analyzer (TGA) method of the present disclosure**

Instrument model: TA Instruments Q500 TGA thermogravimetric analyzer.

Test method: the sample (1 mg to 15 mg) was placed in a platinum crucible, and heated from room temperature to 350°C at a heating rate of 10°C/min under the protection of 40 mL/min dry N2 using the method of segmented high-resolution detection, while the weight change of the sample during the heating process was recorded by the TA software.

**4. Dynamic Vapor Sorption (DVS) method of the present disclosure**

Instrument model: TA Instruments Q5000 SA dynamic moisture adsorption instrument.

Test conditions: 1 mg to 10 mg sample was weighed and placed in a DVS sample tray for testing.

The detailed DVS parameters were as follows: temperature: 25°C, balance: dm/dt=0.01 %/min for 15.00 min, drying: 90 min at 0% RH, RH (%) test gradient: 10% RH(%), test gradient range: 0%-80%-0%.

The hygroscopicity evaluation is classified as follows:

| Hygroscopic classification | Moisture adsorption standard (ΔW%) |
|---|---|
| Deliquescent | Adsorbing enough moisture to form a liquid |
| Very hygroscopic | ΔW% ≥ 15% |
| Hygroscopic | 15% > ΔW% ≥ 2% |
| Slightly hygroscopic | 2% > ΔW% ≥ 0.2% |
| Non-hygroscopic | ΔW% < 0.2% |
| Note: ΔW% represents the hygroscopic weight gain of the test article at 25 ± 1°C and 80 ± 2% RH (European Pharmacopoeia 6.0) | |

**Example 1: Preparation of Compound represented by formula (I)**

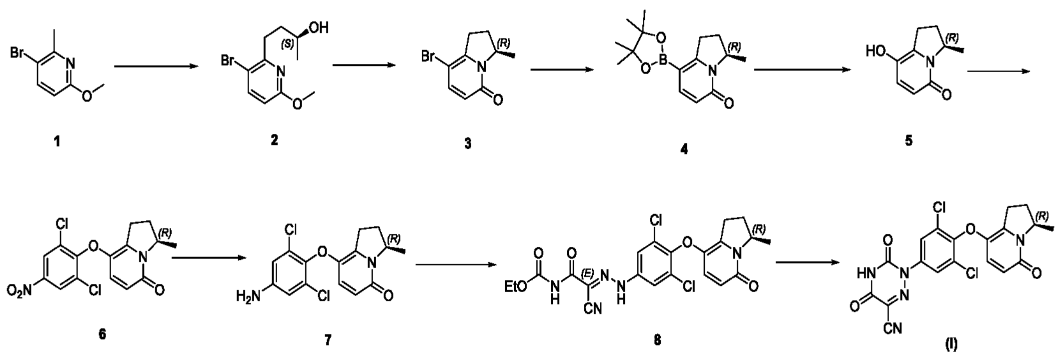

1 2 3 4 5

6 7 8 (I)

**Step 1:**

Compound **1** (5.0 g, 24.75 mmol) was dissolved in anhydrous tetrahydrofuran (100 mL), under nitrogen protection, lithium diisopropylammonium (27.23 mL, 54.45 mmol) was added dropwise at -78C to react at -50°C for 40 min, then (S)-(-)-epoxypropylene (6.587 g) was added to react at -50°C for another 30 min. The reaction mixture was naturally brought to room temperature for 4 h, quenched with 3mL of saturated ammonium chloride aqueous solution, dried over anhydrous sodium sulfate, filtered, concentrated and purified by column chromatography to obtain compound **2** (yellow liquid). MS m/z(ESI): 260.02 $[M+1]^+$, $^1$H NMR(500MHz,DMSO-$d_6$)δ: 7.77-7.75 (d,1H), 6.57-6.55 (d, 1H), 4.49-4.48 (d, 1H), 3.81 (s, 3H), 3.68-3.63 (m, 1H), 2.83-2.75 (m, 2H), 1.71-1.66 (m, 2H), 1.09-1.08 (d, 3H).

**Step 2:**

Compound 2 (2.38 g) was dissolved in toluene (25.0 mL), 2,6-dimethylpyridine (2 g) was added, then trifluoromethanesulfonic anhydride (2.5 g) was added to react for 1 h. Potassium carbonate (3.2 g) was added to react at 75°C for 2 h, 100 mL of water was added, and extracted with ethyl acetate (100 mL×3), washed once with saturated brine, dried over anhydrous sodium sulfate, filtered, concentrated, and THF and hydrobromic acid were added in a flask. The reaction was carried out at about 60°C for 3 days, and the reaction solution was cooled to room temperature, the pH was adjusted to 7 to 8 with sodium bicarbonate, the reaction solution was extracted with EA for three times, the organic phases was combined, washed with 1 N HCl and brine, dried over sodium sulfate and concentrated to obtain compound 3 (white solid). MS m/z(ESI): 227.99 $[M+1]^+$, $^1$HNMR (500MHz, DMSO d6)δ: 7.47-7.45 (d, 1H), 6.16-6.14 (d, 1H), 4.73-4.70 (d, 1H), 3.20-3.13 (m, 1H), 2.97-2.93 (m, 1H), 2.31-2.23 (m, 1H), 1.82-1.77 (m, 1H), 1.28-1.26 (d, 3H)。

**Step 3:**

Compound 3, Dioxane (10V), bis-boron (1.5 eq), AcOK(2.0 eq) and Pd(dppf)$Cl_2$ (0.1 eq) were added into a flask, replaced with nitrogen for three times, reacted at 80C to 90°C for 16 h, and then cooled to room temperature for quenching. The crude product was purified by column to obtain compound 4 (white solid). $^1$HNMR (500MHz,DMSO d6)δ: 7.63-7.6 1(d, 1H), 6.38-6.35 (d, 1H), 4.90-4.83 (m, 1H) , 3.43-3.18 (m, 2H) ,2.29-2.18 (m, 2H), 1.43-1.37 (m, 3H), 1.30-1.24 (m, 12H)。

**Step 4:**

Compound 4 was dissolved in a mixed solvent of 30 mL tetrahydrofuran and 4.0 mL water, then 30% hydrogen peroxide (4.0 ml) was added to react at 25°C for 16 hours, then sodium sulfite aqueous solution was added for quenching, and ethyl acetate (30 mL) was added, then the reaction mixture was dried over anhydrous sodium sulfate, filtered, concentrated and purified by column chromatography to obtain compound 5 (light yellow solid). $^1$H NMR (500MHz,DMSO d6)δ: 8.56 (s, 1H), 7.13-7.11 (d, 1H), 6.06-6.04 (d, 1H), 4.60-4.57 (m, 1H), 2.99-2.93 (m, 2H), 2.20-2.16 (m, 1H), 1.79-1.75 (m, 1H),1.24-1.23 (md, 3H)。

**Step** 5:

Compound 5 (92.5 mg) was dissolved in 2.0 mL of N,N-dimethylformamide, 1,3-dichloro-2-fluoro-5-nitrobenzene (117.6 mg) and potassium carbonate (232.2 mg) were added to react at 25°C for 2 h, and the reaction mixture was concentrated under reduced pressure to remove the solvent, and purified by column chromatography to obtain compound 6 (30.1 g, yellow-green solid). MS m/z (ESI): 355.02$[M+1]^+$。

**Step 6:**

Compound 6 (10 g) was dissolved in 200 mL dichloromethane, palladium carbon (0.5 g) was added to reacted in hydrogen gas environment for 6 h. After filtration, the filtrate was concentrated to obtain compound 7 (0.85 g, yellow solid). MS m/z (ESI): 325.04$[M+1]^+$。 $^1$H NMR (500MHz, DMSO d6)δ: 6.87-6.85 (d, 1H), 6.65 (s, 2H), 6.10-6.08 (d, 1H), 5.61 (s, 2H), 4.66-4.64 (m, 1H), 3.16-3.05 (m, 2H), 2.31-2.26 (m, 1H), 1.86-1.82 (m, 1H), 1.29-1.27 (d, 3H)。

**Step 7:**

Compound 7 (150 mg) was dissolved in a mixed solvent consisted of 3.2 mL acetic acid, 0.8 mL water and 0.4 mL concentrated hydrochloric acid. Sodium nitrite (33.3 mg) was added at 0°C, sodium acetate (113.2.0 mg) was added after 10 minutes of reaction, and N-cyanoacetylurethane (143.6 mg) was added after another 10 minutes of reaction. The reaction mixture was brought to room temperature for 72 h, filtered with 5 mL of water and dried to obtain compound **8** (1.8

g, yellow solid). MS m/z (ESI): 492.08 $[M+1]^+$。 $^1$H NMR (500MHz, DMSO d6)δ 12.15 (s, 1H), 10.92 (s, 1H), 8.00 (s, 2H), 6.97-6.95 (d, 1H), 6.15-6.14 (d, 1H), 4.73-4.68 (m, 1H), 4.23-4.19 (m, 2H), 3.24-3.09 (m, 2H), 2.37-2.30 (m, 1H), 1.92-1.87 (m, 1H), 1.32-1.26 (m, 6H).

**Step 8:**

Compound 8 (180 mg) was dissolved in 3.0 ml of N,N-dimethylacetamide, sodium acetate (108.9 mg) was added to react at 120°C for 2 h. The mixture was concentrated under reduced pressure to remove the solvent, and purified to obtain compound I (1.8 g, light yellow solid). MS m/z (ESI): 446.03 $[M+1]^+$。 $^1$H NMR (500MHz, DMSO $d_6$) δ 13.26 (s, 1H), 7.75 (s, 2H) ,7.01-6.99 (d, 1H), 6.12-6.10 (d, 1H), 4.69-4.68 (m, 1H), 3.23-3.08 (m, 2H), 2.35-2.32 (m, 1H), 1.89-1.85 (m, 1H), 1.31-1.29 (m, 3H).

The prepared compound was further treated according to the following methods in Examples 2 to 12 to obtain compound **I** with different crystalline forms.

**Example 2: Preparation of crystalline form A of compound represented by formula (I)**

**Preparation method 1:**

The compound of formula (I) (100 g) was dissolved in 1100 mL of a solvent mixture of tetrahydrofuran and methanol (THF/MeOH=3/2) and heated to 60°C until completely dissolved. Water (1000 mL) was added to the solution at 20°C to 30°C under stirring. The solution was cooled slowly to 20°C, continued to be stirred for 0.5 h and then filtered. The obtained filter cake was washed with methanol. The filter cake was collected and dried under vacuum to constant weight to obtain the crystalline form A of compound represented by formula (I).

The XRPD pattern with Cu-Kα radiation, the DSC curve and the TGA curve of the prepared crystalline form A of the compound are shown in Figs.1 to 3 respectively.

**Preparation method 2:**

About 100 mg of the compound represented by formula (I) was weighted, the solvent N,N-dimethylformamide (0.4 mL) was added for dissolution to obtain a clear solution and filtered; water (1 mL) was added to the solution at 20°C to 30°C under stirring, solid was precipitated, then stirred overnight, centrifuged, and the obtained filter cake was washed with water. The filter cake was collected and dried under vacuum to constant weight to obtain crystalline form A of the compound represented by formula (I).

**Preparation method 3:**

About 100 mg of the compound represented by formula (I) was weighed, the solvent dimethyl sulfoxide (0.2 mL) was added for dissolution to obtain a clear solution, and then filtered; water (1 mL) was added to the solution at 20°C to 30°C under stirring, solid was precipitated, then the solution was stirred overnight, centrifuged, and the obtained filter cake was washed with water. The filter cake was collected and dried under vacuum to constant weight to obtain crystalline form A of the compound represented by formula (I).

**Preparation method 4:**

About 1 g of the compound represented by formula (I) was weighted, the solvent methanol (75 mL) was added, heated to 60°C until completely dissolved and then filtered; a part of the solvent methanol was concentrated at 40C to 45°C , solid was precipitated, and then the solution was cooled to room temperature of 20°C to 30°C , centrifuged, and the obtained filter cake was washed with methanol. The filter cake was collected and dried under vacuum to constant weight to obtain crystalline form A of the compound represented by formula (I).

**Example 3: Preparation of Crystalline form B of Compound represented by formula (I)**

100 mg of the compound represented by formula (I) was weighted, 4.0 mL of tetrahydrofuran was added for ultrasonic dissolution to obtain a clear solution, and 1.0 mL of toluene was added to keep the solution clear. The solution was left open for evaporation at 40°C to obtain the crystalline form B of the compound represented by formula (I).

The XRPD pattern with Cu-Kα radiation, the DSC curve and the TGA curve of the prepared crystalline form B of the compound are shown in Figs. 4 to 6 respectively.

Example 4: **Preparation of Crystalline form C of Compound represented by formula (I)**

100 mg of the compound represented by formula (I) was weighted, 40.0 mL of acetone was added for ultrasonic dissolution to obtain a clear solution, and 10.0 mL of isopropyl acetate was added to keep the solution clear. The solution was left open for evaporation at room temperature to obtain the crystalline form C of the compound represented by formula (I).
The XRPD pattern with Cu-Kα radiation, the DSC curve and the TGA curve of the prepared crystalline form C of the compound are shown in Figs. 7 to 9 respectively.

**Example 5: Preparation of Crystalline form D of Compound represented by formula (I)**

100 mg of the compound represented by formula (I) was weighted, 0.4 mL of tetrahydrofuran was added for ultrasonic dissolution to obtain a clear solution, and 0.1 mL of water was added to keep the solution clear. The solution was left open for evaporation at room temperature to obtain the crystalline form D of the compound represented by formula (I).
The XRPD pattern with Cu-Kα radiation of the prepared crystalline form D of the compound is shown in FIG. 10.

**Example 6: Preparation of crystalline form E of compound represented by formula (I)**

100 mg of the compound represented by formula (I) was weighted, 4.0 mL of tetrahydrofuran was added for ultrasonic dissolution to obtain a clear solution, and 1.0 mL of water was added to keep the solution clear. The solution was left open for evaporation at room temperature to obtain the crystalline form E of the compound represented by formula (I).
The XRPD pattern with Cu-Kα radiation, the DSC curve and the TGA curve of the prepared crystalline form E of the compound are shown in Figs. 11 to 13 respectively.

**Example 7: Preparation of crystalline form F of compound represented by formula (I)**

100 mg of the compound represented by formula (I) was weighted and placed into a centrifuge tube, left in tetrahydrofuran atmosphere for 8 days at room temperature, and dried under vacuum at room temperature for about 27.5 h to obtain the crystalline form F of the compound represented by formula (I).
The XRPD pattern with Cu-Kα radiation, the DSC curve and the TGA curve of the prepared crystalline form F of the compound are shown in Figs. 14 to 16 respectively.

**Example 8: Preparation of crystalline form G of compound represented by Formula (IV)**

100 mg of the compound represented by formula (I) was weighted, 3.5 mL of tetrahydrofuran was added at room temperature for dissolution to obtain a clear solution, filtered, and the filtrate was added dropwise into 20.0 mL of methyl tert-butyl ether solvent, stirred at room temperature for about 2 h and no solid was precipitated, the solution was transferred to 4°C and stirred for about 21.5 h, solid was precipitated, centrifuged, and dried under vacuum at room temperature for about 4.5 h to obtain the crystalline form G of the compound represented by formula (I).
The XRPD pattern with Cu-Kα radiation, the DSC curve and the TGA curve of the prepared crystalline form G of the compound are shown in Figs. 17 to 19 respectively.

**Example 9: Preparation of crystalline form H of compound represented by formula (I)**

100mg of the compound represented by formula (I) was weighted, 4.0 mL of tetrahydrofuran was added for ultrasonic dissolution to obtain a clear solution, and concentrated under reduced pressure at 60°C to dryness to obtain the crystalline form H of the compound represented by formula (I).
The XRPD pattern with Cu-Kα radiation of the prepared crystalline form H of the compound is shown in Fig. 20.

**Example 10: Preparation of crystalline form I of compound represented by formula (I)**

100 mg of the compound represented by formula (I) was weighted, 3.0 mL of tetrahydrofuran was added for ultrasonic dissolution to obtain a clear solution, and 0.5 mL of acetonitrile was added to keep the solution clear. The solution was left open for evaporation at 40°C to obtain the crystalline form I of the compound represented by formula (I).
The XRPD pattern with Cu-Kα radiation, the DSC curve and the TGA curve of the prepared crystalline form I of the compound are shown in Figs. 21 to 23 respectively.

**Example 11: Preparation of crystalline form J of compound represented by formula (I)**

120 mg of the compound represented by formula (I) was weighted, 8.0 mL of toluene was added, and the suspension was stirred at room temperature for 8 days and centrifuged, and the obtained solid was dried under vacuum at room temperature for about 27.5 h to obtain the crystalline form J of the compound represented by formula (I).
The XRPD pattern with Cu-Kα radiation, the DSC curve and the TGA curve of the prepared crystalline form J of the compound are shown in Figs. 24 to 26 respectively.

**Example 12: Preparation of crystalline form K of compound represented by formula (I)**

20 mg of the compound represented by formula (I) was weighted, 8.0 mL of ethanol was added at room temperature for dissolution to obtain a clear solution, and filtered, 12.0 mL of isopropyl acetate was added dropwise into the filtrate, and stirred at room temperature and no solid was precipitated, and stirred at 4°C for two days and no solid was precipitated, and then the solution was transferred to 40°C and left open for evaporation to obtain the crystalline form K of the compound represented by formula (I).
The XRPD pattern with Cu-Kα radiation of the prepared crystalline form K of the compound is shown in FIG. 27.

**Test example 1: Study on hygroscopicity of crystalline form A of compound represented by formula (I)**

Experimental material: TA Instruments Q5000 SA dynamic moisture adsorption instrument

Experimental method: 1 mg to 10 mg of sample was weighted and placed in a DVS sample tray for testing.

Experimental results: the DVS graph of crystalline form A of the compound represented by formula (I) was as shown in FIG. 28, △W= 0.6%.

Experimental conclusion: the hygroscopic weight gain of crystalline form A of the compound represented by formula (I) is 0.6% at 25°C and 80% RH, with slight hygroscopicity.

**Test Example 2: Solid stability test of crystalline form A of compound represented by formula (I)**

According to the "Guidelines for Stability Test of Active Pharmaceutical Ingredient and Preparations" (General Principles 9001 of Part Four of Chinese Pharmacopoeia 2015 Edition), the crystalline form A of compound represented by formula (I) was investigated for stability at the conditions of high temperature (50C, open), accelerated treatment (40°C/ 75% relative humidity, open), high humidity (25C/ 85% relative humidity, open) and long -term (25°C /65% relative humidity, open).
About 10 mg of the compound represented by formula (I) was accurately weighed, placed in a dry and clean glass bottle, spread out into a thin layer and covered with aluminum foil with small holes and placed under the influence factor test conditions of high temperature (60°C) and accelerated treatment (40C/75% RH), high humidity condition (25C/85% RH) and long-term condition (25C/RH). Samples in duplicate were placed under each condition, and the samples for XRPD detection and melting points were placed separately. Samples placed under different conditions were sampled for XRPD detection at the planned test end point, and the test results were compared with the initial test results at Day 0. The test results are shown in Table 12 below:

Table 12 Test results of solid stability of crystalline form A of compound represented by formula (I)

| Test condition | Time point | Crystalline form | Melting point |
|---|---|---|---|
| - | 0 days | Crystalline form A | 297°C |
| 50°C , open | 10 days | Crystalline form A | 297°C |
| 40°C / 75% RH , open | 10 days | Crystalline form A | 297°C |
| 25 °C / 85% RH , open | 10 days | Crystalline form A | 297°C |
| 25°C / 65% RH , open | 10 days | Crystalline form A | 297°C |

Conclusion: the crystalline form A of the compound represented by formula (I) had good stability under high temperature, high humidity, long-term and accelerated conditions.

**Experimental Example 3: In vitro Enzyme Activity Test of Thyroid Receptor β(THRβ)**

Time-resolved fluorescence resonance energy transfer (TR-FRET) experimental method was used to evaluate the agonist activity in vitro of the compounds to be tested through the binding of thyroid hormone receptor β (THR β) with its co-activating peptide SRC2-2.

GST tag was fused to THR β ligand binding domain (THR β LBD), and its co-activating peptide SRC2-2 was biotinylated. The binding capacity of THR β LBD to SRC2-2 was determined by detecting the tightly bound Europium-labeled anti-GST antibody and Streptavidin labeled with D2 with TR-FRET.

Experimental steps

1) the compounds to be tested were dissolved in DMSO to prepare a 30 mM stock solution that could be stored in a dryer at room temperature for three months. If it is stored for a long time, it will be placed in a refrigerator at -20°C. 1x reaction buffer was prepared from 50 mM HEPES (pH 7.0), 50 mM KF, 1 mM DTT, 0.05% NP-40 and 0.2% BSA.

a) 10 μM positive compound T3 (100×) and 1 mM compound to be tested (100×) (crystalline form A of compound prepared in Example 2) were prepared by using DMSO.

b) In a 96-well plate, 10 μM of T3 and 1 mM of the compound to be tested were diluted by three-fold gradient, and measured at 10 concentration points.

c) the 100>positive compound and the compound to be tested were diluted to 4× with 1×reaction buffer.

d) 5 μL of 4×positive compound or the compound to be tested (see step c) was added to a 384-well experimental plate.

e) 4×THRβ-LBD (0.8 nM) and 4×RXRα (0.8 nM) solutions were prepared by using 1×reaction buffer.

f) 5 μL of the solution in step e was added into an experimental plate (prepared in step d) and reacted at room temperature for 15 min.

g) a mixed solution containing 400 nM 2>Biotin -SRC2-2 coactivating peptide, 25 nM 2×Streptavidin -d2 and 2>Europium anti -GST (1:200) was prepared by using 1×reaction buffer.

h) 10 μL of the mixed solution prepared in step g was added into each hole of the experimental plate to start the reaction.

i) the experimental plate was centrifuged at 1000 rpm for 1 min.
j) incubation was performed for 1 h at room temperature in the dark.
k) the values of wavelengths of 665 nm and 615 nm were read by using BMG.

2) Data analysis

The activity rate is calculated as follows:

$$\%\text{activity rate} = \left( \frac{\text{Ratio value}_{compound} - \text{Ratio value}_{negativecontrol}}{\text{Ratio value}_{positivecontrol} - \text{Ratio value}_{negativecontrol}} \right) \times 100$$

**Ratio value** $_{\textbf{positive control}}$: average value of the ratios of all positive control holes in the whole plate.

**Ratio value** $_{\textbf{negative control}}$: average value of the ratios of all negative control holes in the whole plate.

3) calculation of $EC_{50}$:

The $EC_{50}$ (half excited concentration) of the compound was obtained by using the following nonlinear fitting formula.

$$Y=Bottom + (Top-Bottom)/ (1+10^{\wedge} ((LogEC_{50-x})*HillSlope))$$

X: log value of compound concentration; Y: activity rate of compound.

Table 13 EC50 test results of thyroid receptor β (THRβ) enzyme activity in vitro

| Compound No. | Structure | $EC_{50}$ (μM) |
|---|---|---|
| Compound represented by formula (I) | 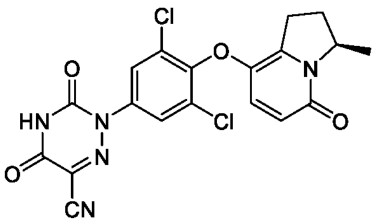 (I) | 0.04596 |

Conclusion: the $EC_{50}$ value of the compound represented by formula (I) was 0.04596 μM, indicating that this compound promotes the binding of THRβ with its co-activating peptide, and has potential agonist activity on THRβ.

**Experimental Example 4: Cytological activity test of thyroid receptor β (THRβ)**

In this study, the fluorescence reporter gene experiment method was used to evaluate the excitatory effects of the two compounds to be tested on THR β target.

PGL4.35[luc2P/9XGAL4UAS/Hygro] contains nine GAL4-UAS repeat sequences (upstream activation sequences). This sequence drives the transcription of luciferase reporter gene luc2P, in response to the binding of the fusion protein containing Gal4 DNA binding domain with the ligand binding domain of thyroid receptor β (THR β) in pBIND THRβ vector. Stimulation on THRβ by the test sample will lead to the increase of luminescence signal.

The experimental process is as follows:

1) The test compound (crystalline form A of the compound prepared in Example 2) was dissolved in DMSO to prepare a 30 mM stock solution. The positive compound was dissolved in DMSO to prepare a 5 mM stock solution, and then diluted to a 0.5 mM stock solution. All compounds were dissolved in DMSO and stored in a refrigerator at -20C. In a 384 -well plate, the compound was diluted with DMSO in 3-fold gradient, with 10 concentration gradients, and the initial concentration was 30 mM.
In a 384-well plate, the positive compound (T3) was diluted with DMSO in 3-fold gradient, with 10 concentration gradients, and the initial concentration was 0.25 mM. 500>positive control (0.25 mM, T3) and 500>negative control (100% DMSO) were prepared.

2) HEK293T cells were cultured according to ATCC standard, and the cells were tested in exponential growth period. The supernatant of the culture medium was discarded gently and the cells were washed with PBS twice. The cells were digested with trypsin digestive juice, and the digestion was terminated with complete medium. The cells were collected and counted. Cell viability greater than 90% can be used in the experiment. $2.5\times10^{6}$ HEK293T cells were inoculated into a 60 mm cell culture dish. The dish inoculated with cells was placed in a 5% $CO_2$ incubator at 37°C for overnight culture for 18h.

3) LipoLTX & PLUS transfection reagent was placed at room temperature; and two 1.5 mL EP tubes were prepared. 12 μL LipoLTX reagent and 250 μL opti-MEM™ medium were added into an EP tube, and be careful not to touch the tube

wall. 6 μg plasmid and 250 μL Opti-MEM™ medium were added into another EP tube, then 6 μL Lipo PLUS reagent was added, mixed well with a pipette, and stood at room temperature for 5 min. The two EP tubes were mixed and stood at room temperature for 15min. The transfection reagent mixed with plasmid was added into a 60 mm cell culture dish. The dish was placed in a 5% $CO_2$ incubator at 37°C for 5 h.

4) 50 nL of the diluted compound was transfered to a 384-well cell culture plate with Echo655. Cells were inoculated into 384-well cell culture plates, with 17,000 cells per well and 25 μL of FBS medium containing 5% carbon adsorption. Cell culture plates were cultured overnight in a 5% $CO_2$ incubator at 37°C for 16 h to 20 h.

5) The Britelite plus detection reagent was placed at room temperature. The 384-well cell plate was placed at room temperature. 25 uL Britelite plus detection reagent was added into each well of the cell culture plate. Envision was used to detect the luminescence value.

6) Agonism percentage is calculated as follows:

$$\text{agonism } \% = \left| \frac{\text{RLU}_{\text{compound}} - \overline{\text{RLU}}_{\text{negative control}}}{\overline{\text{RLU}}_{\text{positive control}} - \overline{\text{RLU}}_{\text{negative control}}} \right| * 100$$

RLU: Resulting Luminescence
$\overline{\text{RLU}}_{\text{positive}}$ control: average value of luminescence of all positive control holes in the whole plate.
$\overline{\text{RLU}}_{\text{negative control}}$: average value of luminescence of all negative control holes in the whole plate.

7) calculation of $EC_{50}$:

The $EC_{50}$ of the compound was obtained by using the following nonlinear fitting formula and using Graphpad8.0.

$$Y=Bottom + (Top-Bottom)/(1+10\text{^}((LogEC50-X)*HillSlope))$$

X: log value of compound concentration; Y: agonism percentage of compound.

The experimental results are shown in Table 14:

Table 14 $EC_{50}$ test results of THR β in vitro cell activity

| **Compound No.** | **Structure** | **$EC_{50}$ (μM)** |
|---|---|---|
| Compound represented by formula (I) | 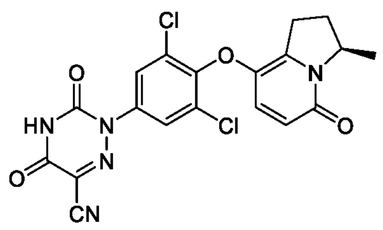 (I) | 0.214 |

Conclusion: At the cellular level, the $EC_{50}$ of the compound represented by formula (I) was 0.214 μM, indicating that the compound represented by formula (I) has good agonist activity on thyroid receptor β (THRβ).

**Experimental example 5 Pharmacokinetic evaluation in rats**

Experimental purpose:

To determine the pharmacokinetic parameters of the compound represented by formula (I) in rats.

Experimental scheme:

1) experimental drug: the compound represented by formula (I); (crystalline form A of the compound prepared in Example 2).
2) Experimental animals: 6 male CD rats aged 6 weeks to 8 weeks;
3) Experimental procedure:

The animals were given the compound at a dose of 5 mg/kg at a concentration of 0.5 mg/mL. Animals were cross-collected for plasma samples at 2, 6 and 10 h after administration. The drug concentration in the plasma samples was determined by LC-MS/MS method, and the kinetic parameters of the test drugs obtained were found in Table 15:

Table 15 test results of liver-blood ratio in rats

| | **Dosage (mg/kg)** | **Time (h)** | **Blood drug concentration P(ng/mL)** | **Liver drug concentration L(ng/g)** | **L/P concentration ratio** | **T1/2 (h)** |
|---|---|---|---|---|---|---|
| Rat PO | 5.0 | 2 | 153.3 | 1178.9 | 7.7 | 3.7 |
| | | 6 | 33.8 | 745.9 | 22.1 | |
| | | 10 | 10.1 | 238.4 | 23.6 | |

Conclusion: The blood drug concentration of the compound represented by formula (I) in rats was relatively low, and the liver drug concentration was relatively high, so the compound represented by formula (I) has good liver targeting.

The above descriptions are merely the preferred examples of the present disclosure, and are not intended to limit the present disclosure in any form. Though the present disclosure has been disclosed by using the preferred examples as above, it is not intended to limit the present disclosure. It is possible for any one skilled in the art to make some amendments and modifications to obtain equivalent variations of the examples by utilizing the technical contents mentioned above, without departing from the scope of the technical solutions of the present disclosure. However, anything that does not departed from the technical solutions of the present disclosure, including any simple amendments, equivalent variations and modifications of the above examples according to the technical essence of the present disclosure, should fall within the scope of technical solutions of the present disclosure.

## Claims

**1.** A compound, which is a compound having a structural formula represented by formula (I) or a pharmaceutically acceptable salt thereof:

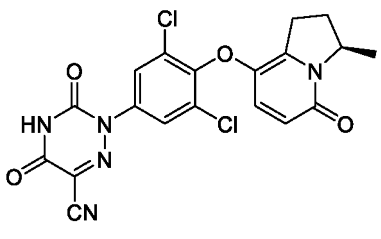

(I) ;

preferably, the compound having the structural formula represented by formula (I) or the pharmaceutically acceptable salt thereof is used for manufacture of a medicament for treating a metabolic disease; and preferably, the medicament is used as a thyroid hormone β receptor agonist; preferably, the metabolic disease includes obesity, hyperlipidemia, hypercholesterolemia, diabetes, hepatic steatosis, nonalcoholic steatohepatitis, atherosclerosis, cardiovascular diseases, thyroid diseases, and tumor in intrahepatic bile duct.

**2.** A crystalline form A of the compound according to claim 1, wherein its X-ray powder diffraction pattern with Cu-Ka

radiation shows characteristic peaks at 2θ angles of 6.10±0.20°, 12.08±0.20° and 16.49±0.20° ;

preferably, the X-ray powder diffraction pattern shows characteristic peaks at 2θ angles of 6.10±0.20° , 12.08 ±0.20° , 13.61±0.20° , 15.71±0.20° , 16.49±0.20° , 20.05±0.20° , 21.47±0.20° and 22.49±0.20° ;
preferably, the X-ray powder diffraction pattern shows characteristic peaks at 2θ angles of 6.10±0.20° , 12.08 ±0.20° , 13.61±0.20° , 15.71±0.20° , 16.49±0.20° , 20.05±0.20° , 20.73±0.20° , 21.47±0.20° , 21.80±0.20° and 22.49±0.20° ;
preferably, an XRPD pattern of the crystalline form A is shown in FIG. 1;
preferably, a differential scanning calorimetry curve of the crystalline form A shows an endothermic peak at 300.1 ±3.0°C;
preferably, a DSC curve of the crystalline form A is shown in FIG. 2;
preferably, a thermogravimetric analysis curve of the crystalline form A shows a weight loss of 0.6% at 150.0C ±3.0°C; and
preferably, a TGA curve of the crystalline form A is shown in FIG. 3.

**3.** A method for preparing the crystalline form A according to claim 2, comprising:

(1) dissolving the compound represented by formula (I) in a mixed solvent of tetrahydrofuran and methanol, and heating until complete dissolution;
(2) adding water to the solution under stirring, cooling the solution slowly, continuously stirring, and filtering; and
(3) washing obtained filter cake with methanol, collecting the filter cake, and drying in vacuum to constant weight to obtain the crystalline form A of the compound represented by formula (I);
preferably, in step (1), a volume ratio of tetrahydrofuran to methanol in the mixed solvent is 2:1 to 1:2;
preferably, in step (1), a concentration of the compound represented by formula (I) in the mixed solvent is 0.02 g/ml to 0.2 g/ml;
preferably, in step (1), the heating is performed to 45C to 65C;
preferably, in step (2), a volume ratio of the added water to the mixed solvent in step (1) is 1:2 to 3:1;
preferably, in step (2), water is added to the solution at 15°C to 35C; the solution is slowly cooled to 15C to 30°C and continuously stirred for 0.5 h to 1 h;
or, the method comprises:

(1) dissolving the compound represented by formula (I) in N,N-dimethylformamide to obtain a clear solution, and then filtering;
(2) adding water to the solution under stirring, precipitating solid, then stirring overnight and centrifuging; and
(3) washing obtained filter cake with water, collecting the filter cake, and drying in vacuum to constant weight to obtain the crystalline form A of the compound represented by formula (I);
preferably, in step (1), a concentration of the compound represented by formula (I) in N,N-dimethylformamide is 0.1 g/ml to 0.3 g/ml;
preferably, in step (2), a volume ratio of the added water to N,N-dimethylformamide in step (1) is 1:2 to 3:1;
preferably, in step (2), water is added to the solution at 15°C to 35°C;
or, the method comprises:

(1) dissolving the compound represented by formula (I) in dimethyl sulfoxide to obtain a clear solution, and then filtering it;
(2) adding water to the solution under stirring, precipitating solid, then stirring overnight and centrifuging; and
(3) washing obtained filter cake with water, collecting the filter cake, and drying in vacuum to constant weight to obtain the crystalline form A of the compound represented by formula (I);
preferably, in step (1), a concentration of the compound represented by formula (I) in dimethyl sulfoxide is 0.3 g/ml to 0.8 g/ml;
preferably, in step (2), a volume ratio of the added water to dimethyl sulfoxide in step (1) is 3:1 to 7:1;
preferably, in step (2), water is added to the solution at 15°C to 35°C;
or, the method comprises:

(1) dissolving the compound represented by formula (I) in methanol, heating to complete dissolution, and filtering;
(2) removing part of methanol by concentration until solid is precipitated, cooling and centrifuging; and

(3) washing obtained filter cake with methanol, collecting the filter cake, and drying in vacuum to constant weight to obtain crystalline form A of the compound represented by formula (I);
preferably, in step (1), a concentration of the compound represented by formula (I) in methanol is 0.01 g/ml to 0.1 g/ml;
preferably, in step (1), the heating is performed to 45C to 65C;
preferably, in step (2), part of methanol is removed by concentration at a temperature of 30C to 50°C;
preferably, in step (2), the cooling is performed at room temperature of 20°C to 30°C.

**4.** A crystalline form B of the compound according to claim 1, wherein its X-ray powder diffraction pattern with Cu-Ka radiation shows characteristic peaks at 2θ angles of 9.35±0.20° , 10.36±0.20° and 18.32±0.20°,

preferably, the X-ray powder diffraction pattern shows characteristic peaks at 2θ angles of 9.35±0.20° , 10.36 ±0.20° , 11.77±0.20° , 12.65±0.20° , 15.21±0.20° , 18.32±0.20° , 19.60±0.20° , and 22.74±0.20°,
preferably, the X-ray powder diffraction pattern shows characteristic peaks at 2θ angles of 9.35±0.20° , 10.36 ±0.20° , 11.77±0.20° , 12.65±0.20° , 15.21±0.20° , 18.32±0.20° , 19.60±0.20° , 20.03±0.20° , 21.30±0.20° and 22.74±0.20° ;
preferably, an XRPD pattern of the crystalline form B is shown in FIG. 4;
preferably, a differential scanning calorimetry curve of the crystalline form B shows endothermic peaks at 139.5°C ±3.0°C and 315.6±3.0°C respectively;
preferably, a DSC curve of the crystalline form B is shown in FIG. 5;
preferably, a thermogravimetric analysis curve of the crystalline form B shows a weight loss of 16.0% at 150.0C ±3.0°C; and
preferably, a TGA curve of the crystalline form B is shown in FIG. 6.

**5.** A crystalline form C of the compound according to claim 1, wherein its X-ray powder diffraction pattern with Cu-Ka radiation shows characteristic peaks at 2θ angles of 5.96±0.20°, 10.96±0.20° and 22.84±0.20°,

preferably, the X-ray powder diffraction pattern shows characteristic peaks at 2θ angles of 5.96±0.20°, 9.25 ±0.20°, 10.96±0.20°, 11.94±0.20°, 21.92±0.20°, 22.84± 0.20°, 23.69±0.20° and 28.12±0.20°,
preferably, the X-ray powder diffraction pattern shows characteristic peaks at 2θ angles of 5.96±0.20° 9.25 ±0.20°, 10.96±0.20°, 11.94±0.20°, 15.52±0.20°, 19.50±0.20°, 21.92±0.20°, 22.84±0.20°, 23.69±0.20° and 28.12±0.20 °;
preferably, an XRPD pattern of the crystalline form C is shown in FIG. 7;
preferably, a differential scanning calorimetry curve of the crystalline form C shows endothermic peaks at 123.7°C ±3.0°C and 315.7±3.0°C respectively;
preferably, a DSC curve of the crystalline form C is shown in FIG. 8;
preferably, a thermogravimetric analysis curve of the crystalline form C shows a weight loss of 10.9% at 150.0°C ±3.0°C; and
preferably, a TGA curve of the crystalline form C is shown in FIG. 9.

**6.** A crystalline form D of the compound according to claim 1, wherein its X-ray powder diffraction pattern with Cu-Ka radiation shows characteristic peaks at 2θ angles of 7.97±0.20°, 11.66±0.20° and 15.83±0.20°;

preferably, the X-ray powder diffraction pattern shows characteristic peaks at 2θ angles of 7.97±0.20°, 9.08 ±0.20°, 11.66±0.20°, 12.47±0.20°, 15.83±0.20°, 17.98±0.20°, 20.47±0.20° and 20.74±0.20°;
preferably, the X-ray powder diffraction pattern shows characteristic peaks at 2θ angles of 7.97°, 9.08°, 11.66°, 12.47°, 1 5.83°, 17.98°, 20.47°, 20.74°, 23.37° and 25.04°;
preferably, an XRPD pattern of the crystalline form D is shown in FIG. 10.

**7.** A crystalline form E of the compound according to claim 1, wherein its X-ray powder diffraction pattern with Cu-Ka radiation shows characteristic peaks at 2θ angles of 12.43±0.20°, 12.70±0.20° and 17.94±0.20°;

preferably, the X-ray powder diffraction pattern shows characteristic peaks at 2θ angles of 9.02±0.20°, 10.15 ±0.20°, 12.43±0.20° 12.70±0.20°, 17.94±0.20°, 19.08±0.20°, 20.66±0.20° and 24.96±0.20°;
preferably, the X-ray powder diffraction pattern shows characteristic peaks at 2θ angles of 9.02±0.20°, 10.14 ±0.20°, 12.43±0.20°, 12.70±0.20°, 17.94±0.20°, 19.08±0.20°, 20.66±0.20°, 22.28±0.20°, 24.95±0.20° and 26.55±0.20°;
preferably, an XRPD pattern of the crystalline form E is shown in FIG. 11;

preferably, a differential scanning calorimetry curve of the crystalline form E shows endothermic peaks at 151.1°C ±3.0°C and 315.2±3.0°C respectively;
preferably, an DSC curve of the crystalline form E is shown in FIG. 12;
preferably, a thermogravimetric analysis curve of the crystalline form E shows a weight loss of 6.6% at 150.0°C ±3.0°C; and
preferably, a TGA spectrum of the crystalline form E is shown in FIG. 13.

**8.** A crystalline form F of the compound according to claim 1, wherein its X-ray powder diffraction pattern with Cu-Ka radiation shows characteristic peaks at 2θ angles of 7.13±0.20°, 12.18±0.20° and 18.25±0.20°;

preferably, the X-ray powder diffraction pattern shows characteristic peaks at 2θ angles of 7.13±0.20°, 12.18 ±0.20°, 12.68±0.20°, 15.59±0.20°, 18.25±0.20°, 19.05±0.20°, 22.34±0.20° and 22.70±0.20°;
preferably, the X-ray powder diffraction pattern shows characteristic peaks at 2θ angles of 7.13±0.20°, 12.18 ±0.20°, 12.68±0.20 °, 15.59±0.20°, 17.73±0.20°, 18.25±0.20°, 19.05±0.20°, 22.34±0.20°, 22.70±0.20° and 24.63±0.20°;
preferably, an XRPD pattern of the crystalline form F is shown in FIG. 14;
preferably, a differential scanning calorimetry curve of the crystalline form F shows endothermic peaks at 158.9°C ±3.0°C and 315.6±3.0°C respectively;
preferably, a DSC curve of the crystalline form F is shown in FIG. 15;
preferably, a thermogravimetric analysis curve of the crystalline form F shows a weight loss of 13.5% at 150.0°C ±3.0°C; and
preferably, a TGA spectrum of the crystalline form F is shown in FIG. 16.

**9.** A crystalline form G of the compound according to claim 1, wherein its X-ray powder diffraction pattern with Cu-Ka radiation shows characteristic peaks at 2θ angles of 12.15±0.20°, 12.74±0.20° and 15.37±0.20°;

preferably, the X-ray powder diffraction pattern shows characteristic peaks at 2θ angles of 7.09±0.20°, 12.15 ±0.20°, 12.74±0.20°, 15 .37±0.20°, 17.63±0.20°, 18.15±0.20°, 22.57±0.20° and 22.76±0.20°;
preferably, the X-ray powder diffraction pattern shows characteristic peaks at 2θ angles of 7.09±0.20°, 12.15 ±0.20°, 12.74±0.20°, 15.37±0.20°, 17.63±0.20°, 18.15±0.20°, 22.21±0.20°, 22.57±0. 20°, 22.76±0.20° and 23.64±0.20°;
preferably, a XRPD pattern of the crystalline form G is shown in FIG. 17;
Preferably, a differential scanning calorimetry curve of the crystalline form G shows endothermic peaks at 157.5°C±3.0°C and 316.6±3.0°C respectivel y;
preferably, a DSC curve of the crystalline form G is shown in FIG. 18;
preferably, a thermogravimetric analysis curve of the crystalline form G shows a weight loss of 11.9% at 175.0°C ±3.0°C; and
preferably, a TGA spectrum of the crystalline form G is shown in FIG. 19.

**10.** A crystalline form H of the compound according to claim 1, wherein its X-ray powder diffraction pattern with Cu-Ka radiation shows characteristic peaks at 2θ angles of 9.59±0.20°, 10.18±0.20° and 18.66±0.20°;

preferably, the X-ray powder diffraction pattern shows characteristic peaks at 2θ angles of 9.59±0.20°, 10.18 ±0.20°, 11.29±0.20°, 11.83±0.20°, 15.57±0.20°, 18.66±0.20°, 20.13±0.20° and 22.80±0.20°;
preferably, the X-ray powder diffraction pattern shows characteristic peaks at 2θ angles of 9.59±0.20°, 10.18 ±0.20°, 11.29±0.20°, 11.83±0.20°, 12.77±0.20°, 15.57±0.20°, 18.66±0.20°, 20.13±0.20°, 22.80±0.20° and 25.12±0.20°;
preferably, an XRPD pattern of the crystalline form H is shown in FIG. 20.

**11.** A crystalline form I of the compound according to claim 1, wherein its X-ray powder diffraction pattern with Cu-Ka radiation shows characteristic peaks at 2θ angles of 9.06±0.20°, 12.45±0.20° and 17.99±0.20°;

preferably, the X-ray powder diffraction pattern shows characteristic peaks at 2θ angles of 9.06±0.20°, 10.18 ±0.20°, 12.45±0.20°, 13.18±0.20°, 17.99±0.20°, 20.79±0.20°, 24.42±0.20°, 25.05±0.20° and 26.57±0.20°;
preferably, the X-ray powder diffraction pattern shows characteristic peaks at 2θ angles of 9.06±0.20°, 10.18 ±0.20°, 12.45±0.20°, , 17.99±0.20°, 18.74±0.20°, 20.53±0.20°, 20.79±0.20°, 24.42±0.20°, 25.05±0.20° and 26.57±0.20°;
preferably, an XRPD pattern of the crystalline form I is shown in FIG. 21;

preferably, a differential scanning calorimetry curve of the crystalline form I shows an endothermic peak at 316.0°C±3.0°C;
preferably, a DSC curve of the crystalline form I is shown in FIG. 22;
preferably, a thermogravimetric analysis curve of the crystalline form I shows a weight loss of 1.1% at 150°C ±3.0°C; and
preferably, a TGA spectrum of the crystalline form I is shown in FIG. 23.

**12.** A crystalline form J of the compound according to claim 1, wherein its X-ray powder diffraction pattern with Cu-Ka radiation shows characteristic peaks at 2θ angles of 9.15±0.20°, 18.08±0.20° and 19.23±0 .20°;

preferably, the X-ray powder diffraction pattern shows characteristic peaks at 2θ angles of 9.15±0.20°, 10.66 ±0.20°, 14.91±0.20°, 15.75±0.20°, 18.08±0.20°, 18.28±0.20°, 19.23±0.20° and 28.19±0.20°;
preferably, the X-ray powder diffraction pattern shows characteristic peaks at 2θ angles of 9.15±0.20°, 10.66 ±0.20°, 14.91±0.20°, 15.75±0.20°, 18.08±0.20°, 18.28±0.20°, 19.23±0.20°, 23.27±0.20°, 23.66±0.20° and 28.19±0.20°;
preferably, an XRPD pattern of the crystalline form J is shown in FIG. 24;
preferably, a differential scanning calorimetry curve of the crystalline form J shows endothermic peaks at 135.3°C ±3.0°C and 315.2±3.0°C respectively;
preferably, a DSC curve of the crystalline form J is shown in FIG. 25;
preferably, a thermogravimetric analysis curve of the crystalline form J shows a weight loss of 15.3% at 170.0°C ±3.0°C; and
preferably, a TGA spectrum of the crystalline form J is shown in FIG. 26.

**13.** A crystalline form K of the compound according to claim 1, wherein its X-ray powder diffraction pattern with Cu-Ka radiation shows characteristic peaks at 2θ angles of 9.11±0.20°, 16.30±0.20° and 18.19±0.20°,

preferably, the X-ray powder diffraction pattern shows characteristic peaks at 2θ angles of 9.11±0.20°, 16.30 ±0.20°, 17.15±0.20°, 18 .19±0.20°, 19.60±0.20°, 24.45±0.20°, 25.01±0.20° and 27.39±0.20°;
preferably, the X-ray powder diffraction pattern shows characteristic peaks at 2θ angles of 9.11±0.20°, 10.41 ±0.20°, 16.30±0.20°, 17.15±0.20°, 18.19±0.20°, 19.60±0.20°, 24.45±0.20°, 25.01±0. 20°, 27.39±0.20° and 36.75±0.20°;
preferably, an XRPD pattern of the crystalline form K is shown in FIG. 27.

**14.** Use of any one of the compound or the pharmaceutically acceptable salt thereof according to claim 1, the crystalline form A of the compound according to claim 2, the crystalline form A of the compound prepared by the method according to claim 3, the crystalline form B of the compound according to claim 4, the crystalline form C of the compound according to claim 5, the crystalline form D of the compound according to claim 6, the crystalline form E of the compound according to claim 7, the crystalline form F of the compound according to claim 8, the crystalline form G of the compound according to claim 9, the crystalline form H of the compound according to claim 10, the crystalline form I of the compound according to claim 11, the crystalline form J of the compound according to claim 12 and the crystalline form K of the compound according to claim 13 in the manufacture of a medicament for treating a metabolic disease;

preferably, the medicament is used as a thyroid hormone β receptor agonist;
preferably, the metabolic disease includes obesity, hyperlipidemia, hypercholesterolemia, diabetes, hepatic steatosis, nonalcoholic steatohepatitis, atherosclerosis, cardiovascular diseases, thyroid diseases, and tumor in intrahepatic bile duct.

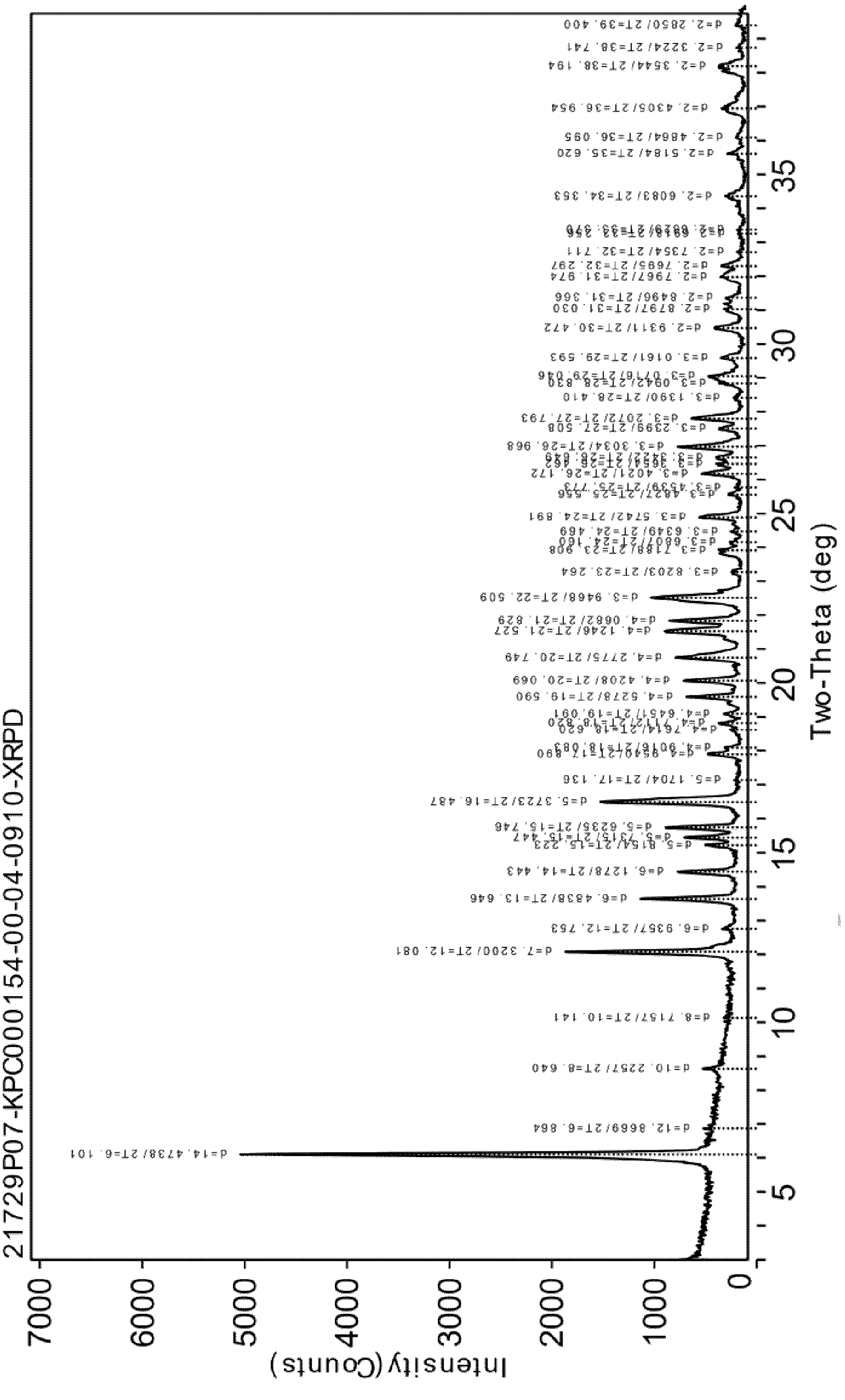
21729P07-KPC000154-00-04-0910-XRPD
Intensity (Counts)
Two-Theta (deg)
0
1000
2000
3000
4000
5000
6000
7000
5
10
15
20
25
30
35

FIG.1

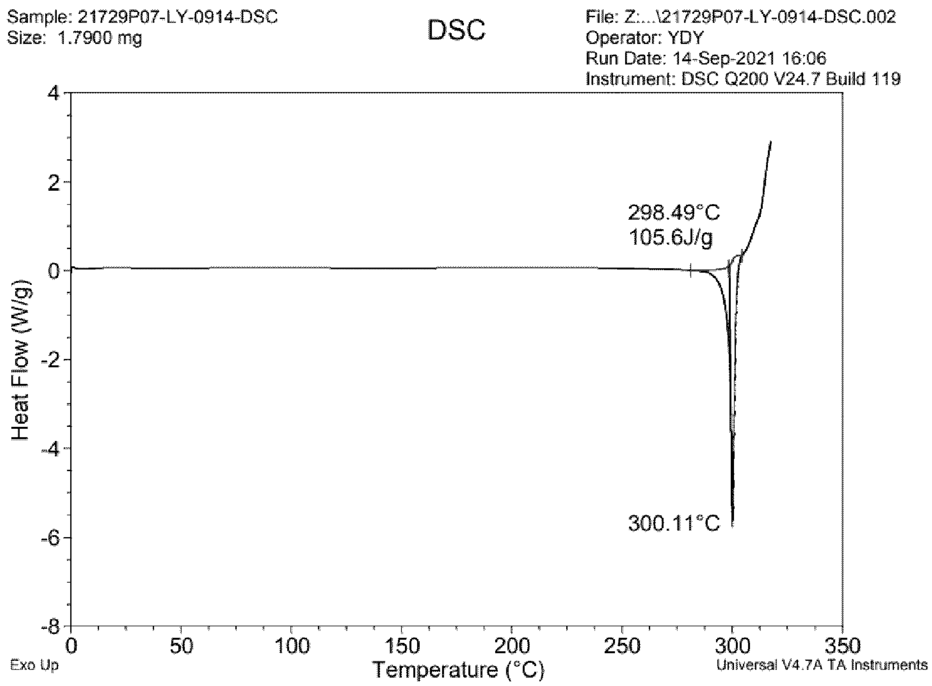
Sample: 21729P07-LY-0914-DSC
Size: 1.7900 mg
DSC
File: Z:...\21729P07-LY-0914-DSC.002
Operator: YDY
Run Date: 14-Sep-2021 16:06
Instrument: DSC Q200 V24.7 Build 119
298.49°C
105.6J/g
300.11°C
Heat Flow (W/g)
Temperature (°C)
Exo Up
Universal V4.7A TA Instruments

FIG.2

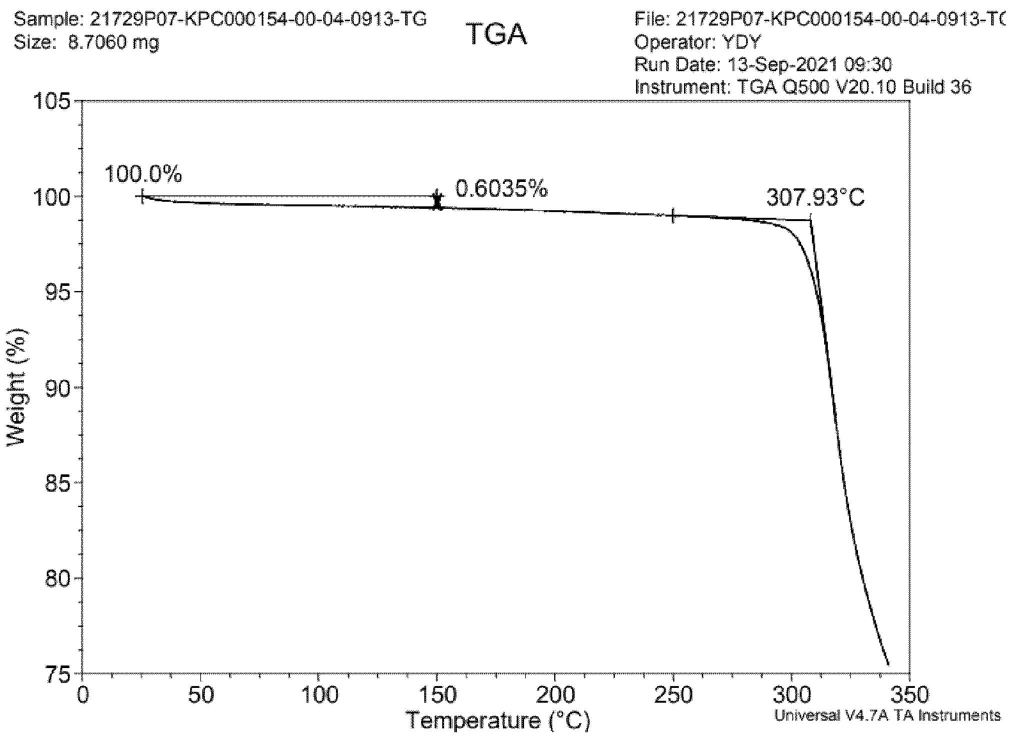
Sample: 21729P07-KPC000154-00-04-0913-TG
Size: 8.7060 mg
TGA
File: 21729P07-KPC000154-00-04-0913-T(
Operator: YDY
Run Date: 13-Sep-2021 09:30
Instrument: TGA Q500 V20.10 Build 36
100.0%
0.6035%
307.93°C
Weight (%)
Temperature (°C)
Universal V4.7A TA Instruments

FIG.3

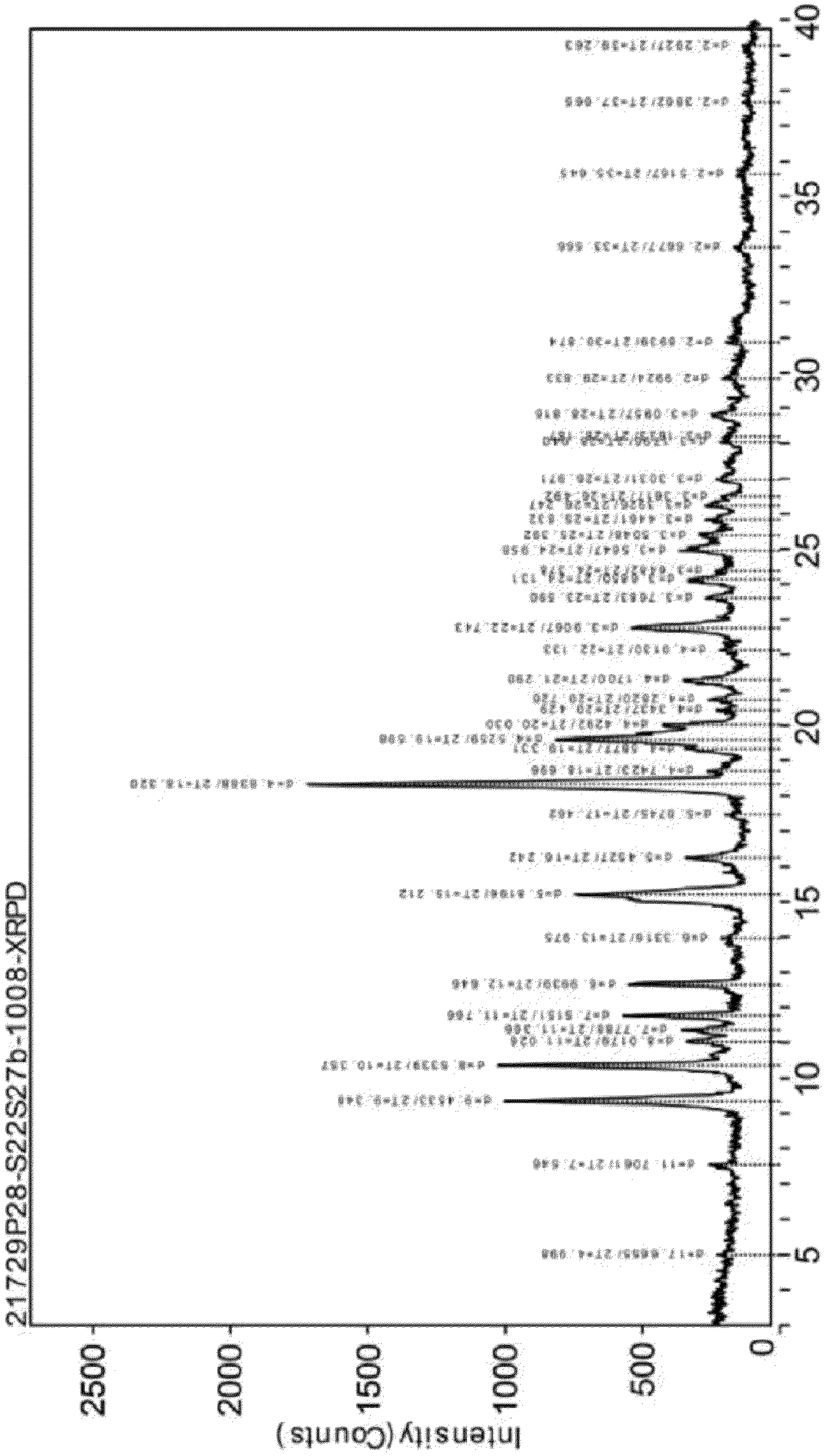


FIG.4

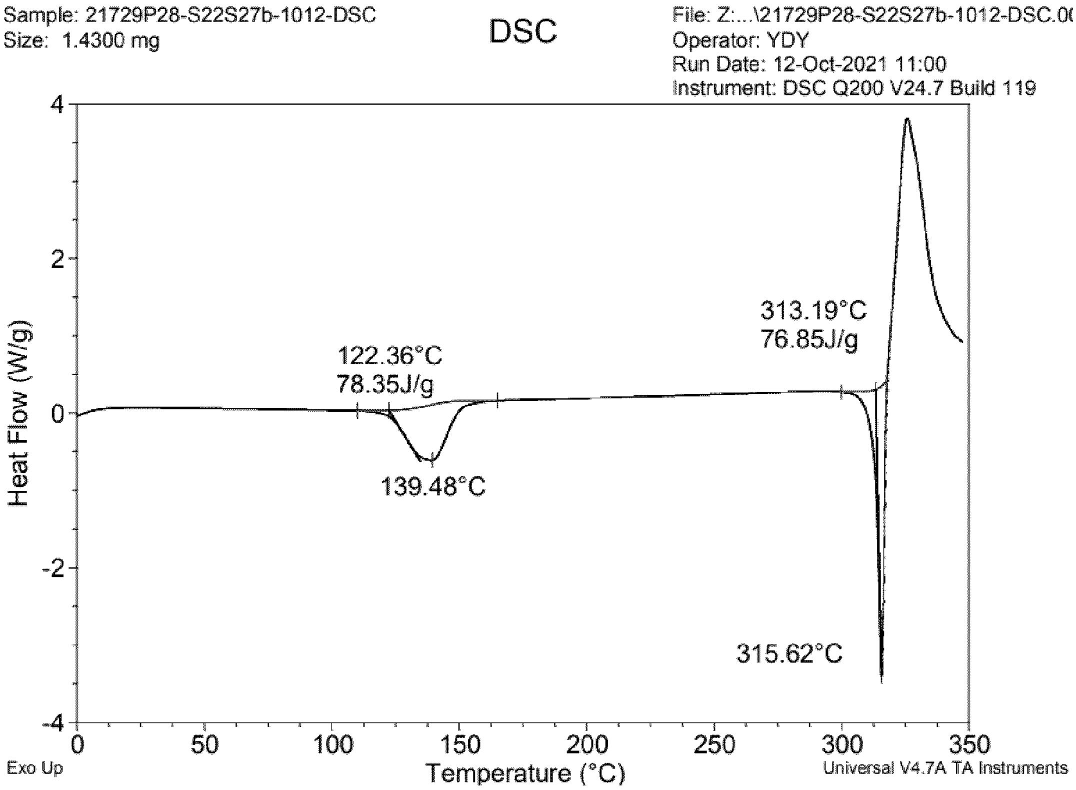
Sample: 21729P28-S22S27b-1012-DSC
Size: 1.4300 mg
DSC
File: Z:...\21729P28-S22S27b-1012-DSC.0(
Operator: YDY
Run Date: 12-Oct-2021 11:00
Instrument: DSC Q200 V24.7 Build 119
Heat Flow (W/g)
122.36°C
78.35J/g
139.48°C
313.19°C
76.85J/g
315.62°C
Exo Up
Temperature (°C)
Universal V4.7A TA Instruments

FIG.5

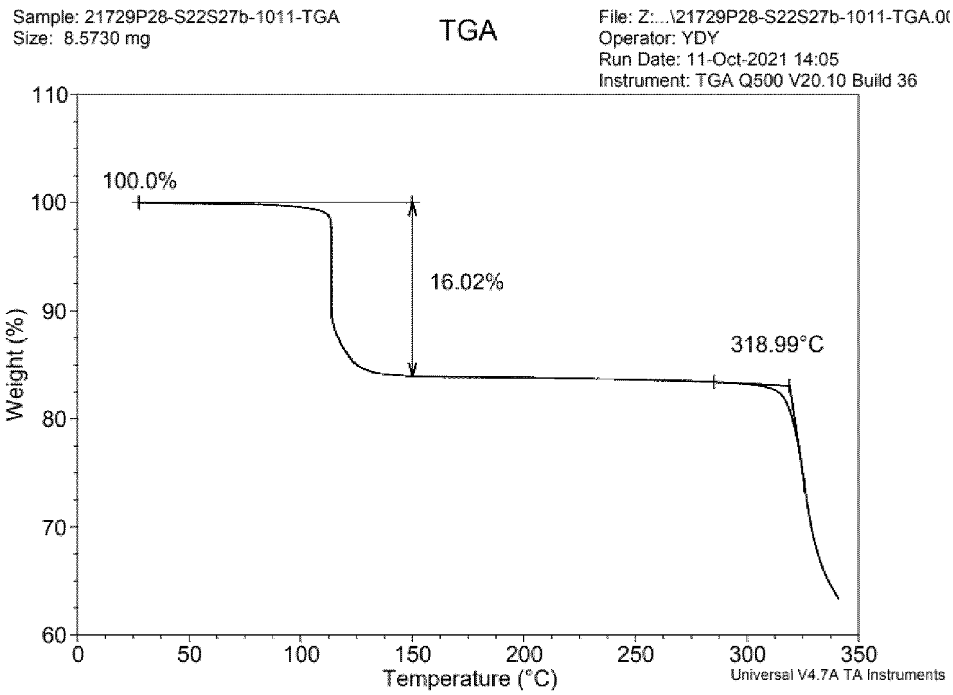
Sample: 21729P28-S22S27b-1011-TGA
Size: 8.5730 mg
TGA
File: Z:...\21729P28-S22S27b-1011-TGA.0(
Operator: YDY
Run Date: 11-Oct-2021 14:05
Instrument: TGA Q500 V20.10 Build 36
Weight (%)
100.0%
16.02%
318.99°C
Temperature (°C)
Universal V4.7A TA Instruments

FIG.6

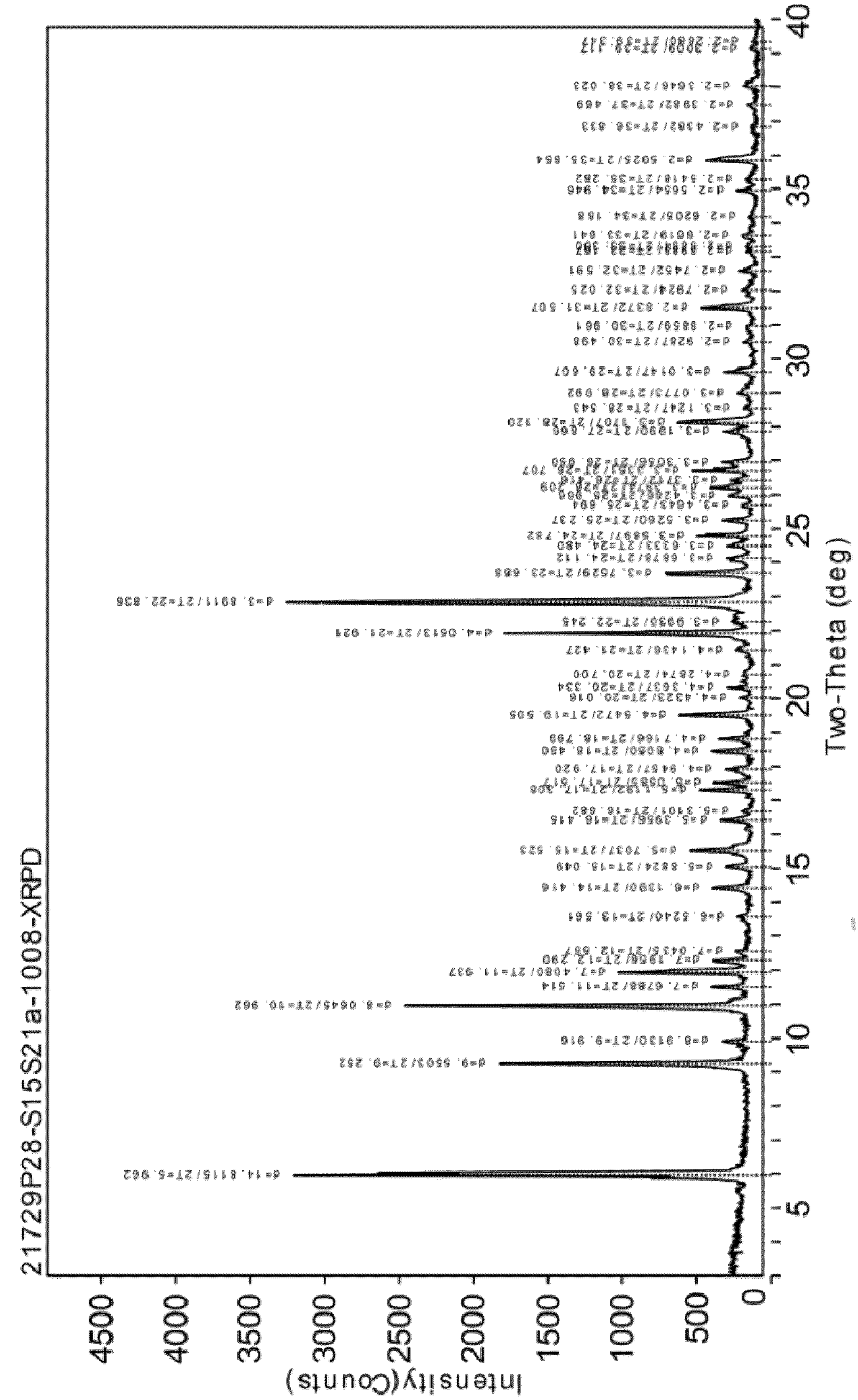


FIG.7

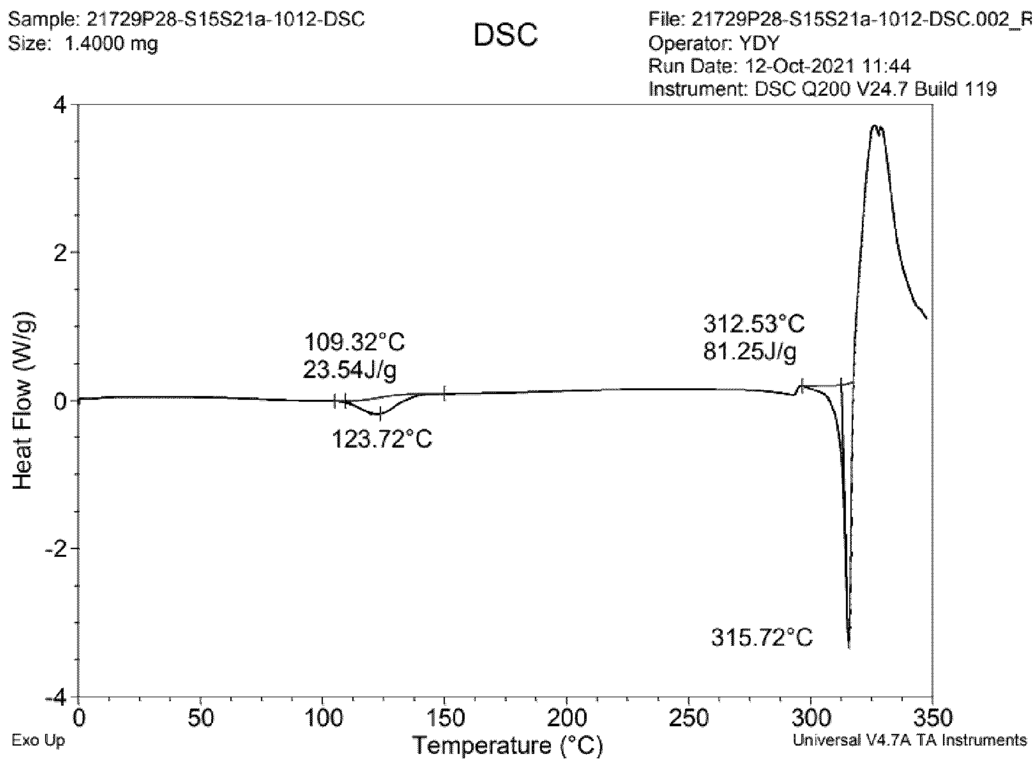
Sample: 21729P28-S15S21a-1012-DSC
Size: 1.4000 mg
DSC
File: 21729P28-S15S21a-1012-DSC.002_R
Operator: YDY
Run Date: 12-Oct-2021 11:44
Instrument: DSC Q200 V24.7 Build 119
109.32°C
23.54J/g
123.72°C
312.53°C
81.25J/g
315.72°C
Heat Flow (W/g)
Exo Up
Temperature (°C)
Universal V4.7A TA Instruments

FIG.8

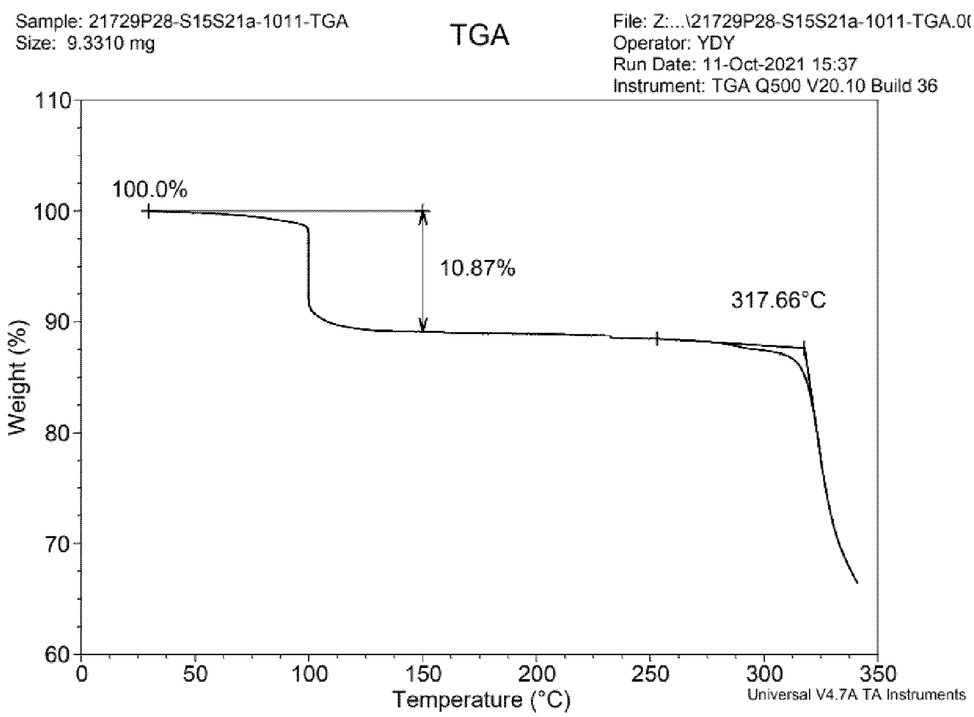
Sample: 21729P28-S15S21a-1011-TGA
Size: 9.3310 mg
TGA
File: Z:...\21729P28-S15S21a-1011-TGA.0(
Operator: YDY
Run Date: 11-Oct-2021 15:37
Instrument: TGA Q500 V20.10 Build 36
100.0%
10.87%
317.66°C
Weight (%)
Temperature (°C)
Universal V4.7A TA Instruments

FIG.9

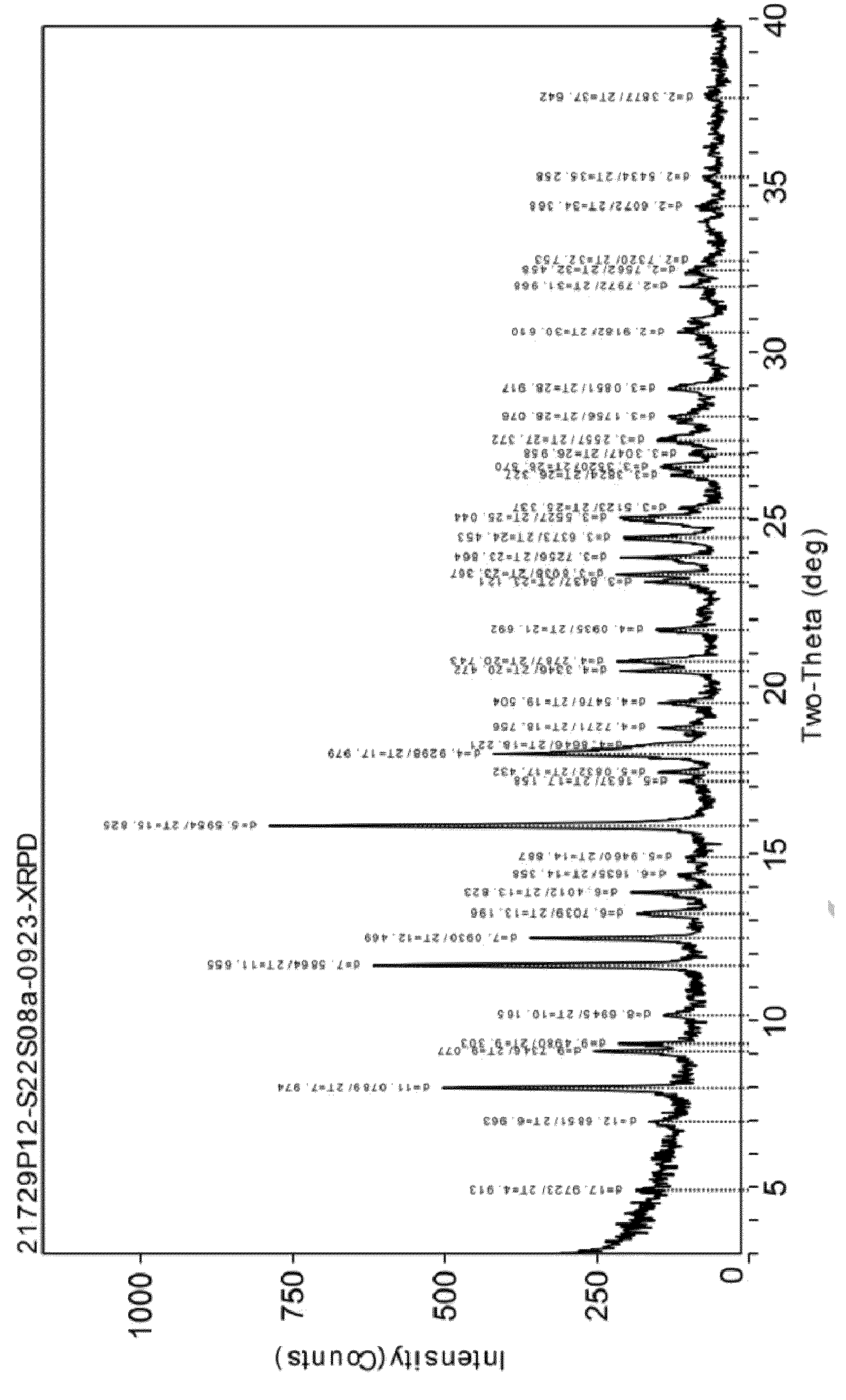
21729P12-S22S08a-0923-XRPD
Intensity(Counts)
0
250
500
750
1000
Two-Theta (deg)
5
10
15
20
25
30
35
40

FIG.10

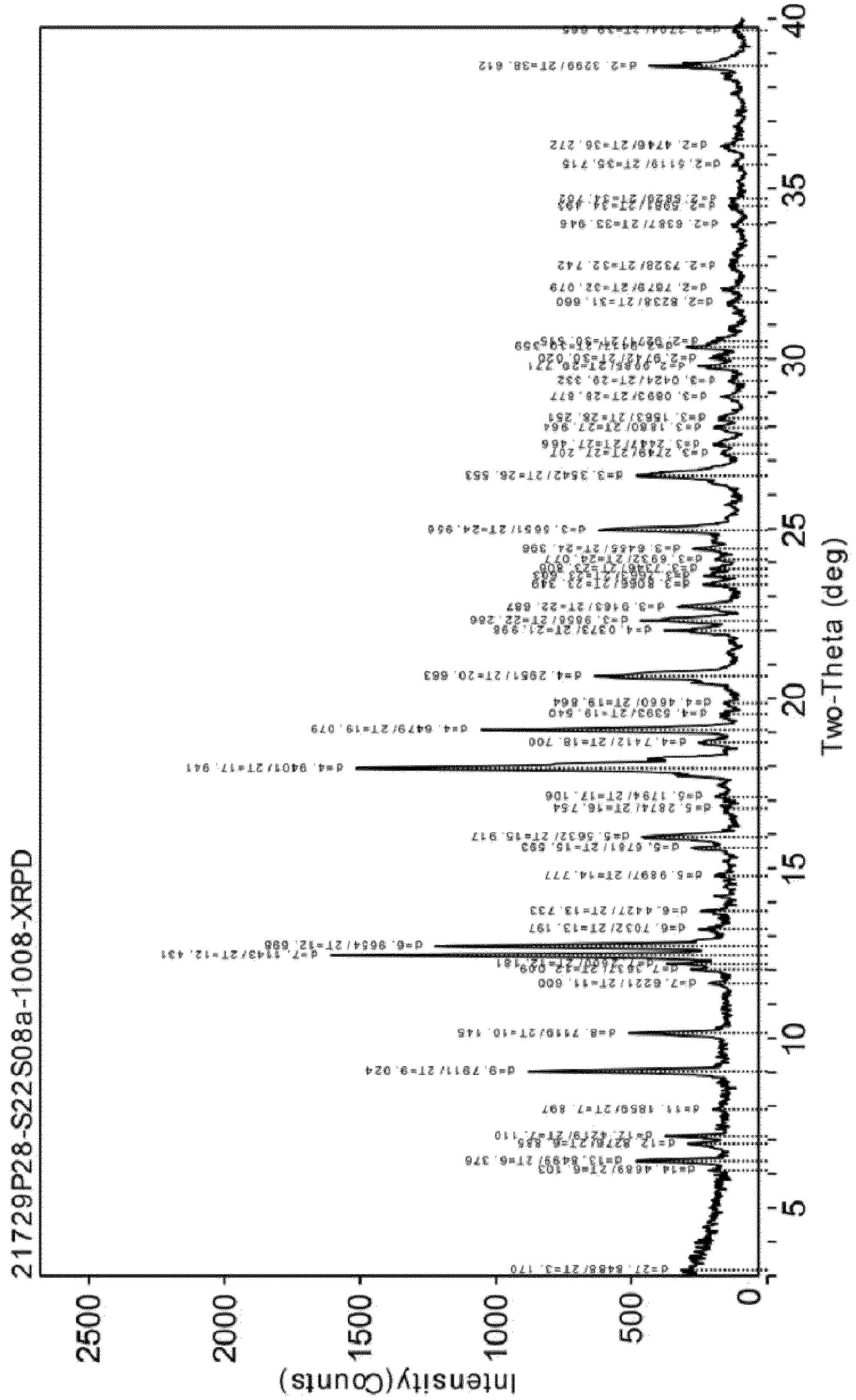
21729P28-S22S08a-1008-XRPD
Intensity(Counts)
0
500
1000
1500
2000
2500
Two-Theta (deg)
5
10
15
20
25
30
35
40

FIG.11

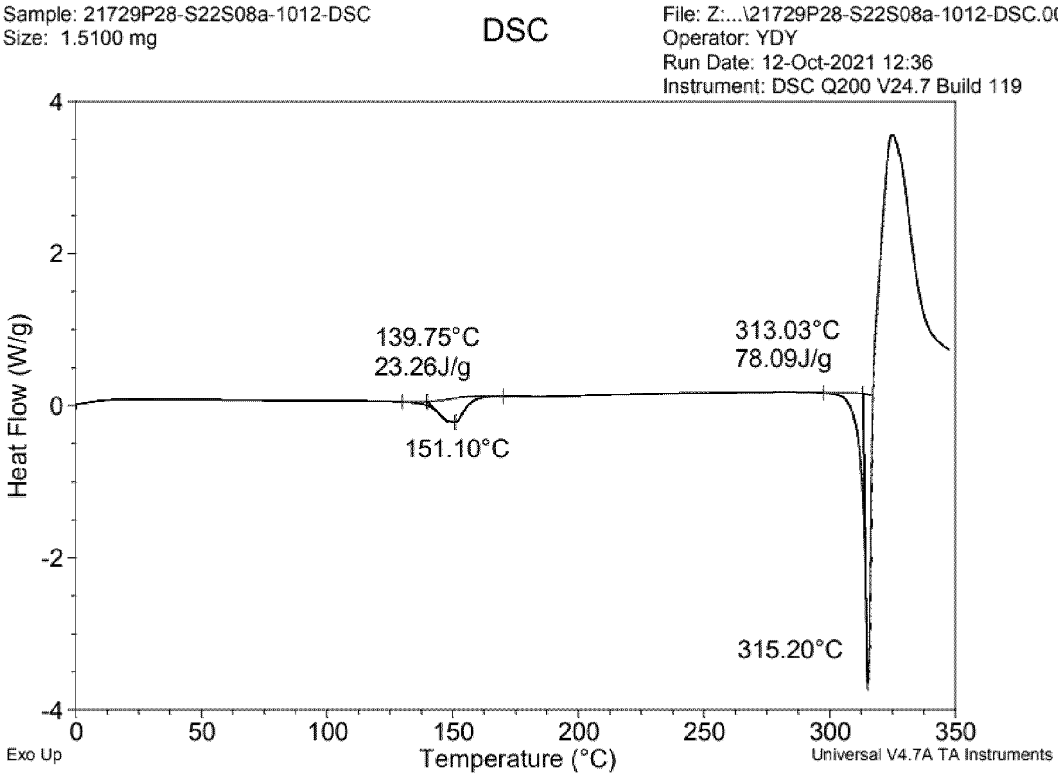
Sample: 21729P28-S22S08a-1012-DSC
Size: 1.5100 mg
DSC
File: Z:...\21729P28-S22S08a-1012-DSC.0(
Operator: YDY
Run Date: 12-Oct-2021 12:36
Instrument: DSC Q200 V24.7 Build 119
Heat Flow (W/g)
139.75°C
23.26J/g
151.10°C
313.03°C
78.09J/g
315.20°C
Exo Up
Temperature (°C)
Universal V4.7A TA Instruments

FIG.12

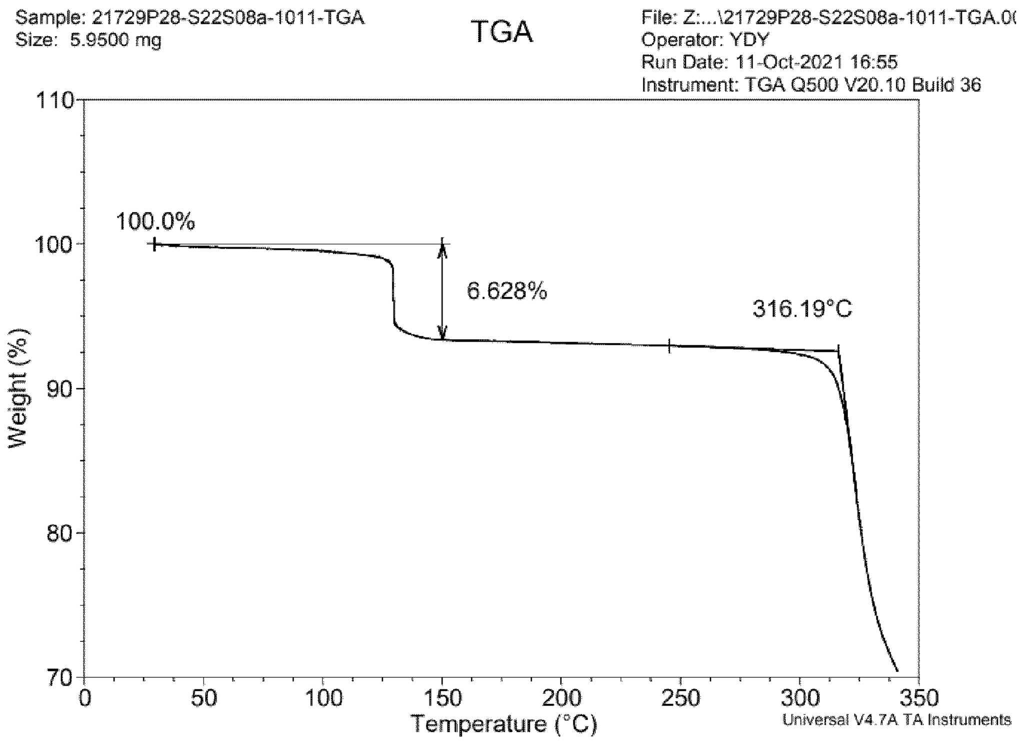
Sample: 21729P28-S22S08a-1011-TGA
Size: 5.9500 mg
TGA
File: Z:...\21729P28-S22S08a-1011-TGA.0(
Operator: YDY
Run Date: 11-Oct-2021 16:55
Instrument: TGA Q500 V20.10 Build 36
Weight (%)
100.0%
6.628%
316.19°C
Temperature (°C)
Universal V4.7A TA Instruments

FIG.13

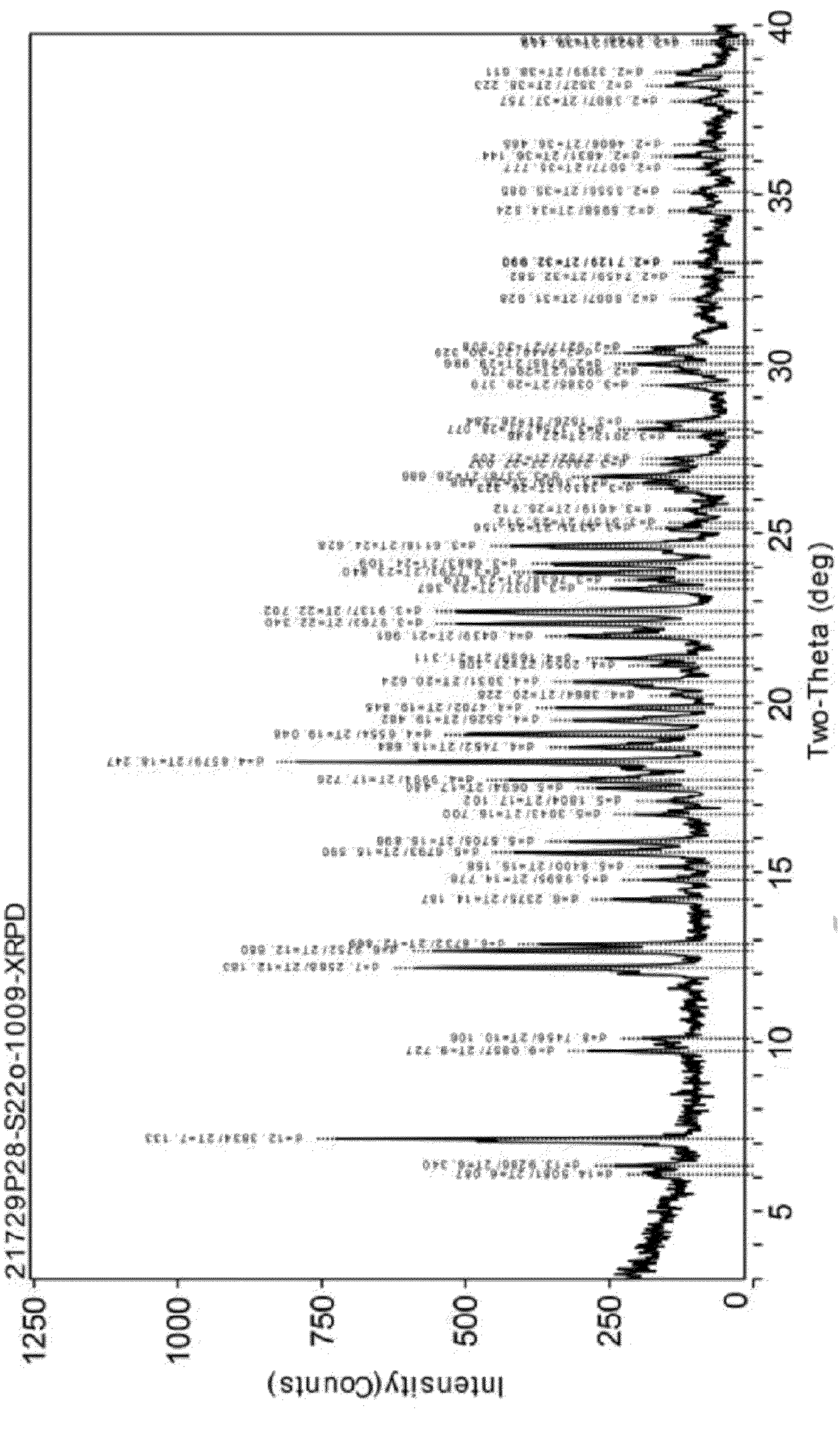


FIG.14

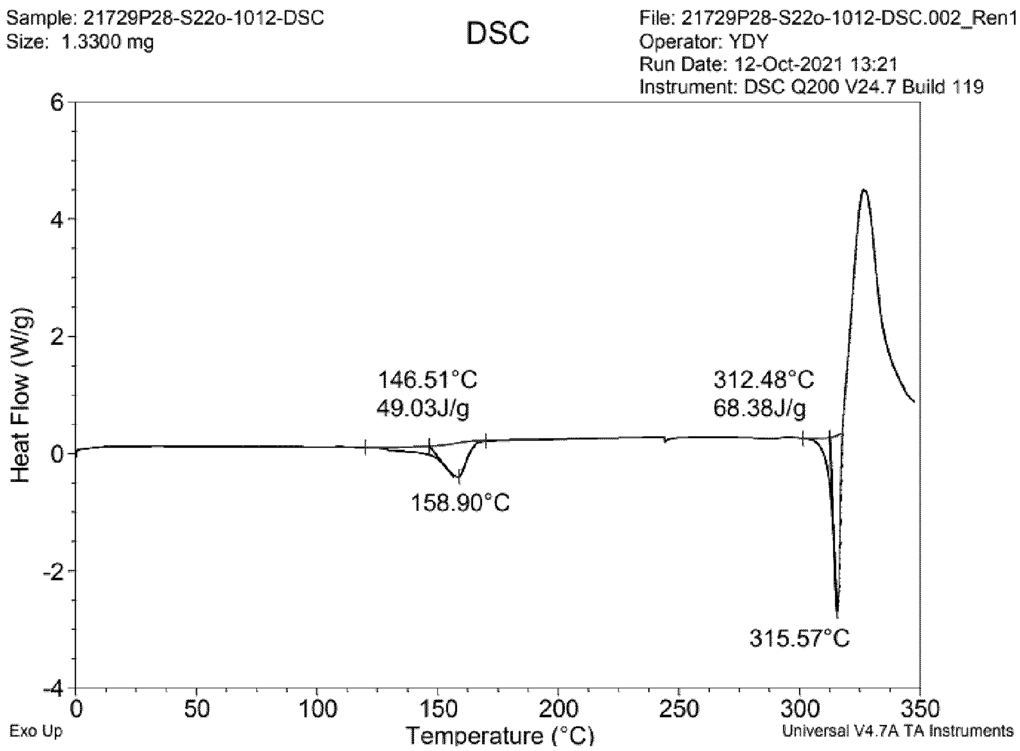
Sample: 21729P28-S22o-1012-DSC
Size: 1.3300 mg
DSC
File: 21729P28-S22o-1012-DSC.002_Ren1
Operator: YDY
Run Date: 12-Oct-2021 13:21
Instrument: DSC Q200 V24.7 Build 119
Heat Flow (W/g)
146.51°C
49.03J/g
158.90°C
312.48°C
68.38J/g
315.57°C
Exo Up
Temperature (°C)
Universal V4.7A TA Instruments

FIG.15

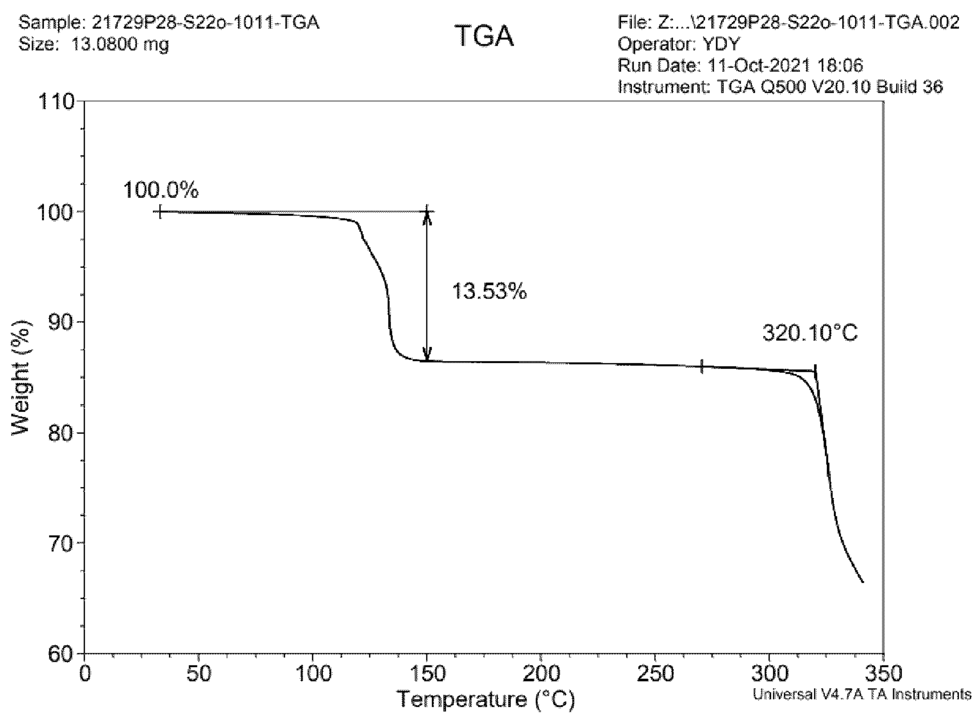
Sample: 21729P28-S22o-1011-TGA
Size: 13.0800 mg
TGA
File: Z:...\21729P28-S22o-1011-TGA.002
Operator: YDY
Run Date: 11-Oct-2021 18:06
Instrument: TGA Q500 V20.10 Build 36
Weight (%)
100.0%
13.53%
320.10°C
Temperature (°C)
Universal V4.7A TA Instruments

FIG.16

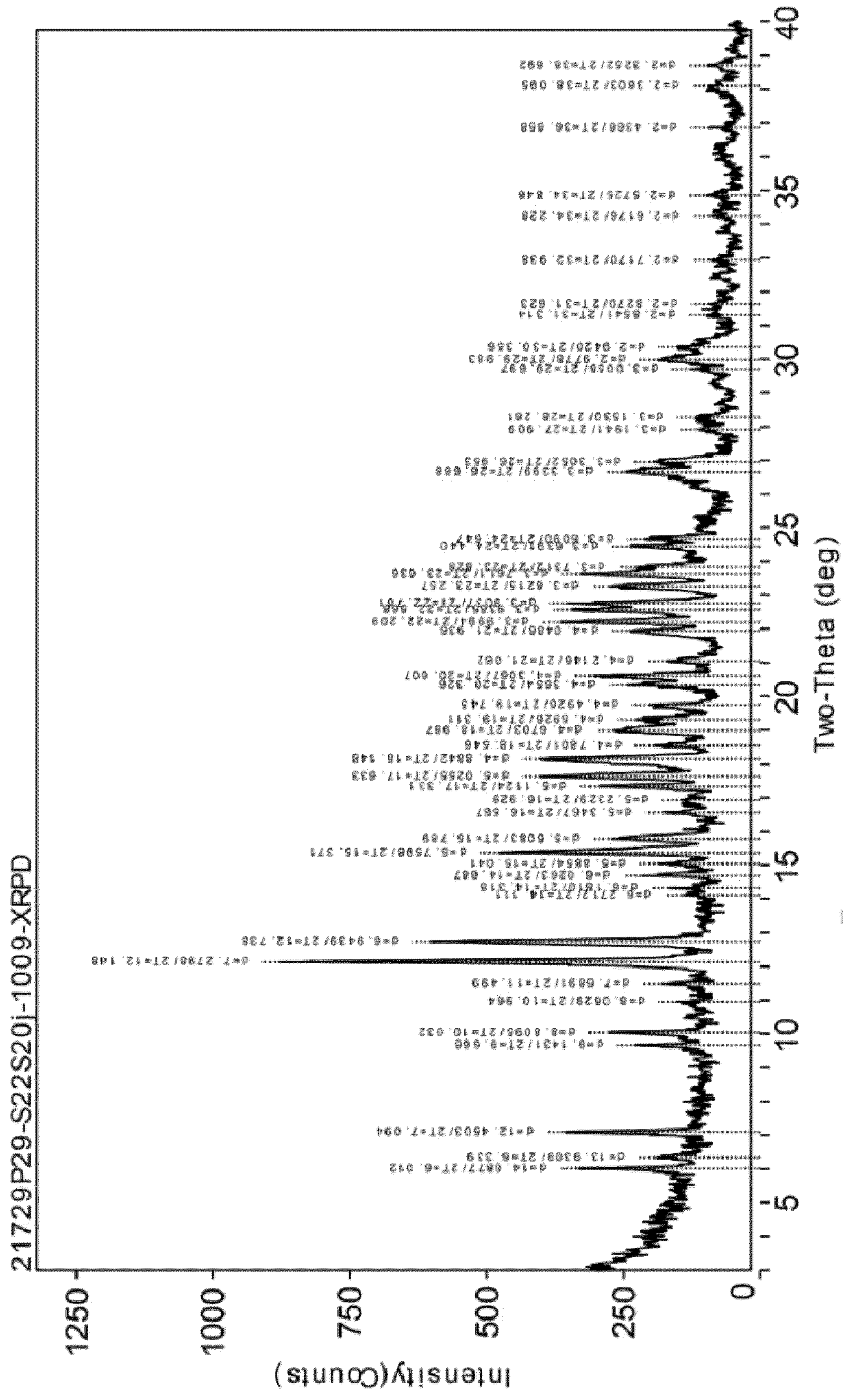


FIG.17

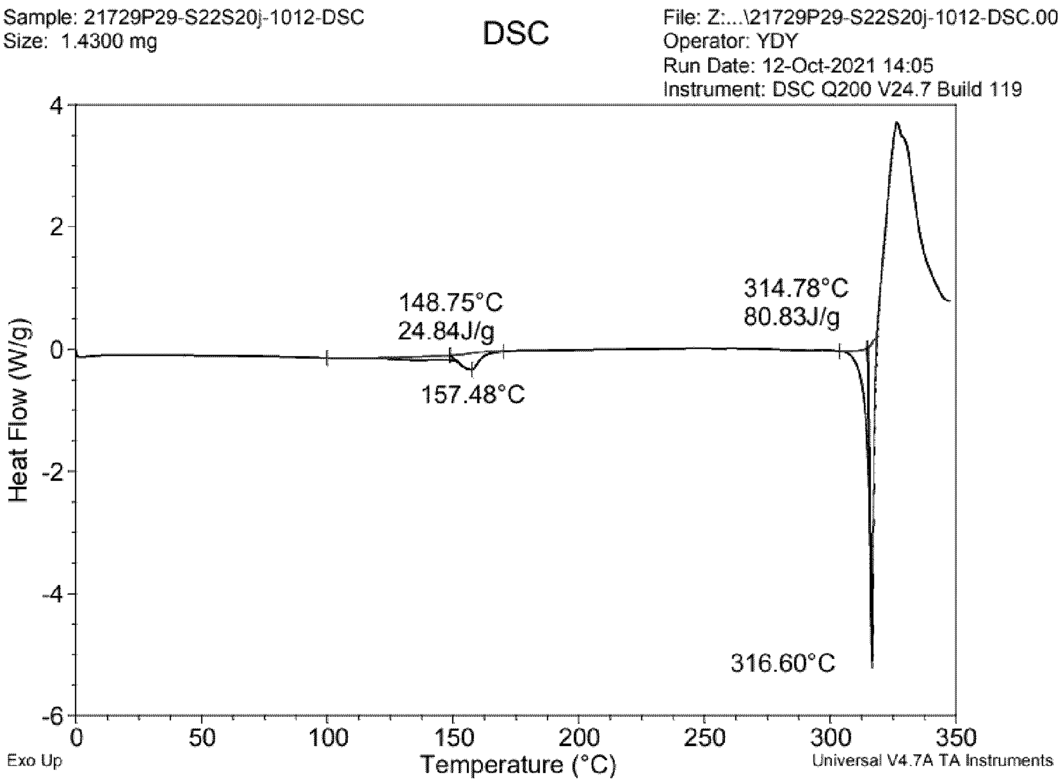
Sample: 21729P29-S22S20j-1012-DSC
Size: 1.4300 mg
DSC
File: Z:...\21729P29-S22S20j-1012-DSC.00
Operator: YDY
Run Date: 12-Oct-2021 14:05
Instrument: DSC Q200 V24.7 Build 119
148.75°C
24.84J/g
157.48°C
314.78°C
80.83J/g
316.60°C
Heat Flow (W/g)
Temperature (°C)
Exo Up
Universal V4.7A TA Instruments

FIG.18

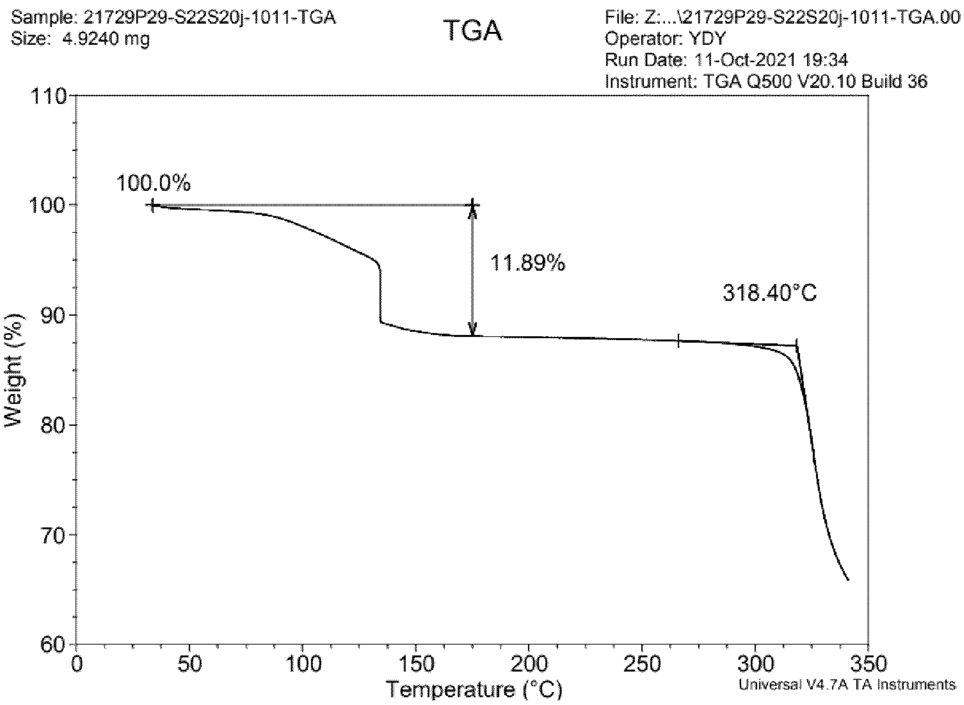
Sample: 21729P29-S22S20j-1011-TGA
Size: 4.9240 mg
TGA
File: Z:...\21729P29-S22S20j-1011-TGA.00
Operator: YDY
Run Date: 11-Oct-2021 19:34
Instrument: TGA Q500 V20.10 Build 36
100.0%
11.89%
318.40°C
Weight (%)
Temperature (°C)
Universal V4.7A TA Instruments

FIG.19

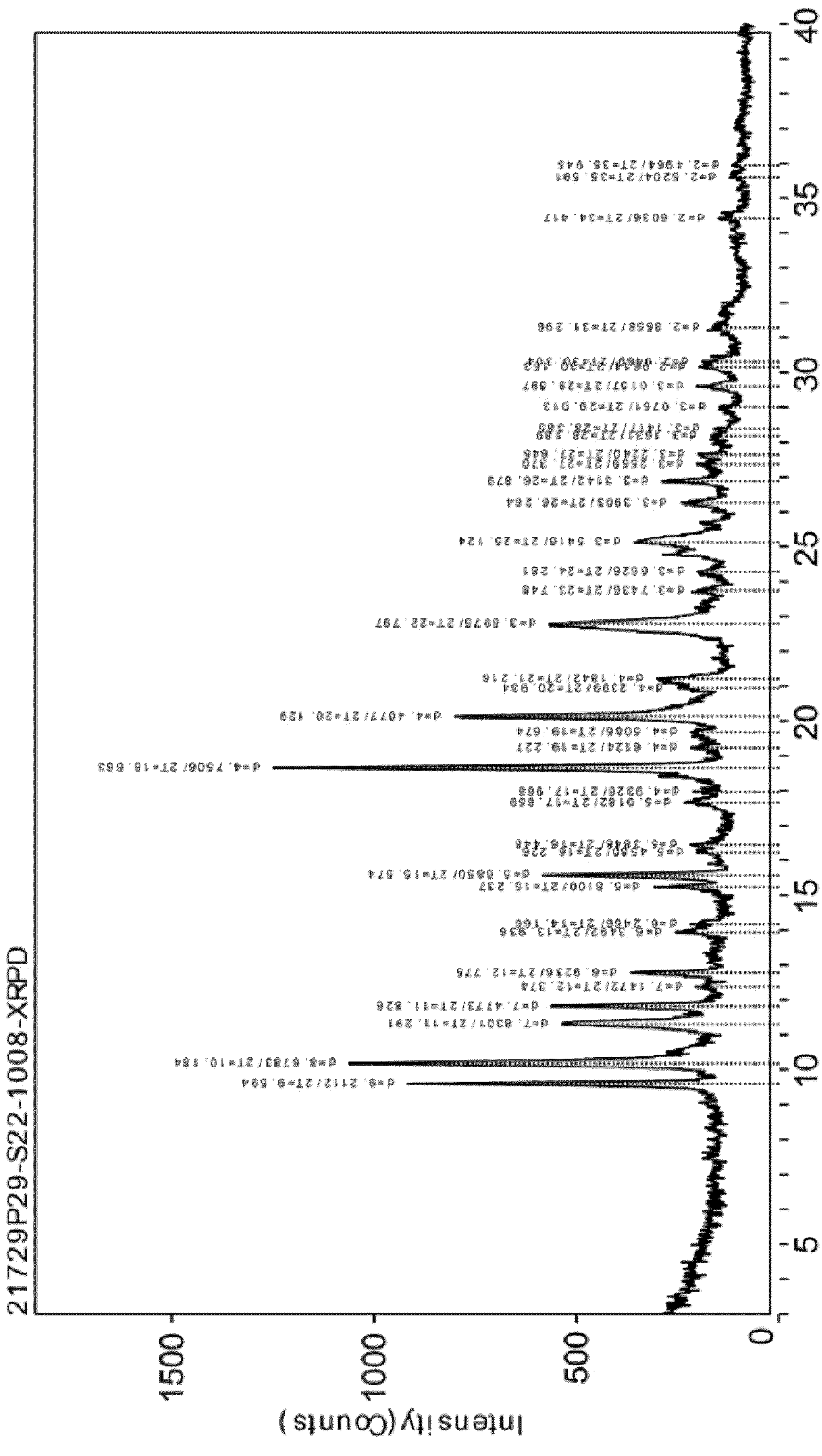
21729P29-S22-1008-XRPD
Intensity (Counts)
0
500
1000
1500
Two-Theta (deg)
5
10
15
20
25
30
35
40

FIG.20

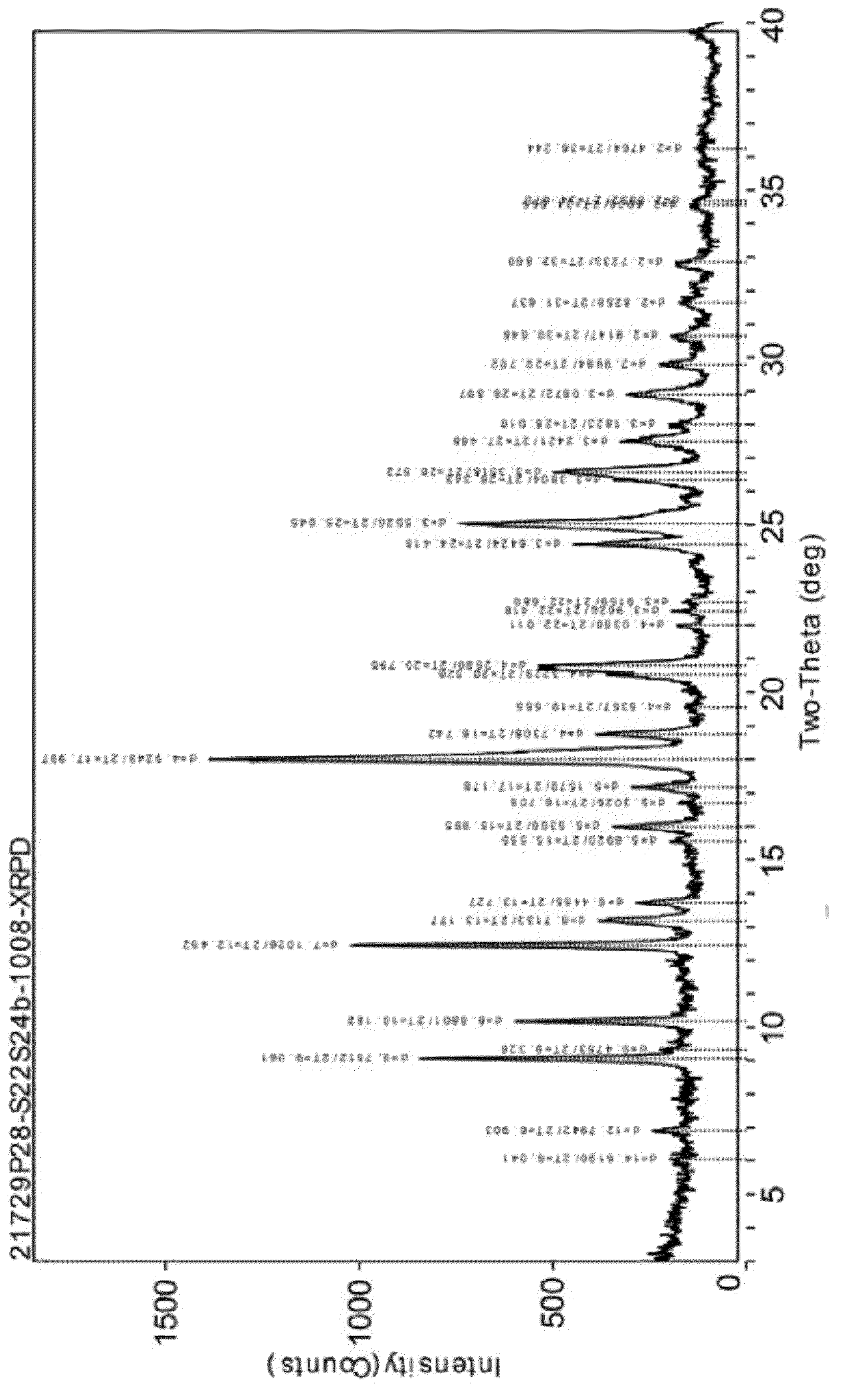
21729P28-S22S24b-1008-XRPD
Intensity (Counts)
Two-Theta (deg)
0
500
1000
1500
5
10
15
20
25
30
35
40

FIG.21

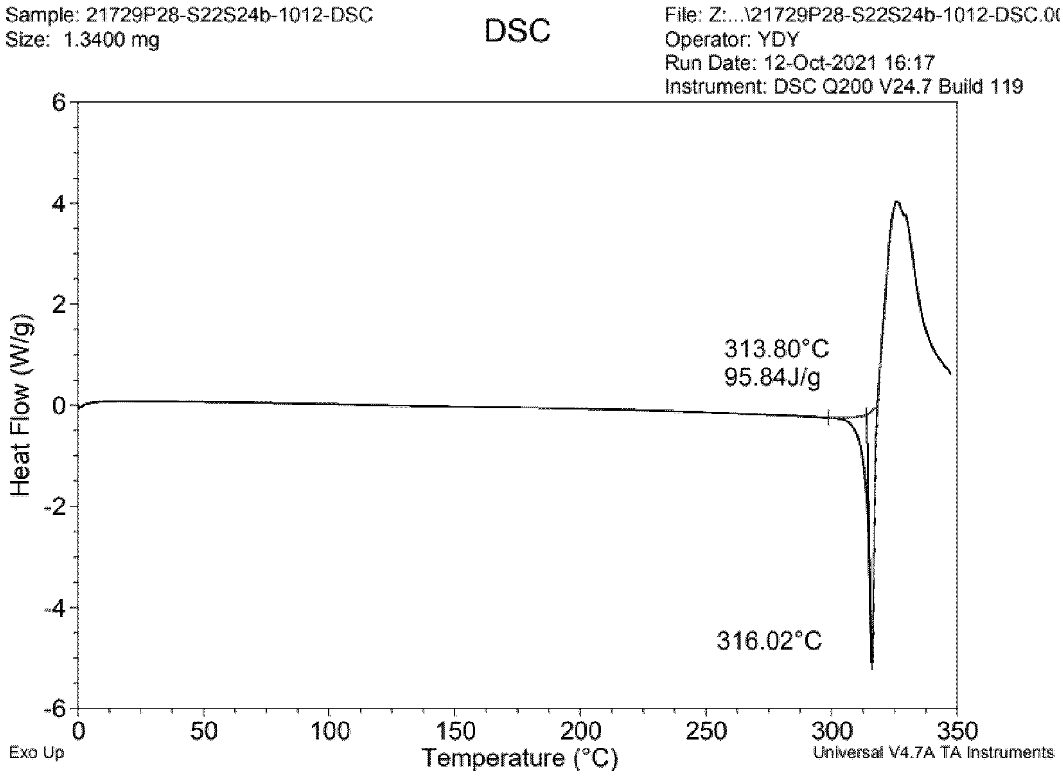
Sample: 21729P28-S22S24b-1012-DSC
Size: 1.3400 mg
DSC
File: Z:...\21729P28-S22S24b-1012-DSC.0(
Operator: YDY
Run Date: 12-Oct-2021 16:17
Instrument: DSC Q200 V24.7 Build 119
Heat Flow (W/g)
313.80°C
95.84J/g
316.02°C
Exo Up
Temperature (°C)
Universal V4.7A TA Instruments

FIG.22

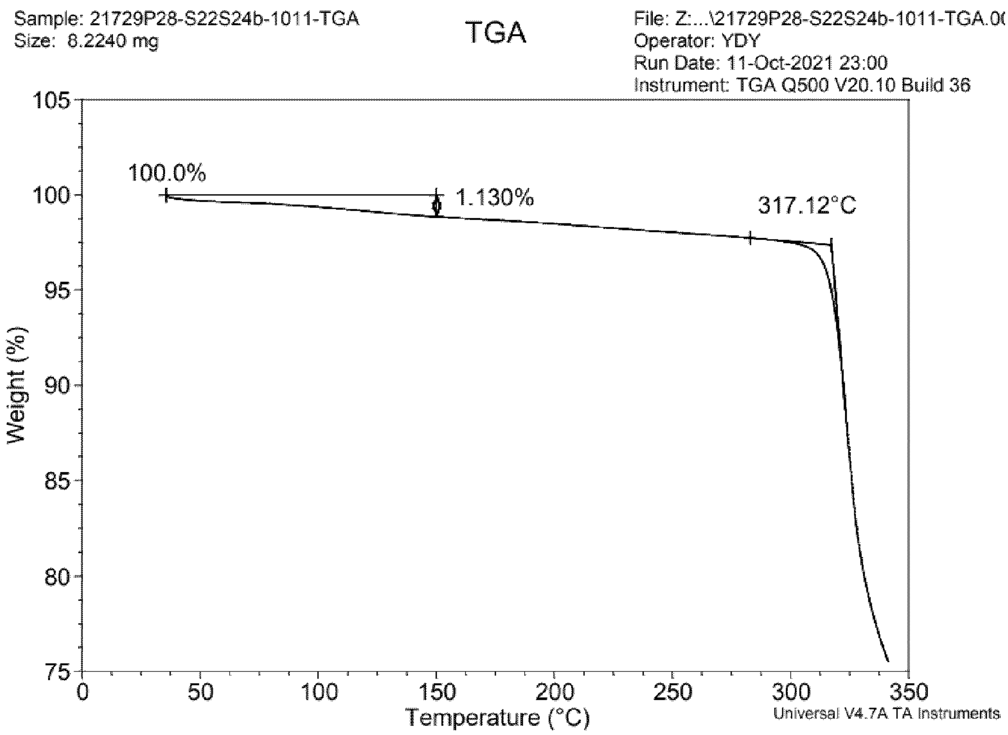
Sample: 21729P28-S22S24b-1011-TGA
Size: 8.2240 mg
TGA
File: Z:...\21729P28-S22S24b-1011-TGA.0(
Operator: YDY
Run Date: 11-Oct-2021 23:00
Instrument: TGA Q500 V20.10 Build 36
Weight (%)
100.0%
1.130%
317.12°C
Temperature (°C)
Universal V4.7A TA Instruments

FIG.23

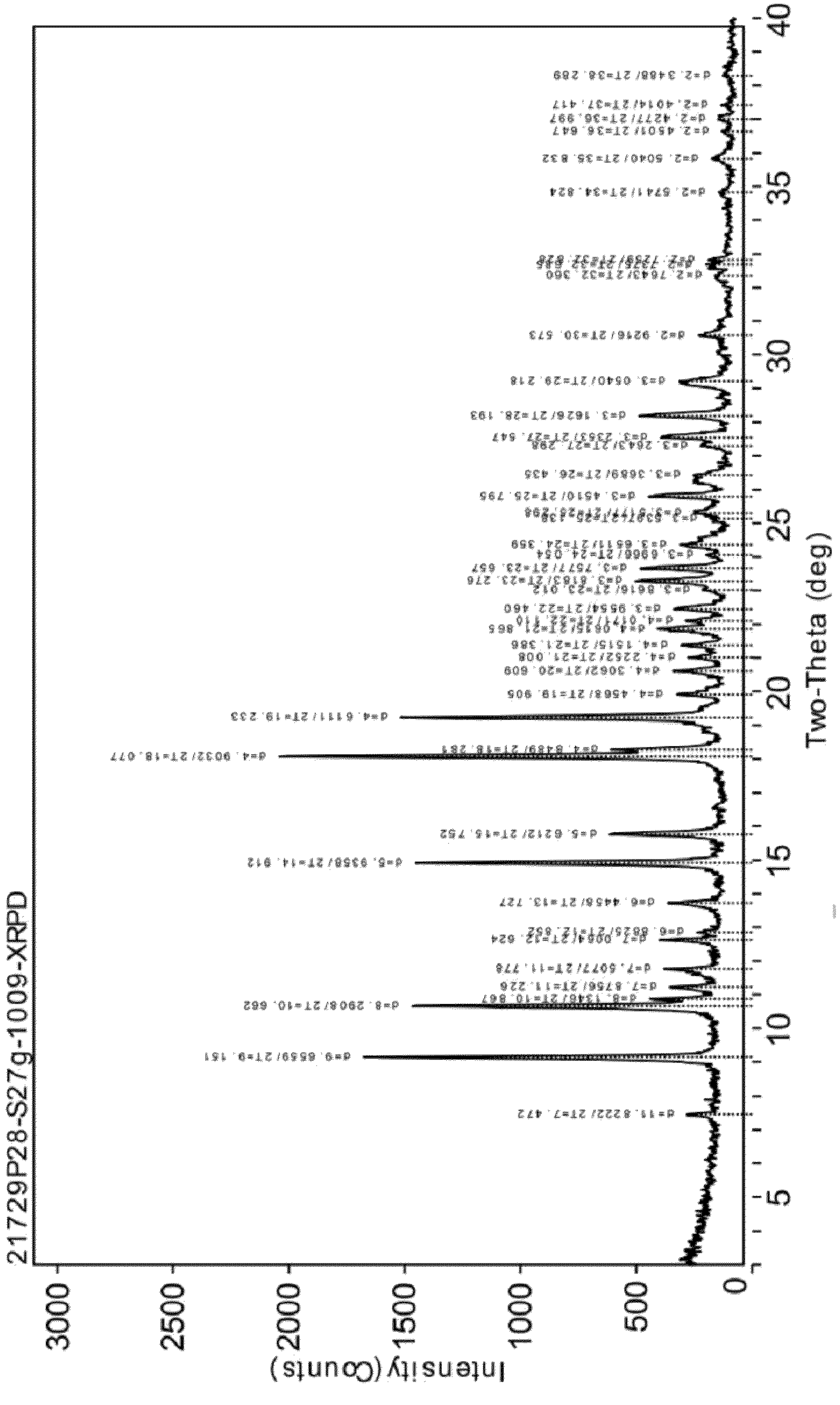


FIG.24

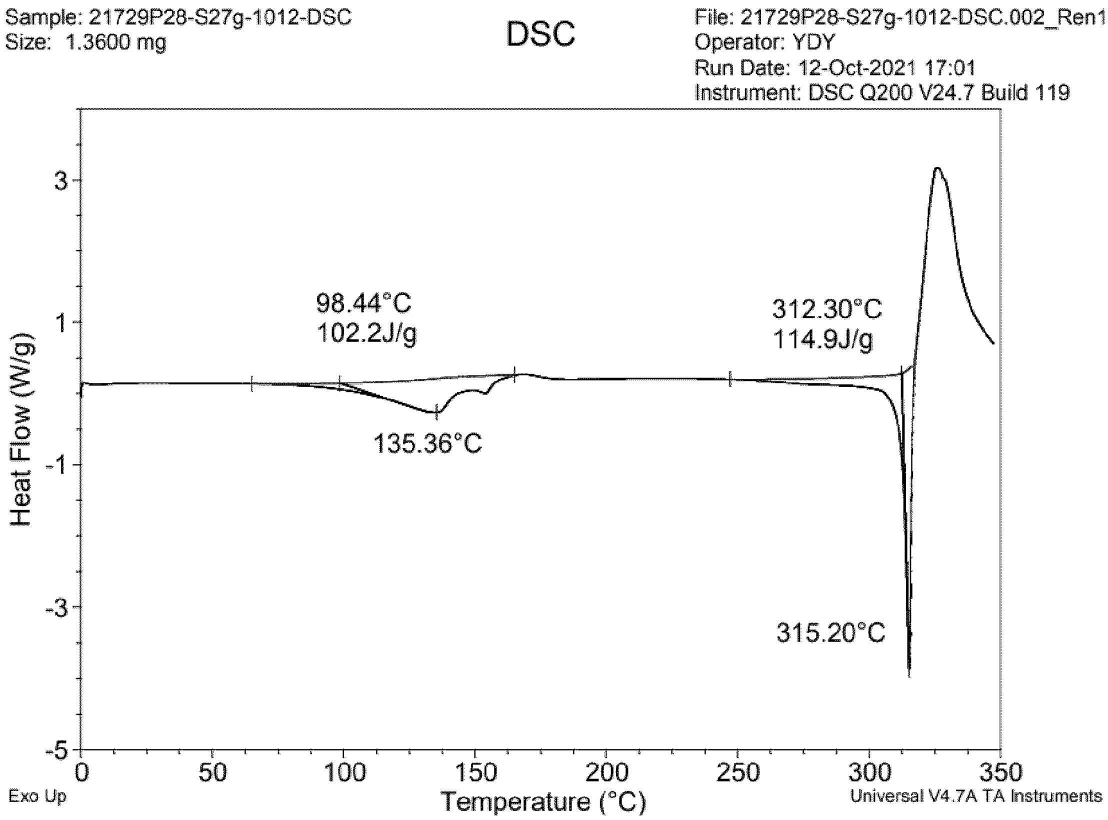
Sample: 21729P28-S27g-1012-DSC
Size: 1.3600 mg
DSC
File: 21729P28-S27g-1012-DSC.002_Ren1
Operator: YDY
Run Date: 12-Oct-2021 17:01
Instrument: DSC Q200 V24.7 Build 119
98.44°C
102.2J/g
135.36°C
312.30°C
114.9J/g
315.20°C
Heat Flow (W/g)
Temperature (°C)
Exo Up
Universal V4.7A TA Instruments

FIG.25

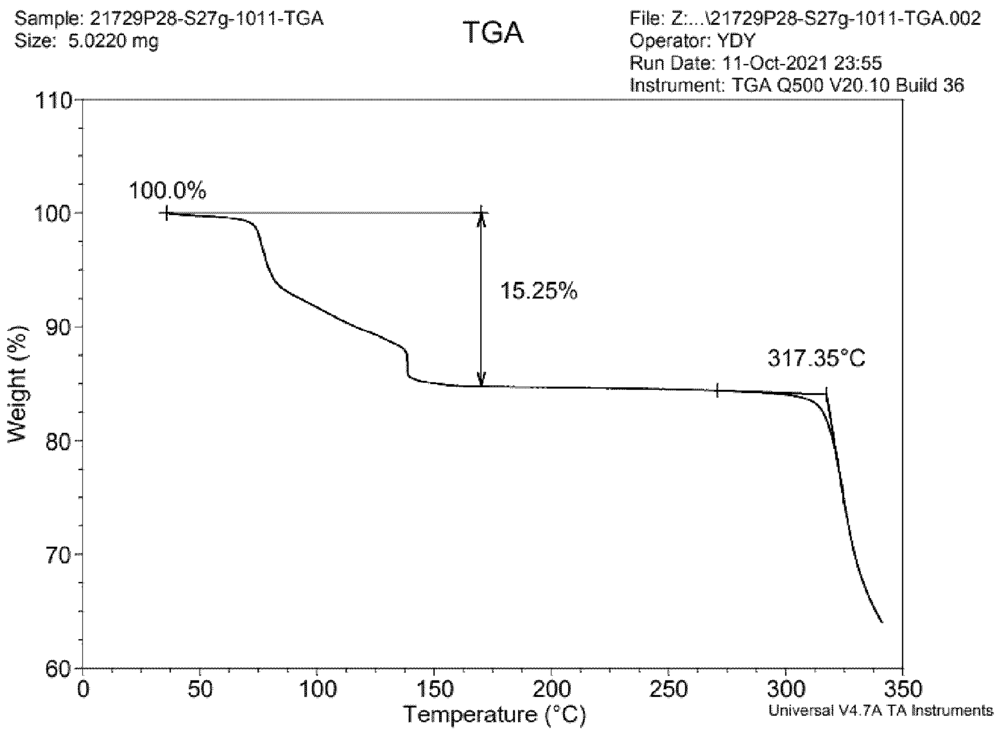
Sample: 21729P28-S27g-1011-TGA
Size: 5.0220 mg
TGA
File: Z:...\21729P28-S27g-1011-TGA.002
Operator: YDY
Run Date: 11-Oct-2021 23:55
Instrument: TGA Q500 V20.10 Build 36
100.0%
15.25%
317.35°C
Weight (%)
Temperature (°C)
Universal V4.7A TA Instruments

FIG.26

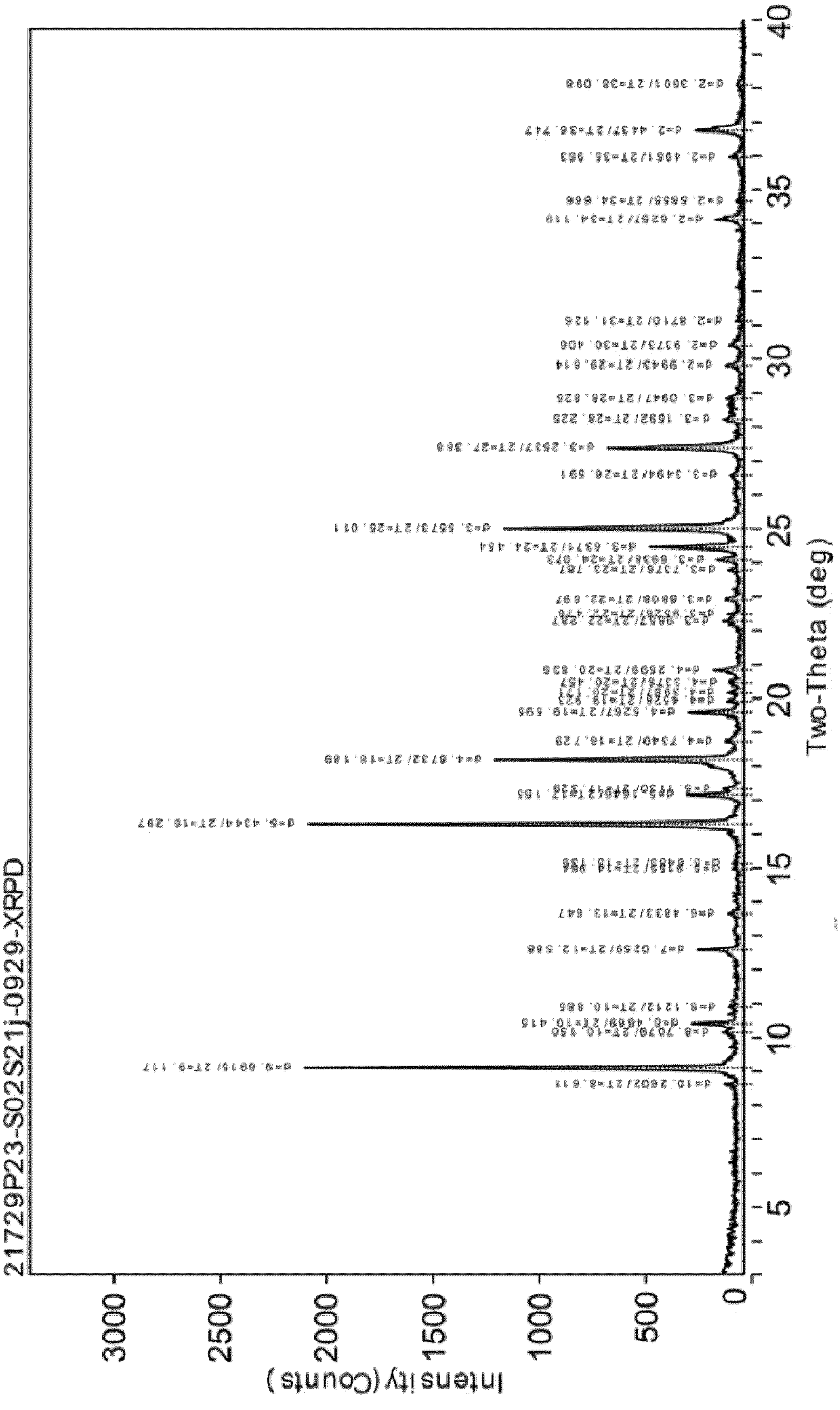


FIG.27

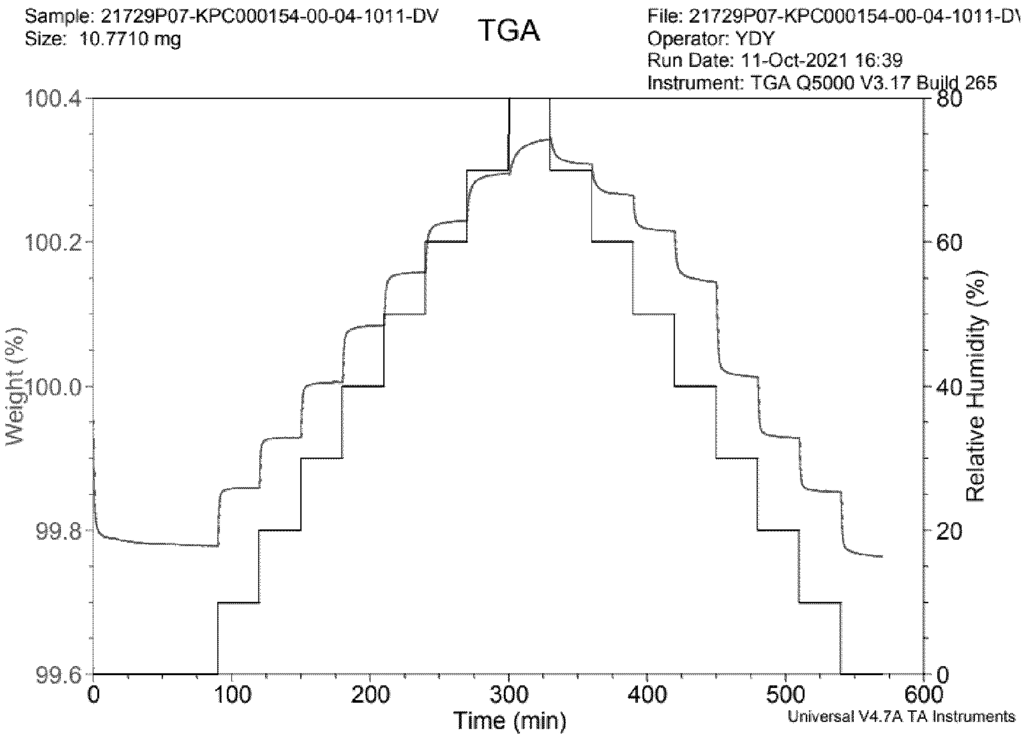

Sample: 21729P07-KPC000154-00-04-1011-DV
Size: 10.7710 mg
TGA
File: 21729P07-KPC000154-00-04-1011-D\
Operator: YDY
Run Date: 11-Oct-2021 16:39
Instrument: TGA Q5000 V3.17 Build 265
Weight (%)
Relative Humidity (%)
Time (min)
Universal V4.7A TA Instruments
100.4
100.2
100.0
99.8
99.6
80
60
40
20
0
0
100
200
300
400
500
600


FIG.28

**INTERNATIONAL SEARCH REPORT**

International application No.
**PCT/CN2023/136702**

**A. CLASSIFICATION OF SUBJECT MATTER**

C07D471/04(2006.01)i; A61K 31/53(2006.01)i; A61P3/04(2006.01)i; A61P3/06(2006.01)i; A61P3/10(2006.01)i; A61P1/16(2006.01)i; A61P9/10(2006.01)i; A61P9/00(2006.01)i; A61P17/14(2006.01)i; A61P35/00(2006.01)i; A61P5/14(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, CNKI, ISI Web of Knowledge, CAPLUS(STN), REGISTRY(STN): 昆药集团股份有限公司, 杨海利, 席亮, 刘军锋, 蒋关禹, 徐安超, 李奎, 甲状腺激素受体, 四氢吲嗪, 三嗪, 甲腈, tetrahydroindoliz+, triazin+, carbonitrile, THR, thyroid hormone

**C. DOCUMENTS CONSIDERED TO BE RELEVANT**

| Category* | Citation of document, with indication, where appropriate, of the relevant passages | Relevant to claim No. |
|---|---|---|
| PX | CN 116178368 A (KPC PHARMACEUTICALS, INC. et al.) 30 May 2023 (2023-05-30) claims 1-9 | 1-14 |
| X | WO 2022099044 A1 (ALIGOS THERAPEUTICS, INC.) 12 May 2022 (2022-05-12) claims 55-65, and description, paragraphs [00436]-[00444] | 1-14 |
| X | CN 114685450 A (KPC PHARMACEUTICALS, INC. et al.) 01 July 2022 (2022-07-01) claims 12-14, and description, embodiment 46 | 1-14 |
| A | US 2022162161 A1 (NANJING RUIJIE PHARMA CO., LTD.) 26 May 2022 (2022-05-26) claims 1-46 | 1-14 |
| A | WO 2021057791 A1 (GUANGDONG HEC PHARMACEUTICAL CO., LTD.) 01 April 2021 (2021-04-01) claims 1-13 | 1-14 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **01 February 2024** | **08 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/136702**

| Patent document cited in search report | Publication date (day/month/year) | Patent family member(s) | Publication date (day/month/year) |
|---|---|---|---|
| CN 116178368 A | 30 May 2023 | None | |
| WO 2022099044 A1 | 12 May 2022 | JP 2023549332 A | 24 November 2023 |
| | | KR 20230124555 A | 25 August 2023 |
| | | US 2022143032 A1 | 12 May 2022 |
| | | US 11786532 B2 | 17 October 2023 |
| | | AU 2021376280 A1 | 15 June 2023 |
| | | TW 202233591 A | 01 September 2022 |
| | | EP 4240730 A1 | 13 September 2023 |
| | | CA 3197220 A1 | 12 May 2022 |
| CN 114685450 A | 01 July 2022 | None | |
| US 2022162161 A1 | 26 May 2022 | EP 3927693 A1 | 29 December 2021 |
| | | EP 3927693 A4 | 05 April 2023 |
| | | WO 2020169069 A1 | 27 August 2020 |
| | | JP 2022521341 A | 06 April 2022 |
| WO 2021057791 A1 | 01 April 2021 | JP 2022549009 A | 22 November 2022 |
| | | US 2022411400 A1 | 29 December 2022 |
| | | EP 4036090 A1 | 03 August 2022 |
| | | EP 4036090 A4 | 15 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202211563493
- WO 2007009913 A
- WO 2010122980 A
- WO 2020073974 A
- CN 111320609 A
- WO 2019240938 A
- WO 2020169069 A
- WO 2021067791 A

### Non-patent literature cited in the description

- *Physiological Reviews*, 2001, vol. 81 (3), 1097-1126
- *Endocrine Reviews*, 1993, vol. 14, 348-399
- *Drugs*, 2017, vol. 77, 1613-1621
- *Agricultural and Biol. Chem.*, 1974, vol. 38 (6), 1169
- *J. Med. Chem.*, 1989, vol. 32, 320
- *J. Med. Chem.*, 2014, vol. 57 (10), 3912